# EUROPEAN PATENT SPECIFICATION

(11) **EP 1 458 717 B1**
(45) Date of publication and mention of the grant of the patent: **07.09.2005**
(21) Application number: 02805310.6
(22) Date of filing: 09.12.2002
(51) Int. Cl.: C07D 471/04, A61K 31/505, A61P 29/00

(54) **AROYL PYRIDINONES**
AROYLPYRIDINONE
AROYL PYRIDINONES

(30) Priority: 21.12.2001 GB 0130723; 31.12.2001 GB 0131102; 08.05.2002 GB 0210511
(43) Date of publication of application: 22.09.2004
(73) Proprietor: Bayer HealthCare AG, 51368 Leverkusen (DE)
(72) Inventor: ALONSO-ALIJA, Cristina, 42781 Haan (DE); MICHELS, Martin, 42653 Solingen (DE); SCHIROK, Hartmut, 44329 Dortmund (DE); SCHLEMMER, Karl-Heinz, 42113 Wuppertal (DE); DODD, Sara, Langley, Berkshire SL3 8LT (GB); FITZGERALD, Mary, Yarnton, Oxfordshire OX5 1QB (GB); BELL, John, Chesham, Buckinghamshire HP5 1LH (GB); GILL, Andrew, Slough, Buckinghamshire SL1 7ET (GB)
(86) International application number: PCT/EP2002/013930
(87) International publication number: WO 2003/053967

(56) References cited:
- WO-A-98/24780
- US-A- 4 288 438

## Description

The present invention relates to imidazolones, processes for their preparation and their use in medicaments, especially for the treatment of COPD.

COPD (chronic obstructive pulmonary disease) is characterised by a neutrophil and macrophage inflammatory burden in the lung. Unlike asthma it has been shown that the inflammation (cells, IL-8, TNF) and airflow obstruction characteristic of COPD is insensitive to therapy with steroids. The critical chemokine driving neutrophilic inflammation is believed to be IL-8, which can be released by a variety of human cells including bronchial epithelial cells, neutrophils and alveolar macrophages.

There are 3 major stress-activated protein kinase pathways 1) p38 mitogen-activated protein (MAP) kinase; 2) extracellular-regulated protein kinase (ERK); 3) c-Jun NH2 terminal kinase (JNK). Activation of human neutrophils and human bronchial epithelial cells results in a rapid activation of p38 MAP kinase which subsequently phosphorylates specific transcription factors, resulting in the synthesis and secretion of inflammatory mediators, particularly IL-8. Studies in vitro with the reference p38 MAP kinase inhibitor, SB 203580, have shown that the release of IL-8 from activated neutrophils and bronchial epithelial cells is linked to the activation of the p38 MAP kinase cascade. The exposure of human bronchial epithelial cells to cigarette smoke extracts also appears to increase the ability of p38 MAP kinase inhibitors to reduce IL-8 release suggesting that exposure to cigarette smoke in vivo may prime the p38 MAP kinase pathway of IL-8 release. These studies suggest that inhibition of p38 MAP kinase may be involved in regulating IL-8 release through an effect on gene expression. Inhibition of p38 MAP kinase may offer an alternative approach to IL-8 antagonism, and may thus provide an effective anti-inflammatory therapy for COPD.

4-Aroyl-5-amino-1-arylpyrazoles are known from WO 01/21591 to inhibit p38 MAP kinase.

2(1H)-pyridone derivatives are known from Yakugaku Zasshi (1979), 99(9), 880-8 and US 4,284,778 to have antiinflammatory activity.

6-Hydroxypyridone derivatives are known from DE 2022817 and DE 2025427 as well as DE 3037911 as coupling agents for azo dyes.

Z. Huang et al. Heterocycles, 1986, 24, 8, 2247 describes the preparation of 8-(benzoyl)-2,3-dihydroimidazo[1,2-a]pyridin-5(1H)-one and its 8-(4-chlorobenzoyl) and 8-(4-methoxybenzoyl) analogs, as well as 9-(benzoyl)-1,2,3,4-tetrahydropyrido[1,2-a]pyrimidin-6-one.

Z. Huang et al. Synthetic Commun., 1989, 19, 1801 describes the preparation of 8-(4-methylbenzoyl)-2,3-dihydroimidazo[1,2-a]pyridin-5(1H)-one and its 8-(2,4-dimethylbenzoyl) analog, 9-(4-methoxybenzoyl)-1,2,3,4-tetrahydropyrido[1,2-a]pyrimidin-6-one and its 9-(4-chlorobenzoyl) analog and 10-benzoyl-2,3,4,5-tetrahydropyrido[1,2-a]-1,3-diazepin-7(1H)-one and its 10-(2,4-dimethylbenzoyl) analog.

Z. Huang et al. J. Chem. Soc. Perkin Trans 1,1993,1085 describes the preparation of 8-(benzoyl)-1-methyl-2,3-dihydroimidazo[1,2-a]pyridin-5(1H)-one and its 8-(4-chlorobenzoyl), 8-(4-methylbenzoyl) and 8-(4-methoxybenzoyl) analogs, as well as 9-(benzoyl)-1,2,3,4-tetrahydropyrido[1,2-a]pyrimidin-6-one and its 9-(4-chlorobenzoyl), 9-(4-methylbenzoyl) and 9-(4-methoxybenzoyl) analogs.

The present invention relates to compounds of formula (I) 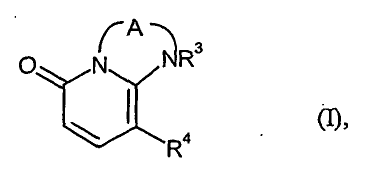 wherein
- A: represents Ethan-1,2-diyl, Propan-1,3-diyl or Butan-1,4-diyl,
which is substituted with 1 to 4 substituents independently selected from the group consisting of C₁-C₆-alkyl, C₁-C₆-alkoxy, C₆-C₁₀-aryl, C₅-C₈-heteroaryl, C₃-C₈-cycloalkyl or COR^{A-1},
wherein C₁-C₆-alkyl, C₁-C₆-alkoxy, C₆-C₁₀-aryl, C₅-C₈-heteroaryl or C₃-C₈-cycloalkyl can be substituted with 0 to 3 substituents R¹⁻¹,
wherein R¹⁻¹ is independently selected from the group consisting of halogen, amino, mono- or di-C₁-C₆-alkylamino, C₁-C₆-alkoxy, hydroxy, C₆-C₁₀-aryl or halogen-C₆-C₁₀-aryl,
and wherein R^{A-1} is OH, C₁-C₆-alkoxy, C₆-C₁₀-aryloxy, amino, mono- or di-C₁-C₆-alkylamino,
and/or
2 substituents on A together with the carbon atoms to which they are attached form a C₃-C₈-cycloalkyl- or C₃-C₈-heterocyclyl-ring,
wherein the C₃-C₈-cycloalkyl- or C₃-C₈-heterocyclyl-ring can be substituted with 0 to 3 substituents independently selected from the group consisting of C₁-C₆-alkyl, C₁-C₆-alkoxy, halogen, cyano, amino, mono- or di-C₁-C₆-alkylamino or COR²⁻², wherein R²⁻² is OH, C₁-C₆-alkoxy, C₆-C₁₀-aryloxy, amino, mono- or di-C₁-C₆-alkylamino,
- R³: represents hydrogen or C₁-C₆-alkyl,
- R⁴: represents -COR⁴⁻¹, wherein
R⁴⁻¹ represents C₆-C₁₀-aryl or C₅-C₈-heteroaryl,
wherein R⁴⁻¹ can be substituted with 0 to 3 substituents independently selected from the group consisting of halogen, amino, C₁-C₆-alkyl, C₂-C₆-alkenyl, C₂-C₆-alkinyl, C₁-C₆-alkoxy, hydroxy, mono or di-C₁-C₆-alkylamino, C₁-C₆-alkycarbonylamino, trifluoromethyl, cyano and nitro,
wherein C₁-C₆-alkyl, C₂-C₆-alkenyl, C₂-C₆-alkinyl and C₁-C₆-alkoxy can be substituted with 0 to 3 substituents independently selected from the group consisting of hydroxy, amino, dimethyl amino, C₁-C₄-alkoxy, 1,3-dioxolan and phenyl, or
R⁴⁻¹ can be substituted with C₆-C₁₀-aryl or C₅-C₈-heteroaryl, which can be optionally be substituted with 0 to 3 substituents independently selected from the group consisting of halogen, amine, C₁-C₆-akoxy, hydroxy or C₆-C₁₀-aryl.

The compounds according to the invention can also be present in the form of their salts, solvates or solvates of the salts.

Depending on their structure, the compounds according to the invention can exist in stereoisomeric forms (enantiomers, diastereomers). The invention therefore relates to the enantiomers or diastereomers and to their respective mixtures. Such mixtures of enantiomers and/or diastereomers can be separated into stereoisomerically unitary constituents in a known manner.

The invention also relates to tautomers of the compounds, depending on the structure of the compounds.

Salts for the purposes of the invention are preferably physiologically acceptable salts of the compounds according to the invention.

Physiologically acceptable salts of the compounds (I) include acid addition salts of mineral acids, carboxylic acids and sulphonic acids, for example salts of hydrochloric acid, hydrobromic acid, sulphuric acid, phosphoric acid, methanesulphonic acid, ethanesulphonic acid, toluenesulphonic acid, benzenesulphonic acid, naphthalenedisulphonic acid, acetic acid, propionic acid, lactic acid, tartaric acid, malic acid, citric acid, fumaric acid, maleic acid and benzoic acid.

Physiologically acceptable salts of the compounds (I) also include salts of customary bases, such as for example and preferably alkali metal salts (for example sodium and potassium salts, alkaline earth metal salts (for example calcium and magnesium salts) and ammonium salts derived from ammonia or organic amines having 1 to 16 carbon atoms, such as illustratively and preferably ethylamine, diethylamine, triethylamine, ethyldiisopropylamine, monoethanolamine, diethanolamine, triethanolamine, dicyclohexylamine, dimethylaminoethanol, procaine, dibenzylamine, N-methylmorpholine, dihydroabietylamine, arginine, lysine, ethylenediamine and methylpiperidine.

Solvates for the purposes of the invention are those forms of the compounds that coordinate with solvent molecules to form a complex in the solid or liquid state. Hydrates are a specific form of solvates, where the coordination is with water.

For the purposes of the present invention, the substituents have the following meanings, unless otherwise specified:
Alkyl per se and "alk" and "alkyl" in alkoxy, alkanoyl, alkylamino, alkylaminocarbonyl, alkylaminosulphonyl, alkylsulphonylamino, alkoxycarbonyl, alkoxycarbonyl amino and alkanoylamino represent a linear or branched alkyl radical having generally 1 to 6 (C₁-C₆-alkyl), preferably 1 to 4 and particularly preferably 1 to 3 carbon atoms, representing illustratively and preferably methyl, ethyl, n-propyl, isopropyl, tert-butyl, n-pentyl and n-hexyl.
C₁-C₆-Alkoxy in general represents a straight-chain or branched hydrocarbon radical having 1 to 6 carbon atoms and bound via an oxygen atom. Non-limiting examples include methoxy, ethoxy, propoxy, isopropoxy, butoxy, isobutoxy, pentoxy, isopentoxy, hexoxy, isohexoxy. The terms "alkoxy" and "alkyloxy" are used synonymously.
C₆-C₁₀-Aryloxy in general represents a aryl radical having 6 to 10 carbon atoms and bound via an oxygen atom. Non-limiting examples include phenoxy.
Cycloalkyl in general represents a cyclic hydrocarbon radical having 3 to 8 carbon atoms. Cyclopropyl, cyclopentyl and cyclohexyl are preferred. Non-limiting examples include cyclopentyl, cyclohexyl, cycloheptyl and cyclooctyl.
Aryl represents a 6- to 10-membered, mono- or bicyclic ring system, which is aromatic at least in one ring. Examples are: phenyl, naphtyl.
Halogen-C₆-C₁₀-aryl represents a 6- to 10-membered, mono- or bicyclic ring system, which is aromatic at least in one ring and which is substituted with one to three halogen atoms, preferably fluorine or chlorine. Examples are: chlorophenyl, 1,3-difluorophenyl.
Mono- or di-C₁-C₆-alkylamino stands for an amino group substituted with 1 or 2 C₁-C₆-alkyl groups. Non-limiting examples include methylamino, dimethylamino, diethylamino.
Heterocyclyl stands for a a saturated or partially unsaturated heterocyclic ring which can contain 1 to 3 heteroatoms selected independently from the group consisting of nitrogen, oxygen or sulfur. It can be attached via a ring nitrogen atom ("N-bound"). C₃-C₈-Heterocyclyl stands for a heterocyclic system containing 3 to 8 ring atoms. Non-limiting examples include tetrahydrofur-2-yl, pyrrolidine-1-yl, piperidine-1-yl, piperidine-2-yl, , piperidine-3-yl, piperidine-4-yl, piperazine-1-yl, piperazine-2-yl morpholine-1-yl, 1,4-diazepine-1-yl or 1,4-dihydropyridine-1-yl.

In the context of the present invention, a 5- to 10-membered aromatic heterocyclic ring ("heteroaryl"), which can contain 1 to 3 heteroatoms selected independently from the group consisting of nitrogen, oxygen or sulfur denotes a ring system, which is mono- or bicyclic, is aromatic at least in one ring, and which can contain 1 to 3 of the abovementioned heteroatoms. It can be attached via a ring carbon atom. C₅-C₈-Heteroaryl stands for a heteroaromat containing 5 to 8 ring atoms. Non-limiting examples include furan-2-yl, furan-3-yl, pyrrol-1-yl, pyrrol-2-yl, pyrrol-3-yl, thienyl, thiazolyl, oxazolyl, imidazolyl, triazolyl, tetrazolyl, pyridyl, pyrimidyl oxadiazolyl or benzoxazolyl.

In the context of the present invention, and/or is an short expression representing the two alternatives "or" and "and". E.g. in the case of substituent A, (a) it can be substituted by the listed substituents or (b) 2 substituents on A form a ring, or (c) it can be substituted by the listed substituents and 2 substituents on A form a ring.

Surprisingly, the compounds of the present invention show p38 MAP kinase inhibitory activity and are therefore suitable for the preparation of medicaments for the treatment of diseases associated with p38 MAP kinase. They may thus provide an effective treatment of acute and chronic inflammatory processes such as toxic shock syndrome, endotoxic shock, tuberculosis, atherosclerosis, diabetes, CSBP/RK/p38 kinase mediated disease is psoriatic arthritis, Reiter's syndrome, gout, traumatic arthritis, rubella arthritis and acute synovitis, rheumatoid arthritis, rheumatoid spondylitis, osteoarthritis, gouty arthritis and other arthritic condition, sepsis, septic shock, endotoxic shock, gram negative sepsis, toxic shock syndrome, cerebral malaria, meningitis, ischemic and hemorrhagic stroke, neurotrauma/open or closed head injury, chronic pulmonary inflammatory disease, silicosis, pulmonary sarcososis, bone resorption disease, osteoporosis, restenosis, cardiac, brain and renal reperfusion injury, thrombosis, glomerularnephritis, chronic renal failure, diabetes, diabetic retinopathy, macular degeneration, graft vs. host reaction, allograft rejection, inflammatory bowel disease, Crohn's disease, ulcerative colitis, neurodegenrative disease, muscle degeneration, diabetic retinopathy, macular degeneration, tumor growth and metastasis, angiogenic disease, eczema, contact dermatitis, psoriasis, sunburn, and conjunctivitis, adult respiratory distress syndrome (ARDS), chronic obstructive pulmonary disease (COPD), fever, periodontal diseases, pyresis, Alzheimer's and Parkinson's diseases, pain and asthma, especially of COPD.

In another embodiment, the present invention relates to compounds of formula (I)
wherein
- A: represents Ethan-1,2-diyl, Propan-1,3-diyl or Butan-1,4-diyl,
which is substituted with 1 to 4 substituents independently selected from the group consisting of C₁-C₆-alkyl, C₁-C₆-alkoxy, C₆-C₁₀-aryl, C₅-C₈-heteroaryl, C₃-C₈-cycloalkyl or COR^{A-1},
wherein C₁-C₆-alkyl, C₁-C₆-alkoxy, C₆-C₁₀-aryl, C₅-C₈-heteroaryl or C₃-C₈-cycloalkyl can be substituted with 0 to 3 substituents R¹⁻¹,
wherein R¹⁻¹ is independently selected from the group consisting of halogen, amino, mono- or di-C₁-C₆-alkylamino, C₁-C₆-alkoxy, hydroxy or C₆-C₁₀-aryl,
and wherein R^{A-1} is OH, C₁-C₆-alkoxy, C₆-C₁₀-aryloxy, amino, mono- or di-C₁-C₆-alkylamino,
and/or
2 substituents on A together with the carbon atoms to which they are attached form a C₃-C₈-cycloalkyl- or C₃-C₈-heterocyclyl-ring,
wherein the C₃-C₈-cycloalkyl- or C₃-C₈-heterocyclyl-ring can be substituted with 0 to 3 substituents independently selected from the group consisting of C₁-C₆-alkyl, C₁-C₆-alkoxy, halogen, cyano, amino, mono- or di-C₁-C₆-alkylamino or COR²⁻², wherein R²⁻² is OH, C₁-C₆-alkoxy, C₆-C₁₀-aryloxy, amino, mono- or di-C₁-C₆-alkylamino,
- R³: represents hydrogen or C₁-C₆-alkyl,
- R⁴: represents -COR⁴⁻¹, wherein
R⁴⁻¹ represents C₆-C₁₀-aryl or C₅-C₈-heteroaryl,
wherein R⁴⁻¹ can be substituted with 0 to 3 substituents independently selected from the group consisting of halogen, amino, C₁-C₆-alkyl, C₂-C₆-alkenyl, C₂-C₆-alkinyl, C₁-C₆-alkoxy, hydroxy, mono or di-C₁-C₆-alkylamino, trifluoromethyl, cyano and nitro,
wherein C₁-C₆-alkyl, C₂-C₆-alkenyl, C₂-C₆-alkinyl and C₁-C₆-alkoxy can be substituted with 0 to 3 substituents independently selected from the group consisting of hydroxy, amino, dimethylamino, C₁-C₄-alkoxy and 1,3-dioxolan, or
R⁴⁻¹ can be substituted with C₆-C₁₀-aryl or C₅-C₈-heteroaryl, which can be optionally be substituted with 0 to 3 substituents independently selected from the group consisting of halogen, amine, C₁-C₆-alkoxy, hydroxy or C₆-C₁₀-aryl.

In another embodiment, the present invention relates to compounds of formula (I),
wherein
- A: represents Ethan-1,2-diyl, Propan-1,3-diyl or Butan-1,4-diyl,
which is substituted with 1 to 4 substituents independently selected from the group consisting of C₁-C₆-alkyl, C₁-C₆-alkoxy, C₆-C₁₀-aryl or C₃-C₈-cycloalkyl,
wherein C₁-C₆-alkyl, C₁-C₆-alkoxy, C₆-C₁₀-aryl or C₃-C₈-cycloalkyl can be substituted with 0 to 3 substituents R¹⁻¹,
wherein R¹⁻¹ is independently selected from the group consisting of halogen, C₁-C₆-alkoxy, hydroxy, C₆-C₁₀-aryl C₆-C₁₀-aryl or halogen-C₆-C₁₀-aryl,
and/or
2 substituents on A together with the carbon atoms to which they are attached form a C₃-C₈-cycloalkyl- or C₃-C₈-heterocyclyl-ring,
wherein the C₃-C₈-cycloalkyl- or C₃-C₈-heterocyclyl-ring can be substituted with 0 to 3 substituents independently selected from the group consisting of C₁-C₆-alkyl, C₁-C₆-alkoxy, halogen and cyano,
- R³: represents hydrogen,
- R⁴: represents -COR⁴⁻¹, wherein
R⁴⁻¹ represents phenyl or naphtyl,
wherein R⁴⁻¹ can be substituted with 0 to 3 substituents independently selected from the group consisting of halogen, C₁-C₆-alkyl, C₂-C₆-alkenyl, C₂-C₆-alkinyl, C₁-C₆-alkoxy, hydroxy, C₁-C₆-alkycarbonylamino, trifluoromethyl and nitro,
wherein C₂-C₆-alkinyl can be substituted with 0 to 2 phenyl.

In another embodiment, the present invention relates to compounds of formula (I),
wherein
- A: represents Ethan-1,2-diyl or Propan-1,3-diyl,
which is substituted with 1 to 4 substituents independently selected from the group consisting of C₁-C₆-akyl, C₁-C₆-alkoxy, C₆-C₁₀-aryl or C₃-C₈-cycloalkyl,
wherein C₁-C₆-alkyl, C₁-C₆-alkoxy, C₆-C₁₀-aryl or C₃-C₈-cycloalkyl can be substituted with 0 to 3 substituents R¹⁻¹,
wherein R¹⁻¹ is independently selected from the group consisting of halogen, C₁-C₆-alkoxy, hydroxy or C₆-C₁₀-aryl,
and/or
2 substituents on A together with the carbon atoms to which they are attached form a C₃-C₈-cycloalkyl- or C₃-C₈-heterocyclyl-ring,
wherein the C₃-C₈-cycloalkyl- or C₃-C₈-heterocyclyl-ring can be substituted with 0 to 3 substituents independently selected from the group consisting of C₁-C₆-alkyl, C₁-C₆-alkoxy, halogen and cyano,
- R³: represents hydrogen,
- R⁴: represents -COR⁴⁻¹, wherein
R⁴⁻¹ represents phenyl,
wherein R⁴⁻¹ can be substituted with 0 to 3 substituents independently selected from the group consisting of halogen, C₁-C₆-alkyl, C₂-C₆-alkenyl, C₂-C₆-akinyl, C₁-C₆-alkoxy, hydroxy and trifluoromethyl.

In another embodiment, the present invention relates to compounds of formula (I),
wherein
- A: represents Ethan-1,2-diyl or Propan-1,3-diyl,
which is substituted with 1 to 4 substituents independently selected from the group consisting of C₁-C₆-alkyl, phenyl, benzyl and chlorobenzyl,
and/or
2 substituents on A together with the carbon atoms to which they are attached form a C₃-C₈-cycloalkyl-ring,
wherein the C₃-C₈-cycloalkyl-ring can be substituted with 0 to 3 substituents independently selected from the group consisting of C₁-C₆-alkyl, C₁-C₆-alkoxy and halogen,
R³ represents hydrogen,
- R⁴: represents -COR⁴⁻¹, wherein
R⁴⁻¹ represents phenyl,
wherein R⁴⁻¹ can be substituted with 0 to 2 substituents independently selected from the group consisting of fluorine, chlorine, bromine, methyl, hydroxy, methoxy and C₁-C₆-alkycarbonylamino.

In another embodiment, the present invention relates to compounds of formula (I),
wherein
- A: represents Ethan-1,2-diyl or Propan-1,3-diyl,
which is substituted with 1 to 4 substituents independently selected from the group consisting of C₁-C₆-alkyl,
and/or
2 substituents on A together with the carbon atoms to which they are attached form a C₃-C₈-cycloalkyl-ring,
wherein the C₃-C₈-cycloalkyl-ring can be substituted with 0 to 3 substituents independently selected from the group consisting of C₁-C₆-alkyl, C₁-C₆-alkoxy and halogen,
- R³: represents hydrogen,
- R⁴: represents -COR⁴⁻¹, wherein
- R⁴⁻¹: represents phenyl,
wherein R⁴⁻¹ can be substituted with 0 to 2 substituents independently selected from the group consisting of fluorine, chlorine, bromine, methyl and hydroxy.

In another embodiment, the present invention relates to compounds of formula (IA) 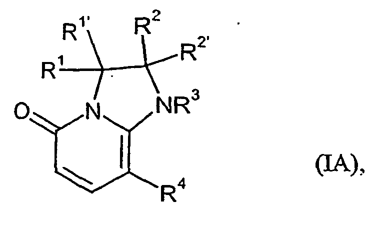 wherein
- R¹: represents hydrogen, C₁-C₆-alkyl, C₆-C₁₀-aryl, C₅-C₈-heteroaryl or C₃-C₈-cycloalkyl, wherein C₁-C₆-akyl, C₆-C₁₀-aryl, C₅-C₈-heteroaryl or C₃-C₈-cycloalkyl can be substituted with 0 to 3 substituents R¹⁻¹, wherein R¹⁻¹ is independently selected from the group consisting of halogen, amino, mono- or di-C₁-C₆-alkylamino, C₁-C₆-alkoxy or C₆-C₁₀-aryl,
- R^{1'}: represents hydrogen or C₁-C₄-akyl,
- R²: represents hydrogen, C₁-C₆-alkyl, C₆-C₁₀-aryl, C₅-C₈-heteroaryl or C₃-C₈-cycloalkyl, wherein C₁-C₆-alkyl, C₆-C₁₀-aryl, C₅-C₈-heteroaryl or C₃-C₈-cycloallcyl can be substituted with 0 to 3 substituents R²⁻¹, wherein R²⁻¹ is independently selected from the group consisting of halogen, amino, mono- or di-C₁-C₆-alkylamino, C₁-C₆-alkoxy or C₆-C₁₀-aryl,
- R^{2'}: represents hydrogen or C₁-C₄-alkyl,
or
- R² and R^{2'}: together with the carbon atom to which they are attached form a oxo group,
or
- R¹ and R²: together with the carbon atoms to which they are attached form a C₃-C₈-cycloalkyl- or C₃-C₈-heterocyclyl-ring,
wherein the ring can be substituted with 0 to 3 substituents independently selected from the group consisting of C₁-C₆-alkyl, C₁-C₆-alkoxy, halogen, cyano, amino, mono- or di-C₁-C₆-alkylamino, COR²⁻², wherein R²⁻² is OH, C₁-C₆-alkoxy, C₆-C₁₀-aryloxy, amino, mono- or di-C₁-C₆-alkylamino,
- R³: represents hydrogen or C₁-C₆-alkyl,
- R⁴: represents -COR⁴⁻¹, wherein
R⁴⁻¹ represents C₆-C₁₀-aryl or heteroaryl,
wherein R⁴⁻¹ can be substituted with 0 to 3 substituents independently selected from the group consisting of halogen, amino, C₁-C₆-alkyl, C₁-C₆-alkoxy, hydroxy, mono or di-C₁-C₆-alkylamino, trifluoromethyl, cyano and nitro, wherein C₁-C₆-alkyl and C₁-C₆-alkoxy can be substituted with 0 to 3 substituents independently selected from the group consisting of hydroxy, amino, dimethylamino, C₁-C₄-alkoxy and 1,3-dioxolan, or
R⁴⁻¹ can be substituted with aryl or heteroaryl, which can be optionally be substituted with 0 to 3 substituents independently selected from the group consisting of halogen, amine, C₁-C₆-alkoxy, hydroxy or aryl,
with the proviso, that R¹, R^{1'}, R² and R^{2'} are not hydrogen at the same time,
or pharmaceutically acceptable salts thereof.

In another embodiment, the present invention relates to compounds according to formula (IA), wherein
- R¹: represents C₁-C₃-alkyl, wherein C₁-C₃-alkyl can be substituted with 0 to 2 substituents R¹⁻¹, wherein R¹⁻¹ is independently selected from the group consisting of halogen, C₁-C₃-alkoxy or benzyloxy,
- R^{1'}: represents hydrogen or methyl,
- R²: represents C₁-C₃-alkyl, wherein C₁-C₃-alkyl can be substituted with 0 to 2 substituents R¹⁻¹, wherein R¹⁻¹ is independently selected from the group consisting of halogen, C₁-C₃-alkoxy or benzyloxy,
- R^{2'}: represents hydrogen,
or
- R¹ and R²: together with the carbon atoms to which they are attached form a C₃-C₆-cycloalkyl-ring,
wherein the ring can be substituted with 0 to 2 substituents independently selected from the group consisting of C₁-C₃-alkyl, C₁-C₃-alkoxy, halogen, amino,
- R³: represents hydrogen or C₁-C₃-alkyl,
- R⁴: represents -COR⁴⁻¹, wherein
R⁴⁻¹ represents phenyl,
wherein phenyl can be substituted with 0 to 2 substituents independently selected from the group consisting of halogen, amino, C₁-C₃-alkyl, C₁-C₃-alkoxy and hydroxy,
with the proviso, that R¹, R^{1'}, R² and R^{2'} are not hydrogen at the same time,
or pharmaceutically acceptable salts thereof.

In another embodiment, the present invention relates to compounds according to formula (IA), wherein
- R¹: represents C₁-C₃-alkyl,
- R^{1'}: represents hydrogen or methyl,
- R²: represents C₁-C₃-alkyl,
- R^{2'}: represents hydrogen,
or
- R¹ and R²: together with the carbon atoms to which they are attached form a C₅- or C₆-cycloalkyl-ring,
- R³: represents hydrogen,
- R⁴: represents -COR⁴⁻¹, wherein
R⁴⁻¹ represents phenyl, wherein phenyl can be substituted with 0 to 2 substituents independently selected from the group consisting of fluorine, chlorine, bromine or methyl,
with the proviso, that R¹, R^{1'}, R² and R^{2'} are not hydrogen at the same time,
or pharmaceutically acceptable salts thereof.

In another embodiment, the present invention relates to compounds according to formula (I), wherein A represents Ethan-1,2-diyl, and 2 substituents on A together with the carbon atoms to which they are attached form a cyclohexylring, which can be substituted as described above.

In another embodiment, the present invention relates to compounds according to formula (I), wherein A represents Propan-1,3-diyl, and 2 substituents on A together with the carbon atoms to which they are attached form a cyclopentylring, which can be substituted as described above and A is substituted with C₁-C₆-alkyl, especially methyl.

In another embodiment, the present invention relates to compounds according to formula (I), wherein A is Propan-1,3-diyl, wherein A can be substituted with methyl or geminal di-C₁-C₆-alkyl.

In another embodiment, the present invention relates to compounds according to formula (LA,), wherein R¹ and R² together with the carbon atoms to which they are attached form a C₅- or C₆-cycloalkyl-ring, especially a cyclohexyl ring.

In another embodiment, the present invention relates to compounds according to formula (I) or (IA), wherein R³ is hydrogen.

In another embodiment, the present invention relates to compounds of formula (I), wherein R⁴ is -C(O)C₆H₅, wherein R⁴ can be substituted with 0 to 3 substituents independently selected from the group consisting of fluorine, chlorine, bromine, hydroxy or methyl, especially fluorine or chlorine, especially double-folded substitution with fluorine or chlorine, preferably with 2,4-difluoro.

In another embodiment, the present invention relates to compounds of formula (IA), wherein R⁴ is -C(O)C₆H₅, wherein R⁴ can be substituted with 0 to 3 substituents independently selected from the group consisting of fluorine, chlorine, bromine or methyl, especially fluorine or chlorine, especially double-folded substitution with fluorine or chlorine, preferably with 2,4-difluoro.

The compounds of the present invention show unexpected, valuable pharmacological and pharmacokinetical properties.

They are thus suitable for the preparation of medicaments for the prevention and treatment of diseases. They can be administered alone or in combination with other active ingredients.

In another embodiment, the present invention relates to compounds of formula (I) with IC₅₀-values [p 38 map kinase] of less than 10 µM, especially less than 1 µM and very especially less than 0,5 µM.

The percentages in the tests and examples which follows are, unless otherwise stated, by weight; parts are by weight. Solvent ratios, dilution ratios and concentrations reported for liquid/liquid solutions are each based on the volume.

### A. Biological Experiments

The *in vitro*-properties of the compounds can be shown in the following experiments:

### P 38 map kinase assay

The assay makes use of the serine/ threonine protein kinase SPA [33-P] +assay kit from Amersham Pharmacia Biotech. The assay is a homogeneous technique using SPA technology for the quantification of serine threonine kinase activity.

It is based on the p38 map kinase catalysed transfer of the γ-phosphate group of the [γ-³³P] ATP to the substrate, biotinylated myelin basic protein (MBP). The resulting [³³P]-labelled biotinylated product is trapped on a PVT SPA bead containing scintillant which has been surface coated with streptavidin.

The beads are allowed to settle to eliminate high background, and therefore only ³³P labelled product attached to the SPA bead is detected.

The assay is carried out in the presence and absence of test compounds to determine their effect on p38 map kinase activity.

A test protocol is as follows:
1. SPA assay kit (Amersham). Components:
   - Assay buffer (store frozen)
   - Stop solution (store frozen)
   - Streptavidin coated SPA beads - reconstitute with 5 mls of PBS.
      (50mg/ml).(Store in fridge)
2. p 38 map kinase enzyme SCRD (500ug/ml) - aliquoted in 1.5 mls.
   - dilute 1: 10 to 50µg/ml
   - 1 plate :- 110µl (stock 500ug/ml) + 990µl PBS.
3. Assay reagent:
   - for 1 plate :- 504µl Assay buffer (500Mm MOPS pH7.2, 10µM ATP,
      50mM MgCl₂, 25µM biotinylated myelin basic protein (MBP)).
   - 2513.4µl Water
   - 1.1µl 33-p -ATP (10µCi /ul) (on activity date / adjust for activity date)
   - 4.534µl X10-2M ATP in water
4. Stop solution:
   - for 1 plate:- 265.92µl streptavidin coated beads (50mg/ml)
   - 1651.68µl stop buffer (500 µM ATP, 50mM EDTA 1% Triton X-100)
   - 7084.32µl PBS.

1. Add 10µl compound Dilutions (5x final conc) Test wells.
2. Add 10µl 12.5% DMSO to control/ blank wells.
3. Add 10µl enzyme (50µg/ml) - final conc 500 ng/well
4. Add 10µl PBS to blank wells.
5. Add 30µl of assay reagent to each well. (final conc 10µM ATP, 2.5 uM substrate)
6. Mix well on plate shaker
7. Incubate 90 min (30°C)
8. Add 75µl of stop solution to each well (final conc 55 µM ATP)
9. Spin plate :-3min /1600rpm /20°C (alternatively leave to settle overnight)
10. Read in Microbeta, Protocol SPA paralux 3.

Representative Data are given in table 1:

**Table 1**

| **Ex. No.** | **IC**_{**50**} **(nM)** |
|---|---|
| 1 | 340 |
| 2-1 | 164 |
| 3 | 455 |
| 9 | 228 |
| 21 | 1797 |
| 24 | 386 |

### Description of the functional Assays

Neutrophils are isolated from human blood via discontinuous Percoll gradient and seeded at 1x10⁶ cells/well. Compounds are added, and the cells are incubated for 1h at 37°C. After 1h, cells are stimulated with TNF-alpha (25ng/ml final conc.) for 18h. Supernatants are harvested and analysed for IL-8 content by ELISA.

The suitability of the compounds for the prevention and treatment of diseases can be shown in the following *in vivo*-model:

### Description of the in vivo model

### Mouse acute lipopolysaccharide (LPS) method

- Animals (species, strain):: Mouse, Balb/C
- Dosing vehicle:: Solutol HS15 (polyethylene glycol 660 12-hydroxystearate; BASF, Germany)/ethanol or tylose (carboxymethylcellulose; Sigma, Germany) as an excipient mixed with either water (enteral studies) or saline (parenteral studies).

### Method of preparation of test substance:

The test substance is ground into a fine powder using a pestle and mortar and dissolved in the excipient. Water or saline is then added to achieve the desired dosing concentration.

### Experimental protocol:

1. Compound administration. Mice are randomly assigned into groups and administered vehicle or test substance, by an enteral or parenteral route, on one occasion within 24 hours of inflammatory challenge, and up to two occasions in the 24 hours thereafter.
2. Inflammatory challenge. Mice are lightly anaesthetised (halothane/O₂) and *intra nasally* administered either saline or LPS (0.1 µg to 10 µg; *Pseudomonas aeruginosa;* Sigma) at a dose volume of 25 µl/nare.
3. Bronchoalveolar lavage (BAL). Within 24 hours of inflammatory challenge, mice are euthanised using sodium pentabarbitone (*i.p.*). BAL fluid is then collected into heparinised phosphate buffered saline and centrifuged. The pellet can be used for the cell counting of neutrophils; and the supematent assayed for KC (R&D Systems), macrophage inflammatory protein 2 (R&D Systems) or tumour necrosis factor-alpha (Biosource International) using commercially available ELISA kits. Lung tissue can also be removed for later myeloperoxidase assay as an index of neutrophil recruitment into the lungs.

- Health Status monitoring:: Mice are monitored for adverse effects.
- Statistical methods:: Data are analysed using an appropriate statistical test and considered significant at the p<0.05 level.

In another embodiment, the present invention relates to the composition containing at least one compound of general formula (I) and a pharmacologically acceptable diluent and the use of such composition for the treatment of acute and chronic inflammatory processes as well as the process for the preparation of such compositions, characterized in that the compounds of general formula (I) together with customary auxiliaries in brought into a suitable application form.. The compounds of general formula (I) are therefor useful for the preparation of medicaments, especially of medicaments for the treatment of acute and chronic inflammatory processes such as described in the introduction, preferably asthma and COPD, especially COPD.

For the treatment of the above-mentioned diseases, the compounds according to the invention can exhibit non-systemic or systemic activity, wherein the latter is preferred. To obtain systemic activity the active compounds can be administered, among other things, orally or parenterally, wherein oral administration is preferred. To obtain non-systemic activity the active compounds can be administered, among other things, topically.

For parenteral administration, forms of administration to the mucous membranes (i.e. buccal, lingual, sublingual, rectal, nasal, pulmonary, conjunctival or intravaginal) or into the interior of the body are particularly suitable. Administration can be carried out by avoiding absorption (i.e. intracardiac, intra-arterial, intravenous, intraspinal or intralumbar administration) or by including absorption (i.e. intracutaneous, subcutaneous, percutaneous, intramuscular or intraperitoneal administration).

For the above purpose the active compounds can be administered per se or in administration forms.

Suitable administration forms for oral administration are, inter alia, normal and enteric-coated tablets, capsules, coated tablets, pills, granules, pellets, powders, solid and liquid aerosols, syrups, emulsions, suspensions and solutions. Suitable administration forms for parenteral administration are injection and infusion solutions.

The active compound can be present in the administration forms in concentrations of from 0.001 - 100 % by weight; preferably the concentration of the active compound should be 0.5 - 90% by weight, i.e. quantities which are sufficient to allow the specified range of dosage.

The active compounds can be converted in the known manner into the above-mentioned administration forms using inert non-toxic pharmaceutically suitable auxiliaries, such as for example excipients, solvents, vehicles, emulsifiers and/or dispersants.

The following auxiliaries can be mentioned as examples: water, solid excipients such as ground natural or synthetic minerals (e.g. talcum or silicates), sugar (e.g. lactose), non-toxic organic solvents such as paraffins, vegetable oils (e.g. sesame oil), alcohols (e.g. ethanol, glycerol), glycols (e.g. polyethylene glycol), emulsifying agents, dispersants (e.g. polyvinylpyrrolidone) and lubricants (e.g. magnesium sulphate).

In the case of oral administration tablets can of course also contain additives such as sodium citrate as well as additives such as starch, gelatin and the like. Flavour enhancers or colorants can also be added to aqueous preparations for oral administration.

For the obtainment of effective results in the case of parenteral administration it has generally proven advantageous to administer quantities of about 0.001 to 100 mg/kg, preferably about 0.01 to 1 mg/kg of body weight. In the case of oral administration the quantity is about 0.01 to 100 mg/kg, preferably about 0.1 to 10 mg/kg of body weight.

It may nevertheless be necessary to use quantities other than those mentioned above, depending on the body weight concerned, the method of administration, the individual response to the active compound, the type of preparation and the time or interval of administration.

In another embodiment, the present invention relates to a process for synthesizing the compounds of general formula (I), characterized in that compounds of general formula (II) 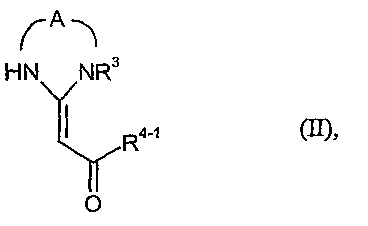 wherein
A, R³ and R⁴⁻¹ have the meaning described above,
are reacted
[A] with propiolic acid in the presence of 1,1-carbonyldiimidazol, or
[B] with C₁-C₆-alkyl propiolate as described in Heterocycles, 1986, 24, 8, 2247, or
[C] with 3-aminoacrylic acid C₁-C₆-alkyl ester or
[D] with 3-alkoxyacrylic acid C₁-C₆-alkyl ester.
[E] with propiolic acid chloride (e.g. generated in situ from propiolic acid and 1-Chlor-N,N,2-trimethylpropenylamin).
[F] with α-chloro acrylic acid chloride (e.g. generated as described in L. M. Sayre, D. L. Larson, A. E. Takemori, P. S. Portoghese, J. Med. Chem. 1984, 27, 1325-1335).

Suitable solvents for the processes [A] to [F] are generally customary organic solvents which do not change under the reaction conditions. These include ethers such as diethyl ether, dioxan or tetrahydrofuran, ethylacetate, acetone, dimethylsulfoxide, dimethylformamide or alcohols such as methanol, ethanol, propanol, butanol or t-butanol, or halogenohydrocarbons such as dichloromethane, dichloroethane, trichloromethane or tetrachloromethane. Preferred for [A] and [B] is tetrahydrofuran, for [C] and [D] toluene or toluene/ethanol.

Process [B] can take place in the presence of a base. Suitable bases are generally inorganic or organic bases. These preferably include alkali alcoholates, such as sodium methylate in methanol. The base is employed in an amount from 1 mol to 10 mol, preferably from 1.0 mol to 4 mol, relative to 1 mol of the compound of the general formula (II).

Process [C] and [D] can be carried out in the presence of molecular sieves (4Å).

The processes [A] to [F] are in general carried out in a temperature range from -30°C to +100°C, preferably from -10°C to +50°C. Most reactions can be carried out at room temperature or reflux temperature of the corresponding solvent.

The processes [A] to [F] are generally carried out at normal pressure. However, it is also possible to carry it out at elevated pressure or at reduced pressure (for example in a range from 0.5 to 5 bar).

The compounds of general formula (II) are known (e.g. from Synth. Comm. 1993, 23, 2533-2546) or can be synthesized by reacting compounds of general formula (IIIa), (IIIb) or (IIIc), 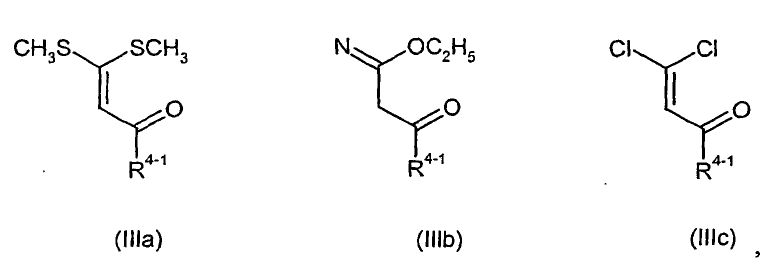 wherein R⁴⁻¹ has the meaning described above,
with compounds of general formula (IV) 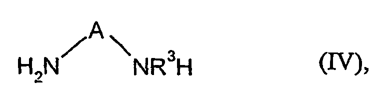 wherein A and R³ have the meaning described above. The use of compounds of formula (IIIa) is preferred.

The compounds of general formula (IIIa) are known or can be synthesized in analogy to Synth. Comm. 1989, 19, 943-958 or Bull. Soc. Chim. Fr. 1959, 1398-1399.

The compounds of general formula (IIIb) are known or can be synthesized in analogy to Synthesis 1957, 4, 357-362.

The compounds of general formula (IIIc) are known or can be synthesized in analogy to J. Org. Chem. USSR 1973, 9, 320-322 from 3,3-dichloroacrylic acid chloride and the corresponding moiety R⁴⁻¹.

The compounds of general formula (IV) are commercially available or can be synthesised analogously to known procedures. For example, alkyl-1,3-diamines can be synthesized by reduction of alkyl dinitriles with sodium boron hydride.

The processes can be illustrated by the following scheme:

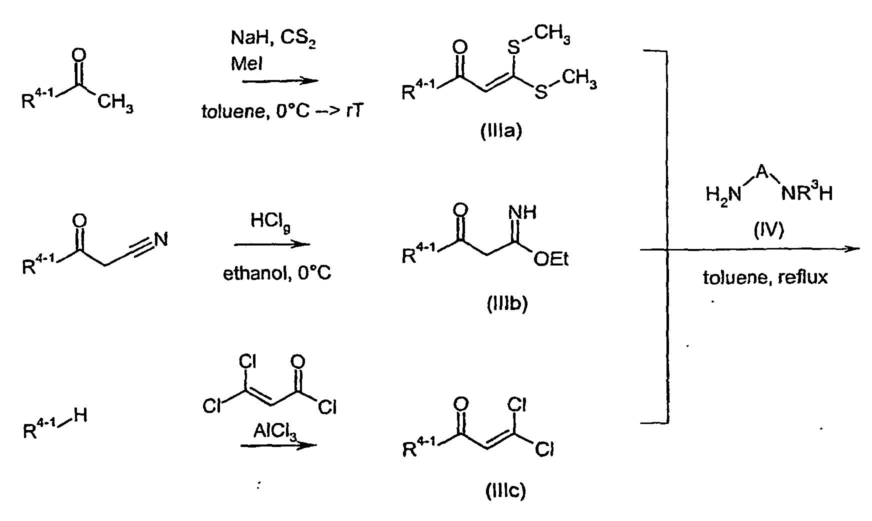

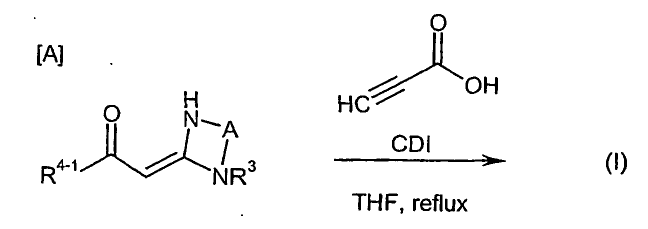

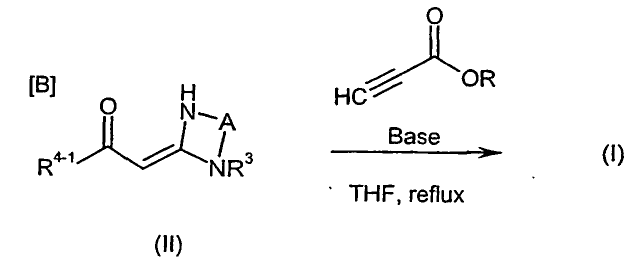

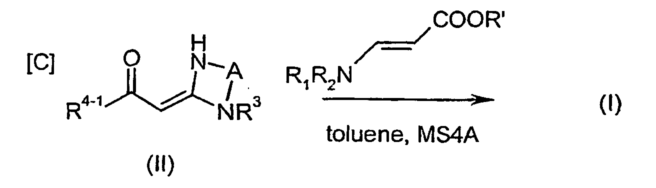

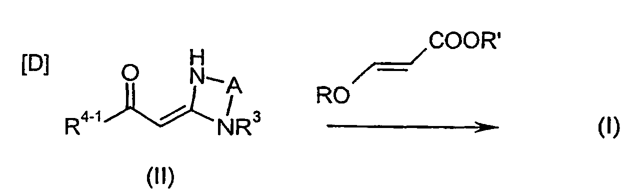

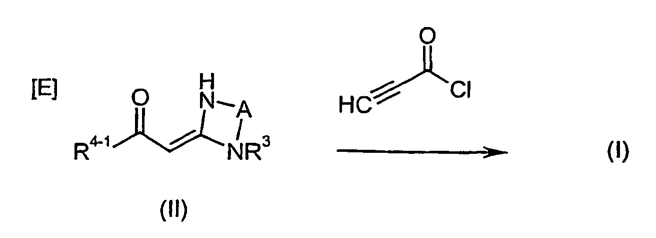

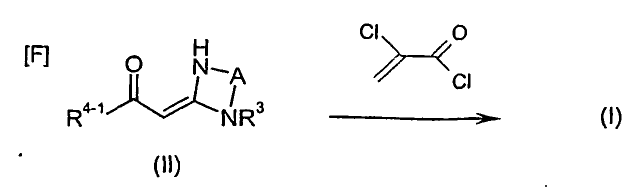

For R³ is H and A is nonsymmetric, depending on the reaction conditions and starting materials, the compounds (I) can be obtained in two different regioisomers.

Compounds of general formula (IIA) 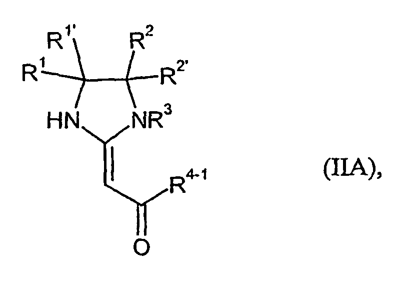 wherein R¹ and R² together with the carbon atoms, to which they are attached, form a cyclohexyl ring, R^{1'} and R^{2'} are hydrogen and R³ and R⁴⁻¹ have the meaning described above, are especially valuable intermediates in the synthesis of the corresponding compounds of the present invention. For this reason they are also part of the present invention with the exception of such compounds, wherein R⁴⁻¹ represents phenyl, p-methylphenyl or p-methoxyphenyl, which are known from Synth. Comm. 1993, 23, 2533-2546. These compounds can exist in form of their corresponding enolates.

### B. Examples

### The following abbreviations are used in the descriptions:

- ACN =: acetonitrile
- aq.=: aqueous
- CDI: 1,1-carbonyldiimidazol
- DCM =: dichloromethane
- DMF =: dimethylsulfoxide
- HPLC =: High Pressure Liquid Chromatography
- min. =: minute
- MS =: mass spectroscopy
- MS4A: molecular sieves 4 Angstrom
- PE =: petroleumether
- Rₜ =: retention time
- rt =: room temperature
- THF =: tetrahydrofuran
- % of th. =: % of theory

### LC/MS

### Method A (MHZ2P)

Instrument: Micromass Platform LCZ, HP1100; column: Symmetry C18, 50 mm x 2.1 mm, 3.5 µm; eluent A: acetonitrile + 0.1% formic acid, eluent B: water + 0.1% formic acid; gradient: 0.0min 10%A → 4.0min 90%A → 6.0min 90%A; Ofen: 40°C, flow: 0.5ml/min, UV-detection: 208-400 nm

### Method B (MHZ2Q)

Instrument: Micromass Quattro LCZ, HP1100; column: Symmetry C18, 50 mm x 2.1 mm, 3.5 µm; eluent A: acetonitrile + 0.1% formic acid, eluent B: water + 0.1% formic acid; gradient: 0.0min 10%A → 4.0min 90%A → 6.0min 90%A; temp.: 40°C, flow: 0.5ml/min, UV-detection: 208-400 nm

### Methode C (SMKL-ZQ-2)

### Instrument: Waters Alliance 2790 LC

Säule: Symmetry C18, 50mm x 2.1, 3.5µm; Eluent A: Wasser+ 0.1% Ameisensäure, Eluent B: Acetonitril + 0.1% Ameisensäure; Gradient: 0.0min 5% B → 5.0min 10%B → 6.0min 10%B; Temperatur: 50°C, Fluss: 1.0ml/min, UV-Detektion: 210nm

### HPLC

### Methode D (SYA-HPPSK2):

Instrument: HP 1100 mit DAD-Detektion; Säule: Kromasil RP-18, 60mm x 2 mm, 3.5µm; Eluent: A=5ml HClO4/l H20, B=ACN; Gradient: 0 min 2%B, 0.5min 2%B, 4.5min 90%B, 9 min 90%B; Fluß:0.75 ml/min, Temp.:30 Grad C, Detektion UV 210 nm

### Example 1a

### 1-(2,4-Difluorophenyl)-3,3-bis(methylsulfanyl)-2-propen-1-one

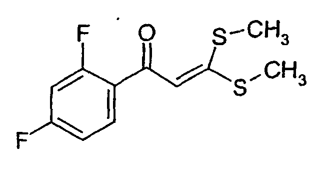

4 g (25.6 mmol) 1-(2,4-difluorophenyl)ethanone, 2.05 g (60%, 51.3 mmol) sodium hydride and 2.3 ml (38.4 mmol) carbon disulfide are dissolved in 200 ml toluene. At room temperature 8 ml *N*,*N*-dimethylformamide are added dropwise during 30 min. The mixture is cooled in an ice bath, and after dropwise addition of 4.8 ml (76.9 mmol) methyl iodide stirred for 1 h in an ice bath. The mixture is diluted with 100 ml toluene and carefully poured on ice water. The organic layer is separated, dried over magnesium sulfate, filtered and concentrated under vacuum. The crude is recrystallized from toluene/diethyl ether, filtered and washed with diethyl ether to yield 1.5 g (22% of th.) 1-(2,4-difluorophenyl)-3,3-bis(methylsulfanyl)-2-propen-1-one.

¹H-NMR (300MHz, DMSO-d₆): δ = 2.51 (s, 3H, under DMSO), 2.58 (s, 3H), 6.63 (s, 1H), 7.21 (m, 1H), 7.38 (m, 1H), 7.84 (m, 1H).

### Example 2a

### 2-[(Rac-cis-3a,7a)-octahydro-2H-benzimidazol-2-ylidene]-1-(2,4-difluorophenyl)ethanone

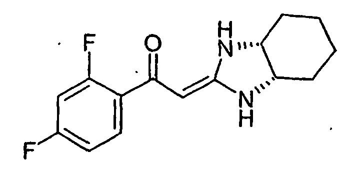

400 mg (1.54 mmol) of the compound of example 1a and 260 mg (2.31 mmol) *cis*-1,2-diaminocyclohexane are dissolved in 25 ml toluene and refluxed for 24 h. Upon cooling a precipitate is formed which is collected by filtration and washed with diethyl ether to yield 200 mg (47% of th.) 2-[(*rac*-*cis*-3a,7a)-octahydro-2H-benzimidazol-2-ylidene]-1-(2,4-difluorophenyl)ethanone.

¹H-NMR (300MHz, DMSO-d₆): δ = 1.1 - 1.8 (m, 8H), 3.67 (m, 1H), 3.78 (m, 1H), 5.12 (s, 1H), 7.07 (m, 1H), 7.16 (m, 1H), 7.52 (br. s, 1H, NH), 7.76 (m, 1H), 9.09 (br. s, 1H, NH).

### Example 3a

### 2-[(Rac-trans-3a,7a)-octahydro-2H-benzimidazol-2-ylidene]-1-(2,4-difluorophenyl)ethanone

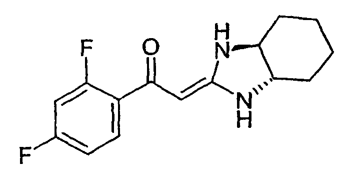

400 mg (1.54 mmol) of the compound of example 1a and 260 mg (2.31 mmol) *rac-trans*-1,2-diaminocyclohexane are dissolved in 25 ml toluene and refluxed for 24 h. Upon cooling a precipitate is formed which is collected by filtration and washed with diethyl ether to yield 317 mg (74% of th.) 2-[(*rac-trans*-3a,7a)-octahydro-2H-benzimidazol-2-ylidene]-1-(2,4-difluorophenyl)ethanone.

¹H-NMR (300MHz, DMSO-d₆): δ = 1.17 - 1.51 (m, 4H), 1.75 (m, 2H), 2.0 (m, 1H), 2.16 (m, 1H), 3.01 (m, 2H), 5.17 (s, 1H), 7.08 (m, 1H), 7.17 (m, 1H), 7.66 (br. s, 1H, NH), 7.74 (m, 1H), 9.21 (br. s, 1H, NH).

### Example 4a

### 3,3-Bis(methylsulfanyl)-1-phenyl-2-propen-1-one

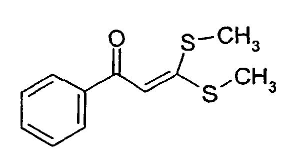

5 g (41.6 mmol) acetophenone, 3.33 g (60%, 83.2 mmol) sodium hydride and 3.8 ml (62.4 mmol) carbon disulfide are dissolved in 250 ml toluene. At room temperature 10 ml *N*,*N*-dimethylformamide are added dropwise during 30 min. The mixture is cooled in an ice bath, and after dropwise addition of 7.8 ml (125 mmol) methyl iodide stirred for 1 h in an ice bath. The mixture is diluted with 100 ml toluene and carefully poured on ice water. The organic layer is separated, dried over magnesium sulfate, filtered and concentrated under vacuum. The crude is purified over preparative HPLC (RP18-column, eluent: acetonitrile-water gradient) to yield 3.3 g (35% of th.) 3,3-bis(methylsulfanyl)-1-phenyl-2-propen-1-one.

¹H-NMR (300MHz, DMSO-d₆): δ = 2.49 (s, 3H, under DMSO), 2.66 (s, 3H), 6.88 (s, 1H), 7.45 - 7.62 (m, 3H), 7.96 (m, 2H).

### Example 5a

### 2-[(Rac-cis-3a,7a)-octahydro-2H-benzimidazol-2-ylidene]-1-phenylethanone

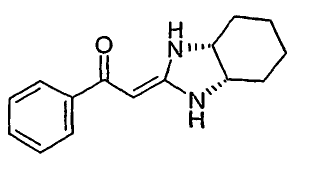

500 mg (2.23 mmol) of the compound of example 4a and 382 mg (3.34 mmol) *cis*-1,2-diaminocyclohexane are dissolved in 25 ml toluene and refluxed for 24 h. Upon cooling a precipitate is formed which is collected by filtration and washed with diethyl ether to yield 340 mg (63% of th.) 2-[(*rac*-*cis*-3a,7a)-octahydro-2H-benzimidazol-2-ylidene]-1-phenylethanone.

¹H-NMR (300MHz, DMSO-d₆): δ = 1.2 - 1.8 (m, 8H), 3.66 (m, 1H), 3.77 (m, 1H), 5.27 (s, 1H), 7.30 - 7.44 (m, 4H, incl. 1NH), 7.66 - 7.77 (m, 2H), 9.21 (s, 1H, NH).

### Example 6a

### 2-[(Rac-trans-3a,7a)-octahydro-2H-benzimidazol-2-ylidene]-1-phenylethanone

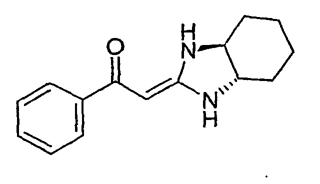

1.0 g (4.46 mmol) of the compound of example 4a and 760 mg (6.67 mmol) *rac-trans*-1,2-diaminocyclohexane are dissolved in 25 ml toluene and refluxed for 24 h. Upon cooling a precipitate is formed which is collected by filtration and washed with diethyl ether to yield 820 mg (76% of th.) 2-[(*rac-trans*-3a,7a)-octahydro-2H-benzimidazol-2-ylidene]-1-phenylethanone.

¹H-NMR (300MHz, CDCl₃): δ = 1.12 - 1.69 (m, 4H), 1.84 (m, 2H), 1.94 - 2.24 (m, 2H), 2.92 - 3.28 (m, 2H), 4.87 (s, 1H, NH), 5.45 (s, 1H), 7.27 - 7.45 (m, 3H), 7.66 - 7.91 (m, 2H), 9.55 (s, 1H, NH).

### Example 7a

### 2-[(Rac-cis-3a,7a)-octahydro-2H-benzimidazol-2-ylidene]-1-(3-fluorophenyl)-ethanone

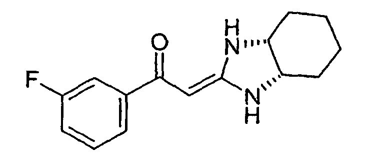

350 mg (1.44 mmol) 1-(3-fluorophenyl)-3,3-bis(methylsulfanyl)-2-propen-1-one and 250 mg (2.16 mmol) *cis*-1,2-diaminocyclohexane are dissolved in 25 ml toluene and refluxed for 24 h. Upon cooling a precipitate is formed which is collected by filtration and washed with diethyl ether to yield 230 mg (62% of th.) 2-[(*rac-cis*-3a,7a)-octahydro-2H-benzimidazol-2-ylidene]-1-(3-fluorophenyl)ethanone.

¹H-NMR (300MHz, DMSO-d₆): δ = 1.11 - 1.84 (m, 8H), 3.56 - 3.90 (m, 2H), 5.26 (s, 1H), 7.19 (m, 1H), 7.29-7.62 (m 3H + s, 1H, NH), 9.19 (s, 1H, NH).

### Example 8a

### 2-[(Rac-trans-3a,7a)-octahydro-2H-benzimidazol-2-ylidene]-1-(3-fluorophenyl)-ethanone

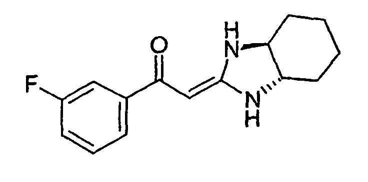

1.0 g (4.13 mmol) 1-(3-fluorophenyl)-3,3-bis(methylsulfanyl)-2-propen-1-one and 710 mg (6.2 mmol) *rac*-*trans*-1,2-diaminocyclohexane are dissolved in 25 ml toluene and refluxed for 24 h. Upon cooling a precipitate is formed which is collected by filtration and washed with diethyl ether to yield 839 mg (78% of th.) 2-[(*rac-trans*-3a,7a)-octahydro-2H-benzimidazol-2-ylidene]-1-(3-fluorophenyl)ethanone.

¹H-NMR (300MHz, DMSO-d₆): δ = 1.15 -1.56 (m, 4H), 1.75 (m, 2H), 2.02 (m, 1H), 2,17 (m, 1H), 2.88 - 3.14 (m, 2H), 5.33 (s, 1H), 7.21 (m, 2H), 7.34 - 7.5 (m, 2H), 7.5 - 7.65 (m, 1H + s, 1H, NH), 9.31 (s, 1H, NH).

In analogy to the procedure for example 8a the enantiomers are synthesized using enantiopure (1S,2S)-(+)-1,2-diaminocyclohexane and (1R,2R)-(-)-1,2-diaminocyclohexane, respectively.

2-[(3aS,7aS)-octahydro-2H-benzimidazol-2-ylidene]-1-(3-fluorophenyl)ethanone: yield: 62%, specific rotation [A]^{t}_{D} = -71.7° (methanol)

2-[(3aR,7aR)-octahydro-2H-benzimidazol-2-ylidene]-1-(3-fluorophenyl)ethanone: yield: 61 %, specific rotation [A]^{t}_{D} = +69.5° (methanol)

### Example 9a

### 2-[(Rac-cis-3a,7a)-octahydro-2H-benzimidazol-2-ylidene]-1-(4-fluorophenyl)-ethanone

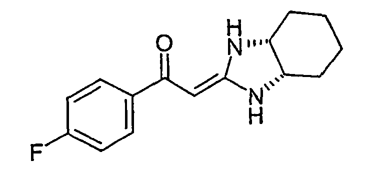

500 mg (2.1 mmol) 1-(4-fluorophenyl)-3,3-bis(methylsulfanyl)-2-propen-1-one and 350 mg (3.1 mmol) *cis*-1,2-diaminocyclohexane are dissolved in 25 ml toluene and refluxed for 24 h. Upon cooling a precipitate is formed which is collected by filtration and washed with diethyl ether to yield 160 mg (30% of th.) 2-[(*rac-cis*-3a,7a)-octahydro-2H-benzimidazol-2-ylidene]-1-(4-fluorophenyl)ethanone.

¹H-NMR (300MHz, DMSO-d₆): δ = 1.13 - 1.86 (m, 8H), 3.66 (m, 1H), 3.77 (m, 1H), 5.24 (s, 1H), 7.17 (m, 2H), 7.38 (br. s, 1H, NH), 7.76 (m, 2H), 9.17 (s, 1H, NH).

### Example 10a

### 2-[(Rac-trans-3a,7a)-octahydro-2H-benzimidazol-2-ylidene]-1-(4-fluorophenyl)-ethanone

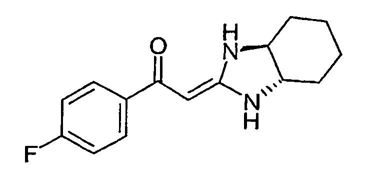

750 mg (3.1 mmol) 1-(4-fluorophenyl)-3,3-bis(methylsulfanyl)-2-propen-1-one and 530 mg (4.6 mmol) *rac-trans*-1,2-diaminocyclohexane are dissolved in 25 ml toluene and refluxed for 24 h. Upon cooling a precipitate is formed which is collected by filtration and washed with diethyl ether to yield 490 mg (58% of th.) 2-[(*rac-trans*-3a,7a)-octahydro-2H-benzimidazol-2-ylidene]-1-(4-fluorophenyl)ethanone.

¹H-NMR (300MHz, DMSO-d₆): δ = 1.2 - 1.5 (m, 4H), 1.74 (m, 2H), 2.0 (m, 1H), 2.16 (m, 1H), 2.88 - 3.1 (m, 2H), 5.31 (s, 1H), 7.18 (m, 2H), 7.55 (s, 1H, NH), 7.77 (m, 2H), 9.29 (br. s, 1H, NH).

### Example 11a

### 2-[(Rac-cis-3a,7a)-octahydro-2H-benzimidazol-2-ylidene]-1-(3-chlorophenyl)-ethanone

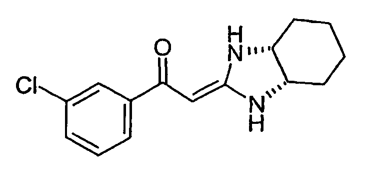

500 mg (1.9 mmol) 1-(3-chlorophenyl)-3,3-bis(methylsulfanyl)-2-propen-1-one and 330 mg (2.9 mmol) *cis*-1,2-diaminocyclohexane are dissolved in 25 ml toluene and refluxed for 24 h. Upon cooling a precipitate is formed which is collected by filtration and washed with diethyl ether to yield 240 mg (45% of th.) 2-[(*rac*-*cis*-3a,7a)-octahydro-2H-benzimidazol-2-ylidene]-1-(3-chlorophenyl)ethanone.

¹H-NMR (300MHz, DMSO-d₆): δ = 1.1 - 1.9 (m, 8H), 3.56 - 3.9 (m, 2H), 5.25 (s, 1H), 7.32 - 7.55 (m, 3H), 7.58 - 7.76 (m, 2H), 9.17 (s, 1H, NH).

### Example 12a

### 2-[(Rac-trans-3a,7a)-octahydro-2H-benzimidazol-2-ylidene]-1-(3-chlorophenyl)-ethanone

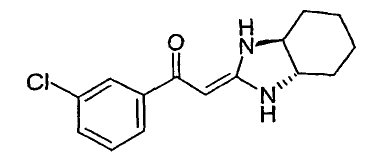

1.0 g (3.9 mmol) 1-(3-chlorophenyl)-3,3-bis(methylsulfanyl)-2-propen-1-one and 660 mg (5.8 mmol) *rac*-*trans*-1,2-diaminocyclohexane are dissolved in 25 ml toluene and refluxed for 24 h. Upon cooling a precipitate is formed which is collected by filtration and washed with diethyl ether to yield 705 mg (66% of th.) 2-[(*rac*-*trans*-3a,7a)-octahydro-2H-benzimidazol-2-ylidene]-1-(3-chlorophenyl)ethanone.

¹H-NMR (300MHz, DMSO-d₆): δ = 1.15 - 1.5 (m, 4H), 1.75 (m, 2H), 2.02 (m, 1H), 2.17 (m, 1H), 2.88 - 3.13 (m, 2H), 5.33 (s, 1H), 7.36 - 7.49 (m, 2H), 7.61 (s, 1H, NH), 7.63 - 7.73 (m, 2H), 9.29 (s, 1H, NH).

### Example 13a

### 2-[(Rac-cis-3a,7a)-octahydro-2H-benzimidazol-2-ylidene]-1-(4-chlorophenyl)-ethanone

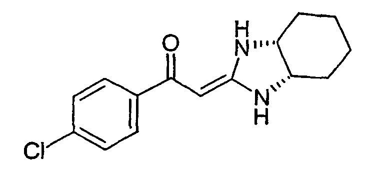

500 mg (1.9 mmol) 1-(4-chlorophenyl)-3,3-bis(methylsulfanyl)-2-propen-1-one and 330 mg (2.9 mmol) *cis*-1,2-diaminocyclohexane are dissolved in 25 ml toluene and refluxed for 24 h. Upon cooling a precipitate is formed which is collected by filtration and washed with diethyl ether to yield 315 mg (59% of th.) 2-[(*rac*-*cis*-3a,7a)-octahydro-2H-benzimidazol-2-ylidene]-1-(4-chlorophenyl)ethanone.

¹H-NMR (300MHz, DMSO-d₆): δ = 1.17 - 1.87 (m, 8H), 3.67 (m, 1H), 3.78 (m, 1H), 5.29 (s, 1H), 7.36 - 7.50 (m, 2H + s, 1H, NH), 7.67 - 7.77 (m, 2H), 9.18 (s, 1H, NH).

### Example 14a

### 2-[(Rac-trans-3a,7a)-octahydro-2H-benzimidazol-2-ylidene]-1-(4-chlorophenyl)-ethanone

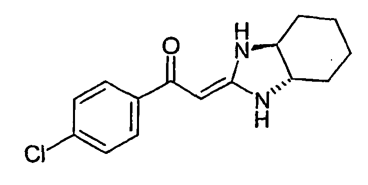

1.0 g (3.9 mmol) 1-(4-chlorophenyl)-3,3-bis(methylsulfanyl)-2-propen-1-one and 660 mg (5.8 mmol) *rac*-*trans*-1,2-diaminocyclohexane are dissolved in 25 ml toluene and refluxed for 24 h. Upon cooling a precipitate is formed which is collected by filtration and washed with diethyl ether to yield 816 mg (76% of th.) 2-[(*rac-trans*-3a,7a)-octahydro-2H-benzimidazol-2-ylidene]-1-(4-chlorophenyl)ethanone.

¹H-NMR (300MHz, CDCl₃): δ = 1.18 - 1.7 (m, 4H), 1.86 (m, 2H), 2.0 - 2.23 (m, 2H), 3.0 - 3.29 (m, 2H), 4.83 (s, 1H, NH), 5.39 (s, 1H), 7.33 (m, 2H), 7.76 (s, 1H, NH), 9.53 (s, 1H, NH).

### Example 15a

### 2-[(Rac-cis-3a,7a)-octahydro-2H-benzimidazol-2-ylidene]-1-(2-chlorophenyl)-ethanone

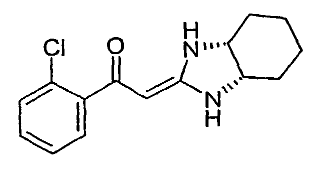

500 mg (1.9 mmol) 1-(2-chlorophenyl)-3,3-bis(methylsulfanyl)-2-propen-1-one and 330 mg (2.9 mmol) *cis*-1,2-diaminocyclohexane are dissolved in 25 ml toluene and refluxed for 24 h. Upon cooling a precipitate is formed which is collected by filtration and washed with diethyl ether to yield 354 mg (66% of th.) 2-[(*rac*-*cis*-3a,7a)-octahydro-2H-benzimidazol-2-ylidene]-1-(2-chlorophenyl)ethanone.

¹H-NMR (300MHz, DMSO-d₆): δ = 1.1 - 1.85 (m, 8H), 3.66 (m, 1H), 3.78 (m, 1H), 4.74 (s, 1H), 7.29 (m, 2H), 7.36 (m, 2H), 7.45 (s, 1H, NH), 8.93 (s, 1H, NH).

### Example 16a

### 2-[(Rac-trans-3a,7a)-octahydro-2H-benzimidazol-2-ylidene]-1-(2-chlorophenyl)-ethanone

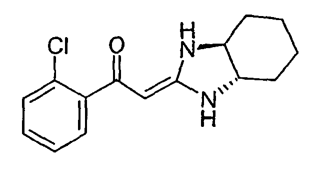

1.0 g (3.9 mmol) 1-(2-chlorophenyl)-3,3-bis(methylsulfanyl)-2-propen-1-one and 660 mg (5.8 mmol) *rac-trans*-1,2-diaminocyclohexane are dissolved in 25 ml toluene and refluxed for 24 h. Upon cooling a precipitate is formed which is collected by filtration and washed with diethyl ether to yield 881 mg (82% of th.) 2-[(*rac-trans*-3a,7a)-octahydro-2H-benzimidazol-2-ylidene]-1-(2-chlorophenyl)ethanone.

¹H-NMR (300MHz, DMSO-d₆): δ = 1.14 - 1.53 (m, 4H), 1.74 (m, 2H), 2.0 (m, 1H), 2.18 (m, 1H), 2.87 - 3.12 (m, 2H), 4.80 (s, 1H), 7.24 - 7.42 (m, 4H), 7.62 (s, 1H, NH), 9.07 (s, 1H, NH).

### Example 17a

### 1-(2-Fluorophenyl)-3,3-bis(methylsulfanyl)-2-propen-1-one

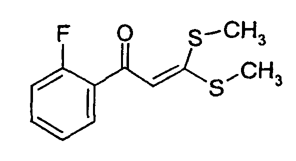

10.6 g (76.5 mmol) 1-(2-fluorophenyl)ethanone, 6.12 g (60%, 153 mmol) sodium hydride and 6.9 ml (115 mmol) carbon disulfide are dissolved in 250 ml toluene. At room temperature 20 ml *N*,*N*-dimethylformamide are added dropwise during 30 min. The mixture is cooled in an ice bath, and after dropwise addition of 14.3 ml (230 mmol) methyl iodide stirred for 1 h in an ice bath. The mixture is diluted with 100 ml toluene and carefully poured on ice water. The organic layer is separated, dried over magnesium sulfate, filtered and concentrated under vacuum. The crude is purified over preparative MPLC (Biotage silica-column, eluent: cyclohexane-ethyl acetate gradient) to yield 6.1 g (29% of th. 88% purity) 1-(2-fluorophenyl)-3,3-bis(methylsulfanyl)-2-propen-1-one. The product is washed with 2-propanol used without further purification.

LC-MS (MHZ2Q): Rₜ = 4.28 min.

### Example 18a

### 2-[(Rac-cis-3a,7a)-octahydro-2H-benzimidazol-2-ylidene]-1-(2-fluorophenyl)-ethanone

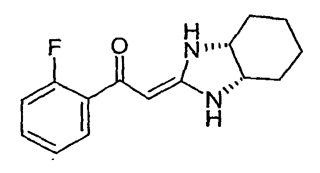

500 mg (1.8 mmol) of the compound of example 17a and 311 mg (2.7 mmol) *cis-1,2*-diaminocyclohexane are dissolved in 25 ml toluene and refluxed for 24 h. Upon cooling a precipitate is formed which is collected by filtration and washed with diethyl ether to yield 422 mg (89% of th.) 2-[(*rac-cis*-3a,7a)-octahydro-2H-benzimidazol-2-ylidene]-1-(2-fluorophenyl)ethanone.

¹H-NMR (300MHz, DMSO-d₆): δ = 1.07 - 1.95 (m, 8H), 3.53 - 3.93 (m, 2H), 5.13 (s, 1H), 7.02 - 7.27 (m, 2H), 7.29 - 7.45 (m, 1H), 7.54 (s, 1H, NH), 7.68 (m, 1H), 9.11 (s, 1H, NH).

### Example 19a

### 2-[(Rac-trans-3a,7a)-octahydro-2H-benzimidazol-2-ylidene]-1-(2-fluorophenyl)-ethanone

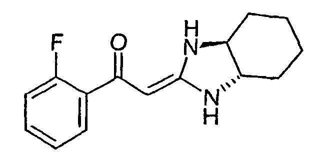

500 mg (1.8 mmol) of the compound of example 17a and 311 mg (2.7 mmol) *rac-trans*-1,2-diaminocyclohexane are dissolved in 25 ml toluene and refluxed for 24 h. Upon cooling a precipitate is formed which is collected by filtration and washed with diethyl ether to yield 330 mg (68% of th.) 2-[(*rac-trans*-3a,7a)-octahydro-2H-benzimidazol-2-ylidene]-1-(2-fluorophenyl)ethanone.

¹H-NMR (300MHz, DMSO-d₆): δ = 1.1 - 1.57 (m, 4H), 1.58 - 1.87 (m, 2H), 2.0 (m, 1H), 2.17 (m, 1H), 2.80 - 3.17 (m, 2H), 5.19 (s, 1H), 7.05 - 7.29 (m, 2H), 7.30 - 7.48 (m, 1H), 7.55 - 7.81 (s, 1H, NH + m, 1H), 9.23 (s, 1H, NH).

### Example 20a

### 2-[(Rac-cis-3a,7a)-octahydro-2H-benzimidazol-2-ylidene]-1-(3-bromophenyl)-ethanone

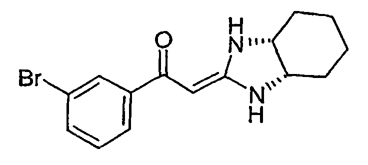

500 mg (1.7 mmol) 1-(3-bromophenyl)-3,3-bis(methylsulfanyl)-2-propen-1-one and 280 mg (2.5 mmol) *cis*-1,2-diaminocyclohexane are dissolved in 25 ml toluene and refluxed for 24 h. Upon cooling a precipitate is formed which is collected by filtration and washed with diethyl ether to yield 400 mg (76% of th.) 2-[(*rac*-*cis*-3a,7a)-octahydro-2H-benzimidazol-2-ylidene]-1-(3-bromophenyl)ethanone.

¹H-NMR (300MHz, DMSO-d₆): δ = 1.1 - 1.9 (m, 8H), 3.55 - 3.91 (m, 2H), 5.24 (s, 1H), 7.34 (m, 1H), 7.45 (s, 1H, NH), 7.56 (m, 1H), 7.69 (m, 1H), 7.84 (m, 1H), 9.15 (s, 1H, NH).

### Example 21a

### 2-[(Rac-trans-3a,7a)-octahydro-2H-benzimidazol-2-ylidene)-1-(3-bromophenyl)-ethanone

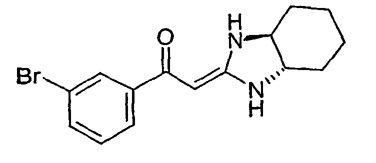

1.0 g (3.9 mmol) 1-(3-bromophenyl)-3,3-bis(methylsulfanyl)-2-propen-1-one and 660 mg (5.8 mmol) *rac-trans*-1,2-diaminocyclohexane are dissolved in 25 ml toluene and refluxed for 24 h. Upon cooling a precipitate is formed which is collected by filtration and washed with diethyl ether to yield 870 mg (82% of th.) 2*-*[(*rac-trans*-3a,7a)-octahydro-2H-benzimidazol-2-ylidene]-1-(3-bromophenyl)ethanone.

¹H-NMR (300MHz, DMSO-d₆): δ = 1.17 - 1.5 (m, 4H), 1.75 (m, 2H), 2.01 (m, 1H), 2.17 (m, 1H), 2.85 - 3.13 (m, 2H), 5.32 (s, 1H), 7.35 (m, 1H), 7.58 (m, 1H), 7.61 (s, 1H, NH), 7.70 (m, 1H), 7.84 (m, 1H), 9.28 (s,1H, NH).

### Example 22a

### 2-[(Rac-cis-3a,7a)-octahydro-2H-benzimidazol-2-ylidene]-1-(2-methylphenyl)-ethanone

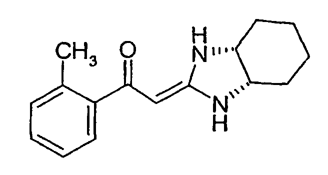

500 mg (2.1 mmol) 1-(2-methylphenyl)-3,3-bis(methylsulfanyl)-2-propen-1-one and 360 mg (3.1 mmol) *cis*-1,2-diaminocyclohexane are dissolved in 25 ml toluene and refluxed for 24 h. Upon cooling a precipitate is formed which is collected by filtration and washed with diethyl ether to yield 300 mg (55% of th.) 2-[(*rac-cis*-3a,7a)-octahydro-2H-benzimidazol-2-ylidene]-1-(2-methylphenyl)ethanone.

¹H-NMR (300MHz, DMSO-d₆): δ = 1.2 - 1.83 (m, 8H), 2.32 (s, 3H), 3.63 (m, 1H), 3.77 (m, 1H), 4.74 (s, 1H), 7.05 - 7.27 (m, 4H), 7.31 (s, 1H, NH), 9.04 (s, 1H, NH).

### Example 23a

### 2-[(Rac-trans-3a,7a)-octahydro-2H-benzimidazol-2-ylidene]-1-(2-methylphenyl)-ethanone

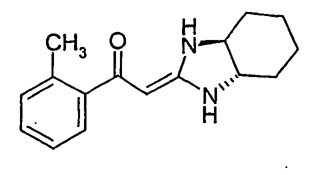

1.0 g (4.2 mmol) 1-(2-methylphenyl)-3,3-bis(methylsulfanyl)-2-propen-1-one and 720 mg (6.3 mmol) *rac*-*trans*-1,2-diaminocyclohexane are dissolved in 25 ml toluene and refluxed for 24 h. Upon cooling a precipitate is formed which is collected by filtration and washed with diethyl ether to yield 303 mg (28% of th.) 2-[(*rac*-*trans*-3a,7a)-octahydro-2H-benzimidazol-2-ylidene]-1-(2-methylphenyl)ethanone.

¹H-NMR (300MHz, CDCl₃): δ = 1.2 - 1.71 (m, 4H), 1.85 (m, 2H), 2.04 (m, 1H), 2.15 (m, 1H), 2.44 (s, 3H), 2.97 - 3.29 (m, 2H), 4.79 (s, 1H, NH), 5.02 (s, 1H), 7.07 - 7.23 (m, 3H), 7.34 (m, 1H), 9.43 (s, 1H, NH).

### Example 24a

### (2Z)-1-(4-Chlorophenyl)-2-(4,4-dimethyl-2-imidazolidinylidene)ethanone

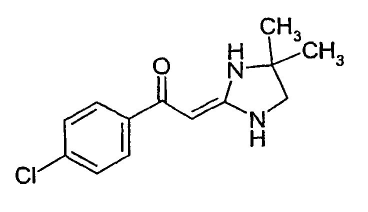

734 mg (2.84 mmol) 1-(4-chlorophenyl)-3,3-bis(methylsulfanyl)-2-propen-1-one and 300 mg 3.4 mmol) 2-methyl-1,2-propanediamine are dissolved in 4 ml toluene and refluxed for 16 h. The solvent is removed in vacuo and the crude product purified by column chromatography (silica, eluent: dichloro methane : methanol 40 : 1) to yield 559 mg (79% of th.) (2Z)-1-(4-chlorophenyl)-2-(4,4-dimethyl-2-imidazolidinylidene)ethanone.

¹H-NMR (300MHz, DMSO-d₆): δ = 1.42 (s, 3H), 1.69 (s, 3H), 3.34 (s, 1H), 3.50 (s, 1H), 4.55 (d, 1H), 4.26 (d, 1H), 7.32 (m, 2H), 7.75 (dd, 2H), 9.53 (br. d, 1H).

### Example 25a

### 2-(5,5-Dimethyltetrahydro-2(1H)-pyrimidinylidene)-1-phenylethanone

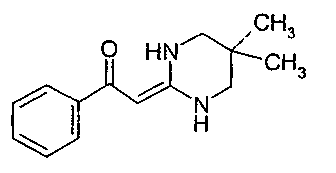

210 mg (0.94 mmol) of 3,3-bis(methylsulfanyl)-1-phenyl-2-propen-1-one and 110 mg (1.12 mmol) 1,3-diamino-2,2-dimethylpropane are dissolved in 15 ml toluene and refluxed for 24 h. Upon cooling a precipitate is formed which is collected by filtration and washed with diethyl ether to yield 148 mg (68% of th.) 2-(5,5-dimethyltetrahydro-2(1H)-pyrimidinylidene)-1-phenylethanone.

¹H-NMR (200MHz, CDCl₃): δ = 1.09 (s, 6H), 2.93 - 3.17 (m, 4H), 4.72 (br. s, 1H, NH), 5.11 (s, 1H), 7.35 (m, 3H), 7.80 (m, 2H), 11.45 (br. s, 1H, NH).
LC-MS (B): Rₜ =1.13 min, MS (ESIpos): m/z = 231 (M+H)⁺.

### Example 26a

### 1-(4-Chlorophenyl)-2-(5,5-dimethyltetrahydro-2(1H)-pyrimidinylidene)ethanone

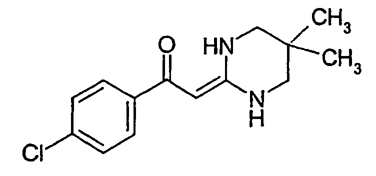

200 mg (0.77 mmol) of 1-(4-chlorophenyl)-3,3-bis(methylsulfanyl)-2-propen-1-one and 95 mg (0.93 mmol) 1,3-diamino-2,2-dimethylpropane are dissolved in 15 ml toluene and refluxed for 24 h. Upon cooling a precipitate is formed which is collected by filtration and washed with diethyl ether to yield 136 mg (67% of th.) 1-(4-chlorophenyl)-2-(5,5-dimethyltetrahydro-2(1H)-pyrimidinylidene)ethanone.

¹H-NMR (200MHz, CDCl₃): δ = 1.09 (s, 6H), 2.93 - 3.17 (m, 4H), 4.76 (br. s, 1H, NH), 5.06 (s, 1H), 7.31 (d, 2H), 7.73 (d, 2H), 11.38 (br. s, 1H, NH).
LC-MS (B): Rₜ = 2.22 min, MS (ESIpos). m/z = 265 (M+H)⁺.

### Example 27a

### 1-(4-Fluorophenyl)-2-(5,5-dimethyltetrahydro-2(1H)-pyrimidinylidene)ethanone

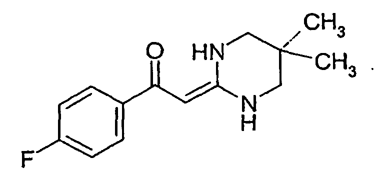

500 mg (2.06 mmol) of 1-(4-fluorophenyl)-3,3-bis(methylsulfanyl)-2-propen-1-one and 253 mg (2.78 mmol) 1,3-diamino-2,2-dimethylpropane are dissolved in 15 ml toluene and refluxed for 24 h. Upon cooling a precipitate is formed which is collected by filtration and washed with diethyl ether to yield 464 mg (91% of th.) 1-(4-fluorophenyl)-2-(5,5-dimethyltetrahydro-2(1H)-pyrimidinylidene)ethanone.

¹H-NMR (200MHz, CDCl₃): δ = 1.09 (s, 6H), 2.93 - 3.17 (m, 4H), 4.70 (br. s, 1H, NH), 5.06 (s, 1H), 7.02 (m, 2H), 7.78 (m, 2H), 11.37 (br. s, 1H, NH).
LC-MS (B): Rₜ = 1.39 min, MS (ESIpos): m/z = 248 (M+H)⁺.

### Example 28a

### (2E/Z)-2-(4-Methyloctahydro-2H-cyclopenta[d]pyrimidin-2-ylidene)-1-phenylethanone

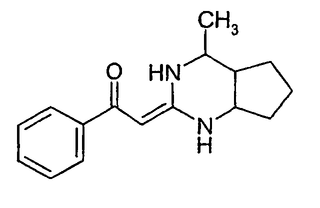

250 mg (1.11 mmol) of 3,3-bis(methylsulfanyl)-1-phenyl-2-propen-1-one and 171 mg (1.34 mmol) 2-(aminomethyl)cyclopentanamine are dissolved in 15 ml toluene. Lithium-bis(trimethylsilyl) amide (2,23 mL of IN solution in THF) is added and the mixture is refluxed for 24 h. The mixture is cooled to room temperature, concentrated under vacuum and dissolved in ethyl acetate. The organic phase is washed with saturated sodium hydrogen carbonate solution and water, dried over magnesium sulfate, filtered and concentrated under vacuum. The crude is purified over preparative HPLC (RP18-column, eluent: acetonitrile-water gradient) to yield 150 mg (53% of th.) (2*E*/*Z*)-2-(4-methyloctahydro-2H-cyclopenta[d]pyrimidin-2-ylidene)-1-phenylethanone. The mixture of stereo- and regio isomers is used in the next step without further separation.
LC-MS (B): 1) Rₜ = 1.65 min, MS (ESIpos): m/z = 256 (M+H)⁺, 2) Rₜ = 1.94 min, MS (ESIpos): m/z = 256 (M+H)⁺.

### Example 29a

### (2E/Z)-1-(4-Fluorophenyl)-2-(4-methyloctahydro-2H-cyclopenta[d]pyrimidin-2-ylidene)ethanone

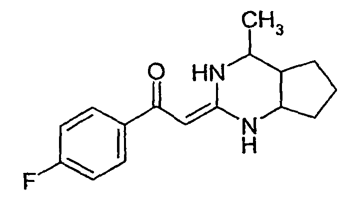

1.0 g (4.13 mmol) of 1-(4-fluorophenyl)-3,3-bis(methylsulfanyl)-2-propen-1-one and 635 mg (4.95 mmol) 2-(aminomethyl)cyclopentanamine are dissolved in 25 ml toluene and refluxed for 48 h. Upon cooling a precipitate is formed which is collected by filtration and washed with diethyl ether to yield 1.1 g (90% of th.) (2*E*/*Z*)-1-(4-fluorophenyl)-2-(4-methyloctahydro-2H-cyclopenta[d]pyrimidin-2-ylidene)ethanone. The mixture of stereo- and regio isomers is used in the next step without further separation.
LC-MS (A): 1) Rₜ = 0.63 min, MS (ESIpos): m/z = 275 (M+H)⁺, 2) Rₜ = 1.67 min, MS (ESIpos): m/z = 275 (M+H)⁺.

### Example 30a

### (rac)-1-(4-Fluorophenyl)-2-(5-hydroxytetrahydro-2(1H)-pyrimidinylidene)ethanone

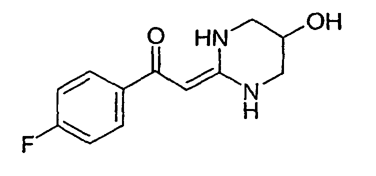

500 mg (2.06 mmol) of 1-(4-fluorophenyl)-3,3-bis(methylsulfanyl)-2-propen-1-one and 205 mg (2.27 mmol) 1,3-diamino-2-propanol are dissolved in 15 ml toluene and refluxed for 24 h. Upon cooling a precipitate is formed which is collected by filtration and washed with diethyl ether to yield 454 mg (91% of th.) (*rac*)-1-(4-fluorophenyl)-2-(5-hydroxytetrahydro-2(1H)-pyrimidinylidene)ethanone.

¹H-NMR (200MHz, DMSO-d₆): δ = 2.9 - 3.5 (m, br. 4H), 3.98 (m, 1H), 5.05 (s, 1H), 5.23 (s, br., 1H, OH), 7.12 (m, 2H), 7.27 (s, br., 1H, NH) 7.68 (m, 2H), 11.05 (br. s, 1H,NH).
LC-MS (B): Rₜ = 0.72 min, MS (ESIpos): m/z = 237 (M+H)⁺.

### Example 31a

### (Rac)-9-(4-fluorobenzoyl)-3-hydroxy-1,2,3,4-tetrahydro-6H-pyrido[1,2-a]pyrimidin-6-one

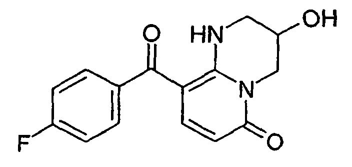

200 mg (2.88 mmol) propiolic acid and 560 mg (3.46 mmol) 1,1-carbonyldiimidazol are dissolved in 25 ml tetrahydrofuran and stirred during 1 h at room temperature. 450 mg (1.92 mmol) of the compound of example 30a are added and the mixture is heated to reflux during 24 h. The mixture is cooled to room temperature, concentrated under vacuum and dissolved in ethyl acetate. The organic phase is washed with saturated sodium hydrogencarbonate solution and water, dried over magnesium sulfate, filtered and concentrated under vacuum. The crude is purified over preparative HPLC (RP18-column, eluent: acetonitrile-water gradient) to yield 205 mg (35% of th.) (*rac*)-9-(4-fluorobenzoyl)-3-hydroxy-1,2,3,4-tetrahydro-6H-pyrido[1,2-a]pyrimidin-6-one.

¹H-NMR (200MHz, DMSO-d₆): δ = 3.41 (m, 1H), 3.56 (m, 2H), 4.19 (m, 1H), 4.29 (m, 1H), 5.42 (d, br, 1H), 5.57 (d, 1H), 7.24 - 7.38 (d, 1H + m, 2H), 7.50 (m, 2H), 10.92 (s, br, 1H, NH).
LC-MS (B): Rₜ = 3.10 min, MS (ESIpos): m/z = 289 (M+H)⁺.

### Example 32a

### (2E/Z)-2-(4-ethyltetrahydro-2(1H)-pyrimidinylidene)-1-(4-fluorophenyl)ethanone

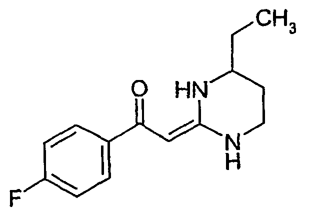

536 mg (2.21 mmol) of 1-(4-fluorophenyl)-3,3-bis(methylsulfanyl)-2-propen-1-one and 251 mg (2.65 mmol) 1,3-diaminopentane are dissolved in 15 ml toluene and refluxed for 24 h. Upon cooling a precipitate is formed which is collected by filtration and washed with diethyl ether to yield 540 mg (98% of th.) (2*E*/*Z*)-2-(4-ethyltetrahydro-2(1H)-pyrimidinylidene)-1-(4-fluorophenyl)ethanone.
LC-MS (A): Rₜ = 1.30 min, MS (ESIpos): m/z = 249 (M+H)⁺.

The following examples have been prepared according to the abovementioned procedure of Example 1A:

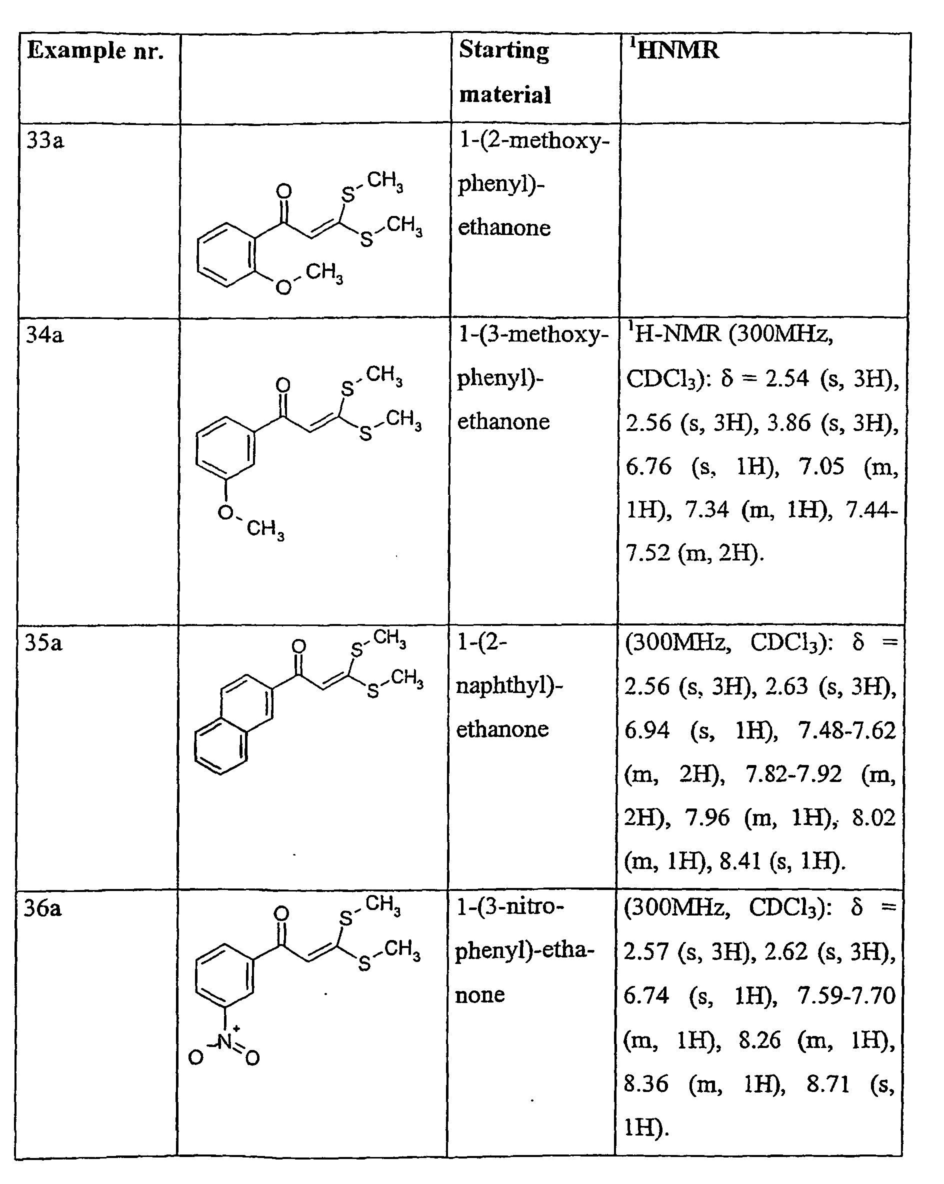

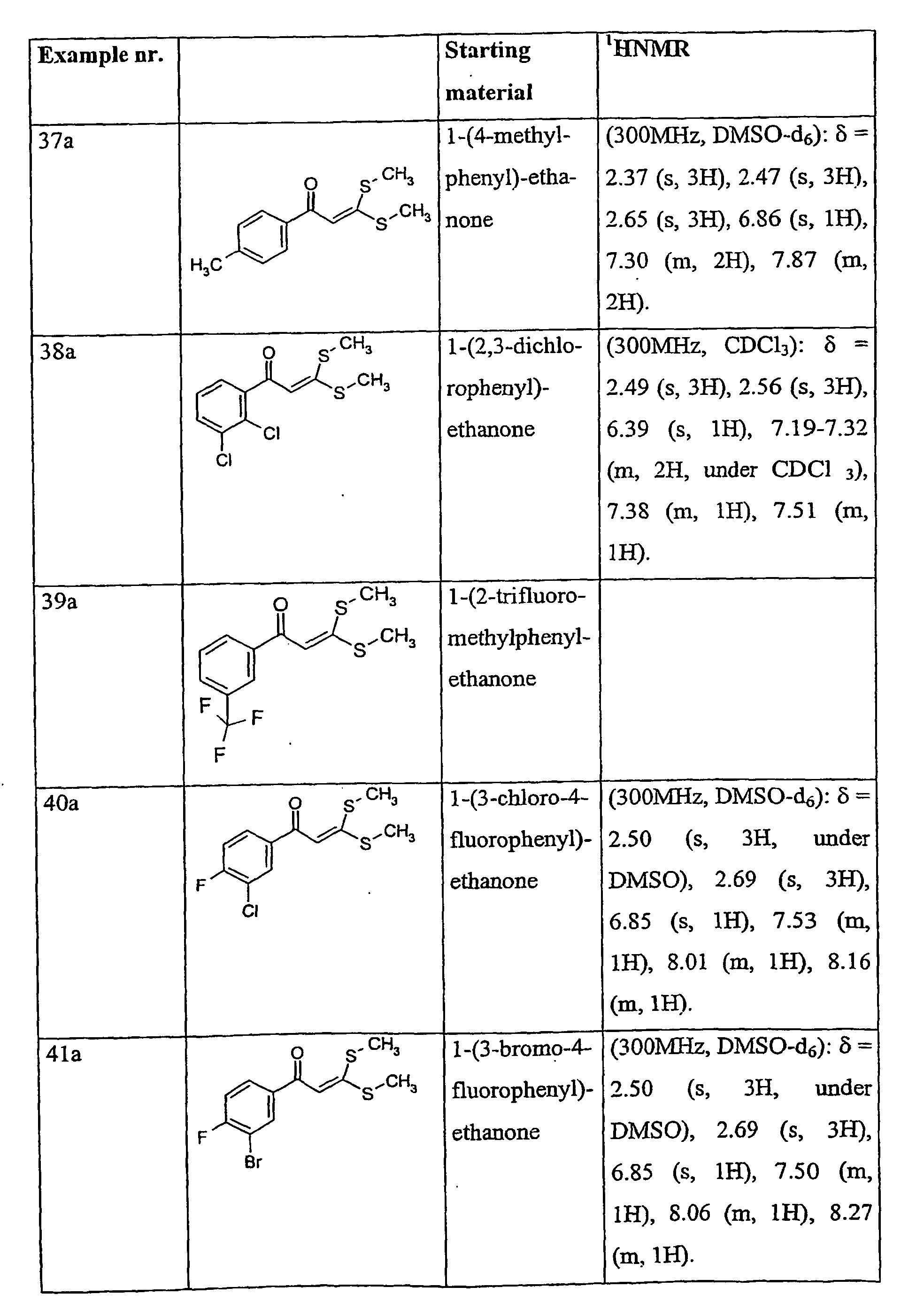

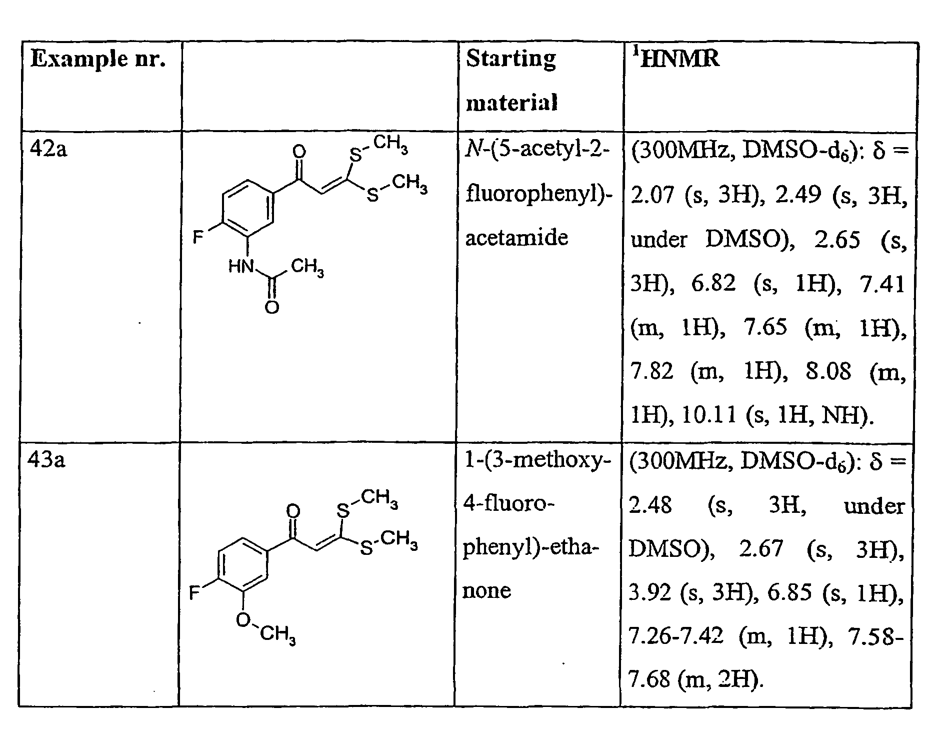

The following examples have been prepared according to the abovementioned procedures of examples 2A, 3A and 26A:

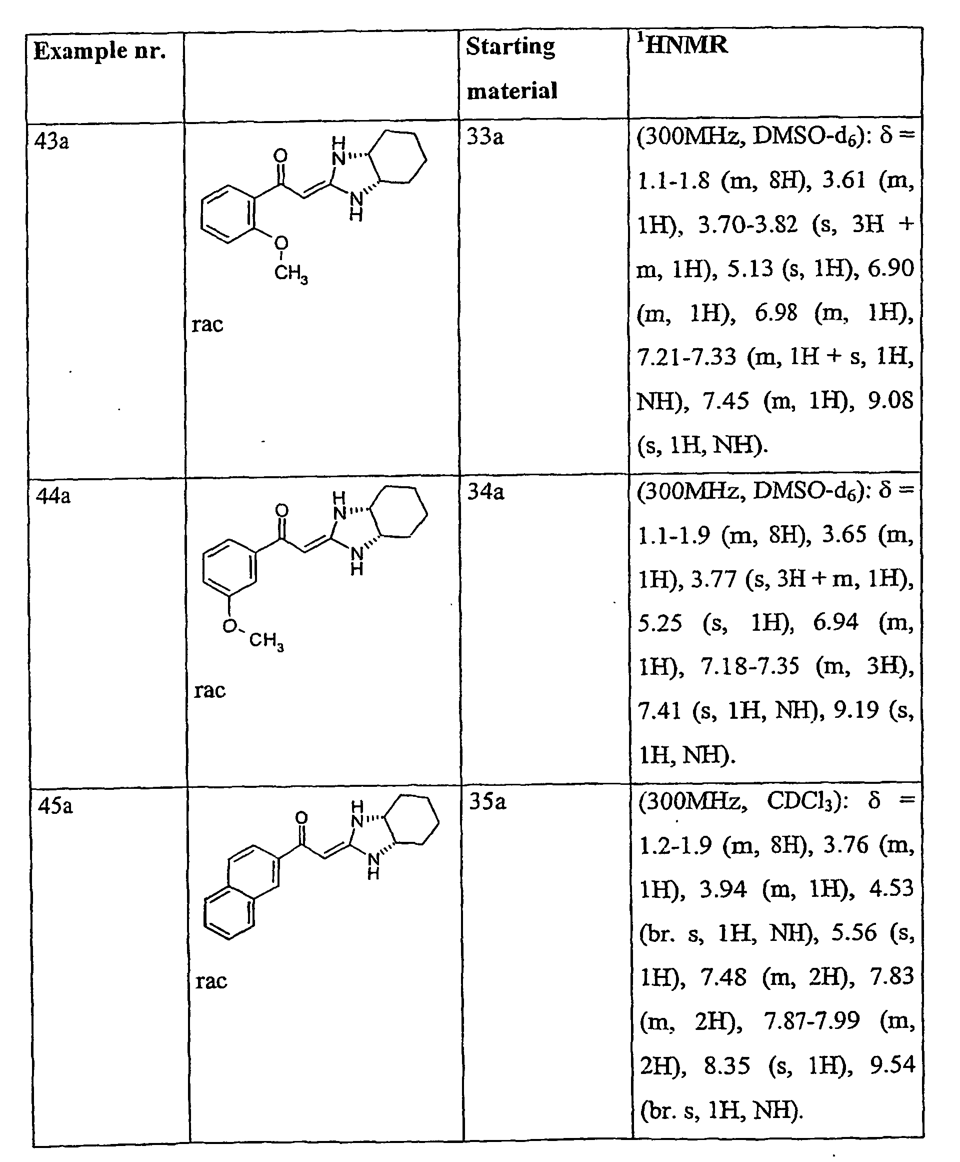

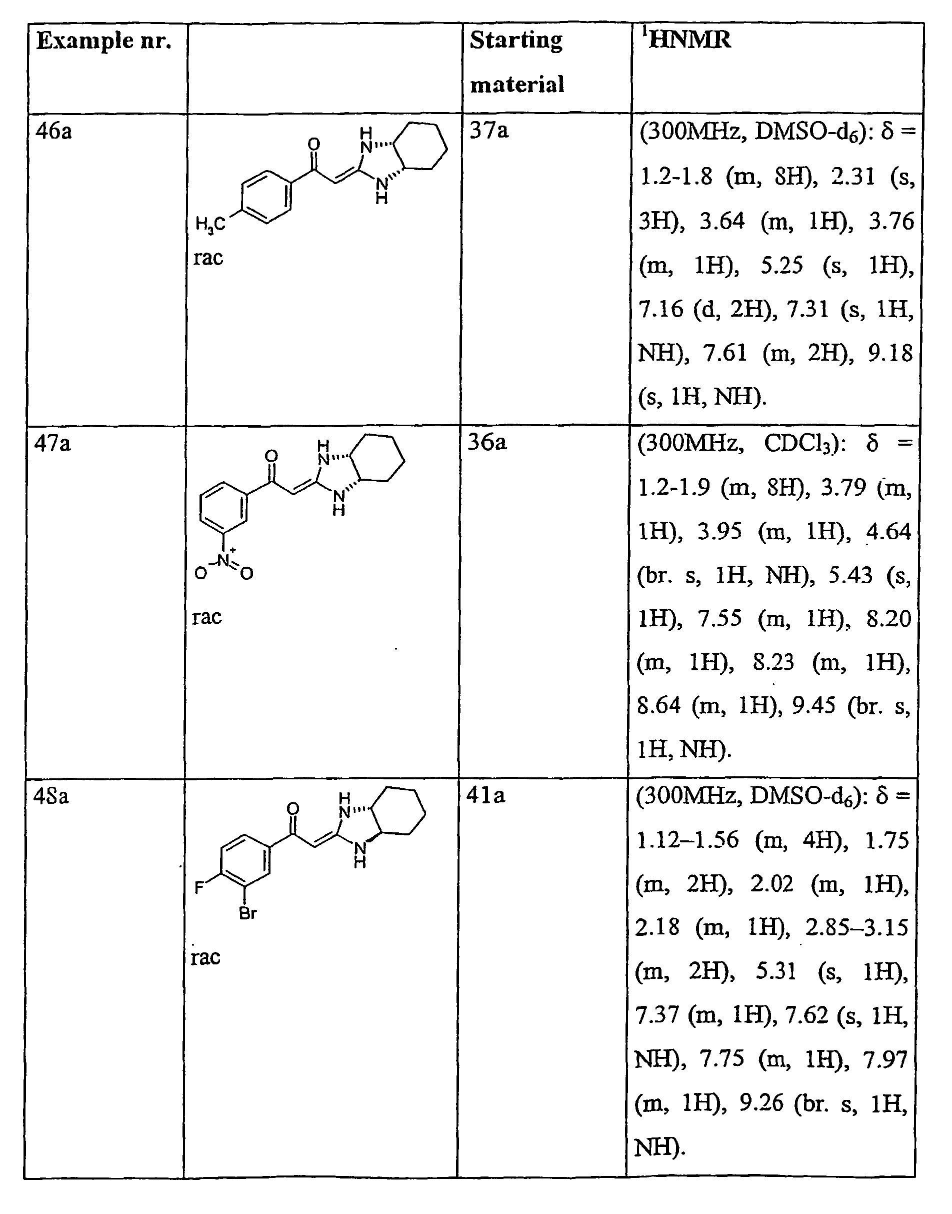

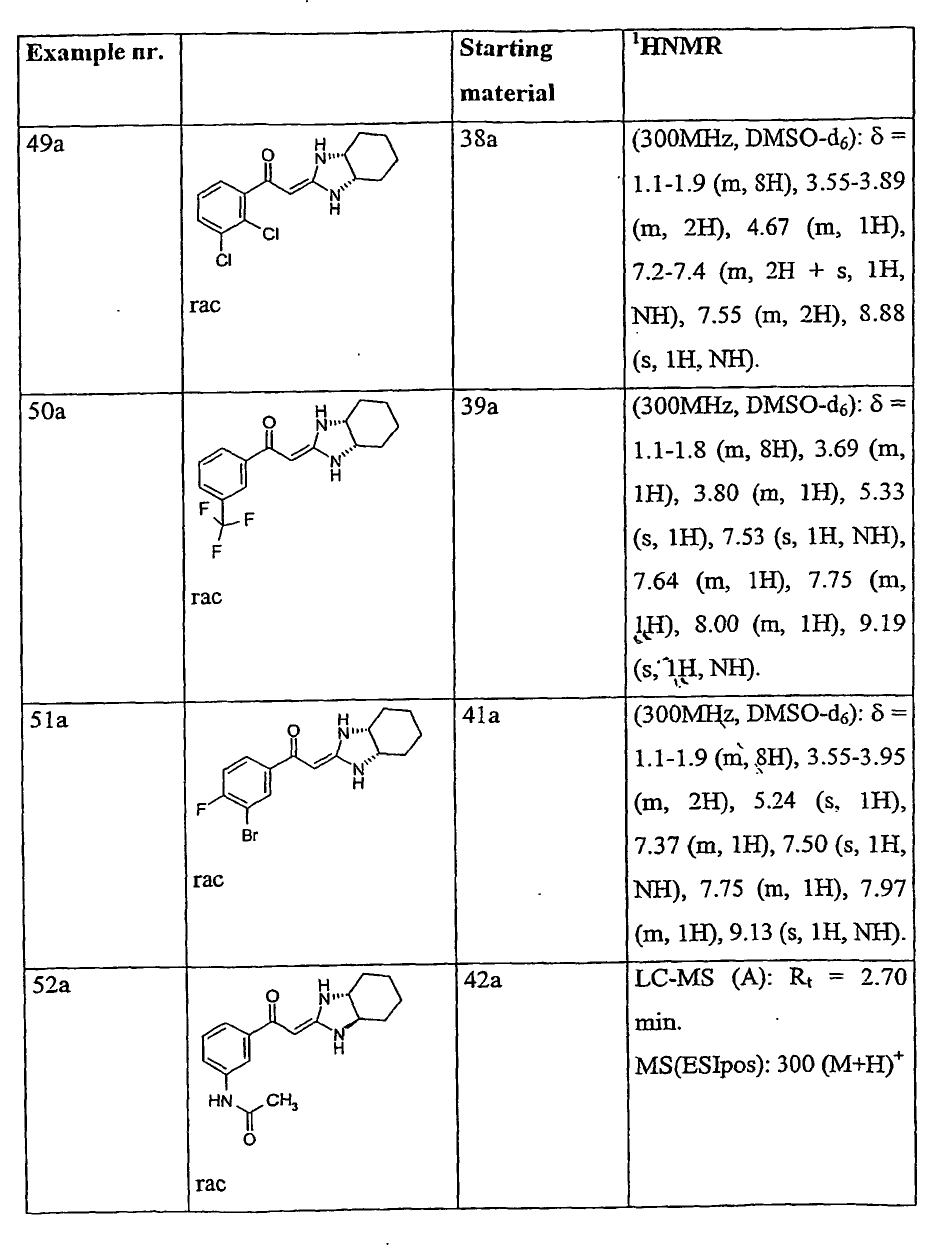

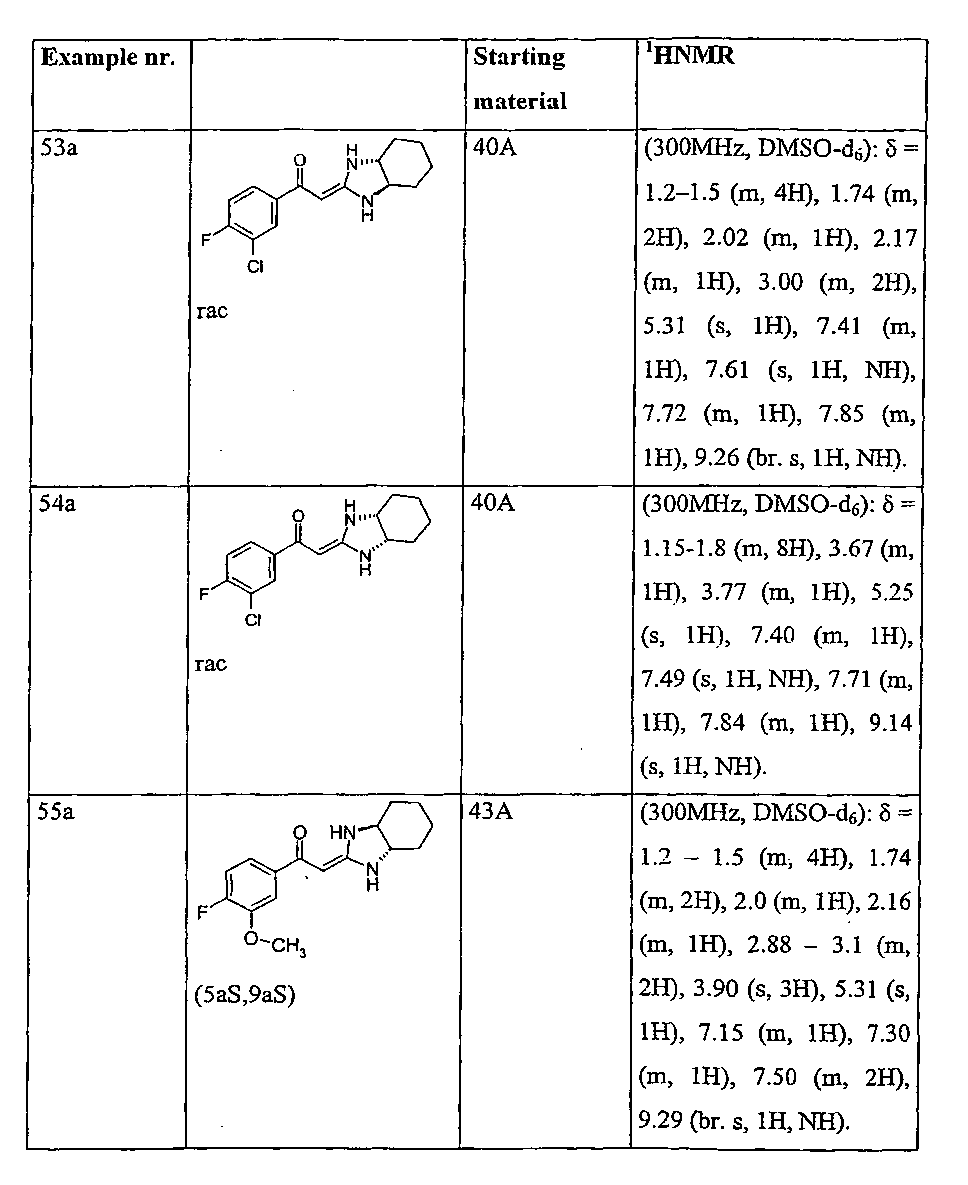

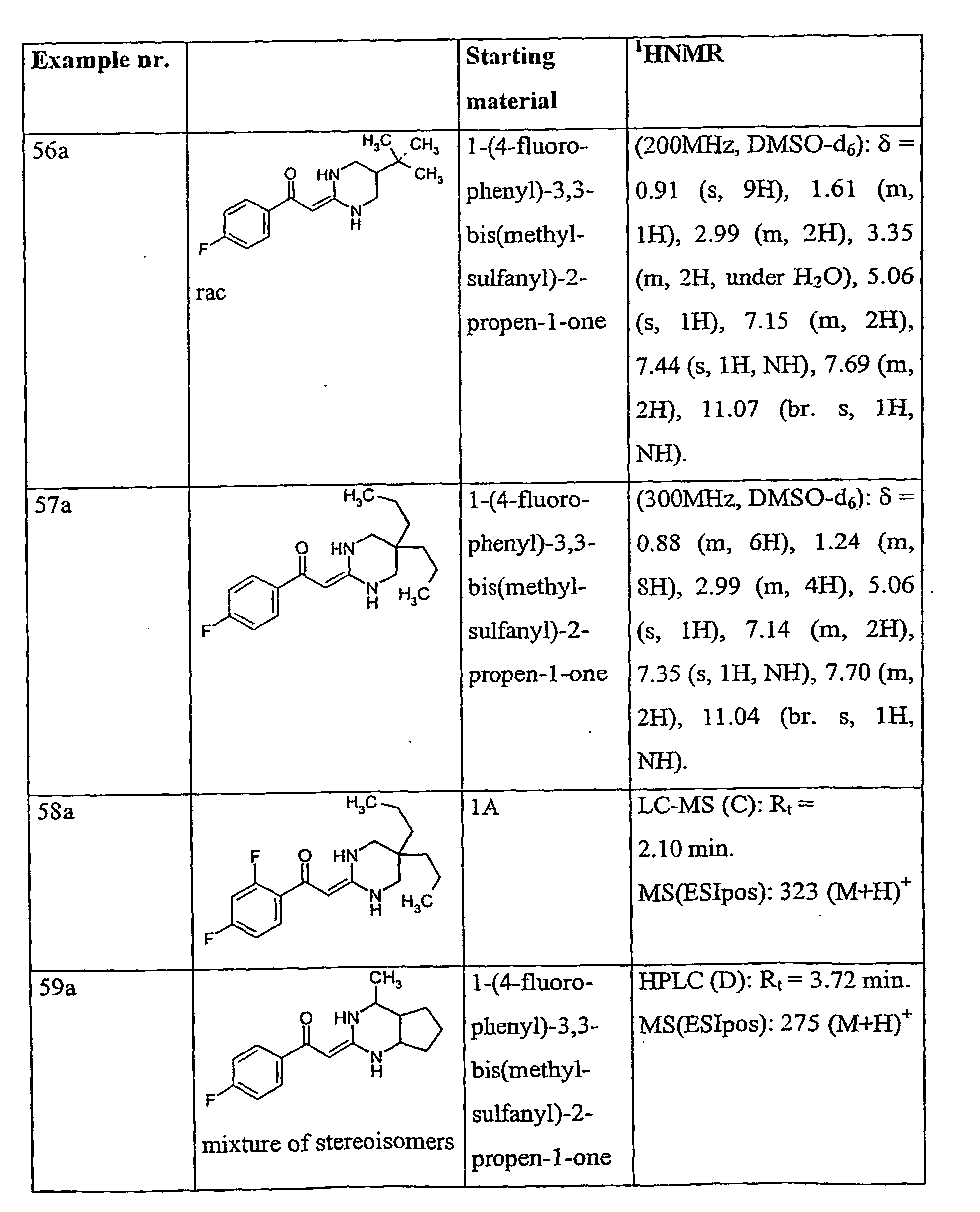

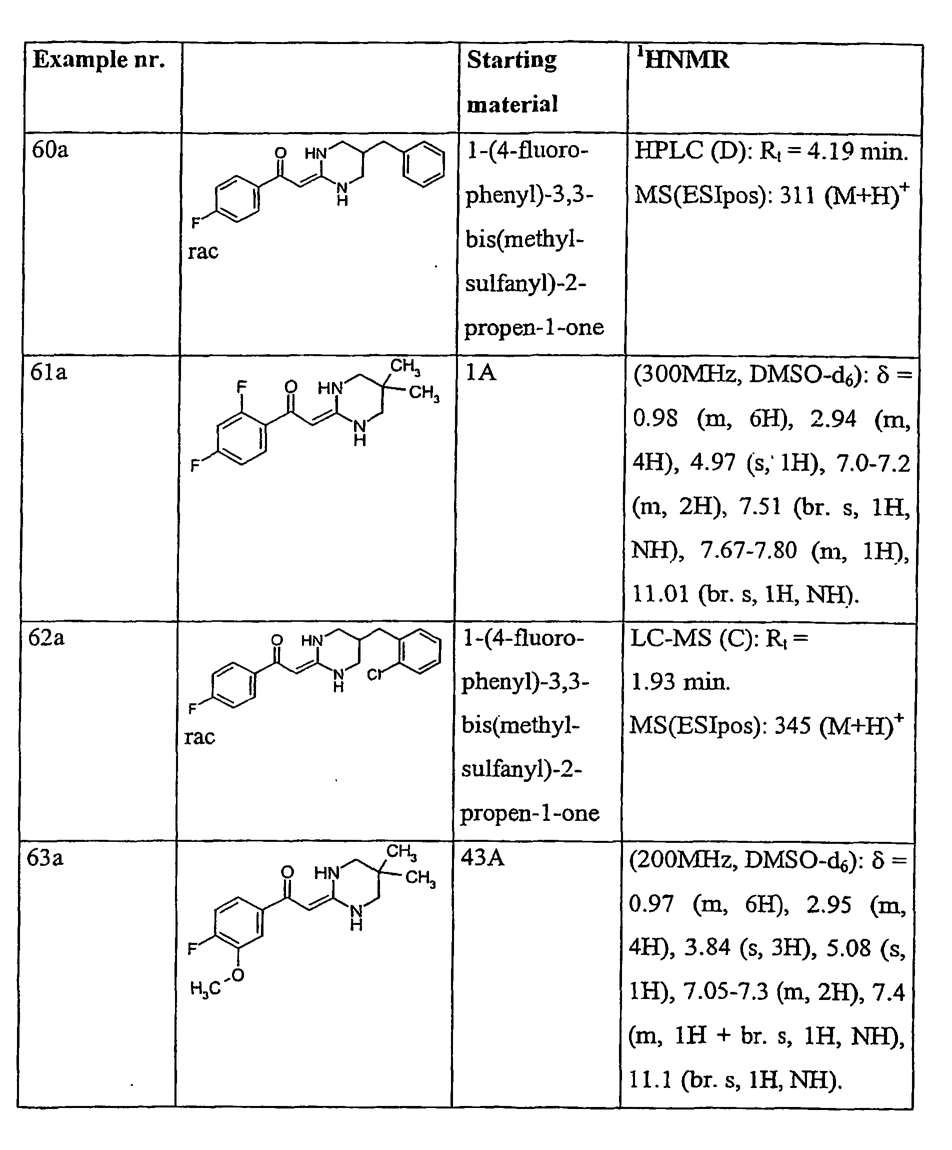

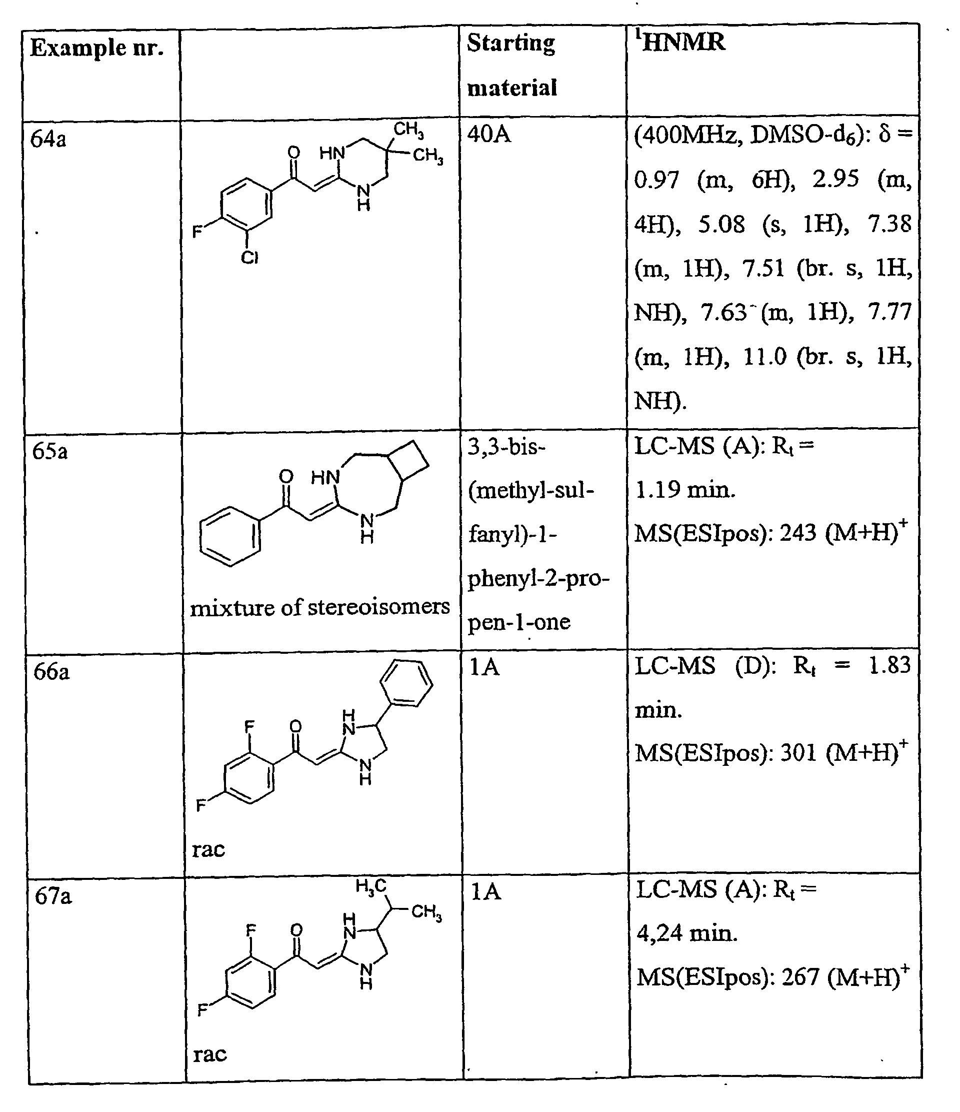

### Preparative Examples

### Example 1

### (Rac-cis-5a,9a)-4-(2,4-difluorobenzoyl)-5a,6,7,8,9,9a-hexahydropyrido[1,2-a]benzimidazol-1(5H)-one

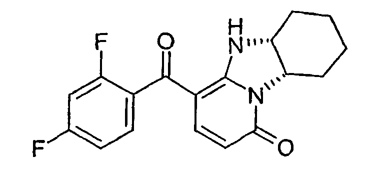

85 mg (1.2 mmol) propiolic acid and 230 mg (1.4 mmol) 1,1-carbonyldiimidazol are dissolved in 30 ml tetrahydrofuran and stirred during 1h at room temperature. 223 mg (0.8 mmol) of the compound of example 2a are added and the mixture is heated to reflux during 24h. The mixture is cooled to room temperature, concentrated under vacuum and dissolved in ethyl acetate. The organic phase is washed with saturated sodium hydrogencarbonate solution and water, dried over magnesium sulfate, filtered and concentrated under vacuum. The crude is purified over preparative HPLC (RP18-column, eluent: acetonitrile-water gradient) to yield 100 mg (44% of th.) (*rac-cis*-5a,9a)-4-(2,4-difluorobenzoyl)-5a,6,7,8,9,9a-hexahydropyrido[1,2-a]-benzimidazol-1(5H)-one.

¹H-NMR (300MHz, DMSO-d₆): δ = 1.2 - 1.8 (m, 5H), 1.85 (m, 1H), 2.11 (m, 1H), 2.43 (m, 1H), 4.23 (m, 1H), 4.63 (m, 1H), 5.75 (d, 1H), 6.90 (m, 1H), 6.98 (m, 1H), 7.20 (d, 1H) 7.39 (m, 1H), 8.40 (s, 1H, NH).

### Example 2

### (Rac-trans-5a,9a)-4-(2,4-difluorobenzoyl)-5a,6,7,8,9,9a-hexahydropyrido[1,2-a]benzimidazol-1(5H)-one

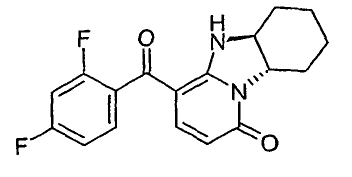

85 mg (1.2 mmol) propiolic acid and 230 mg (1.4 mmol) 1,1-carbonyldiimidazol are dissolved in 30 ml tetrahydrofuran and stirred during 1h at room temperature. 223 mg (0.8 mmol) of the compound of example 3a are added and the mixture is heated to reflux during 24h. The mixture is cooled to room temperature, concentrated under vacuum and dissolved in ethyl acetate. The organic phase is washed with saturated sodium hydrogencarbonate solution and water, dried over magnesium sulfate, filtered and concentrated under vacuum. The crude is purified over preparative HPLC (RP18-column, eluent: acetonitrile - water gradient) to yield 172 mg (65% of th.) (*rac*-*trans*-5a,9a)-4-(2,4-difluorobenzoyl)-5a,6,7,8,9,9a-hexahydropyrido[1,2-a]benzimidazol-1(5H)-one.

¹H-NMR (300MHz, DMSO-d₆): δ = 1.15 - 1.95 (m, 6H), 2.32 (m, 1H), 3.02 (m, 1H), 3.42 (m, 1H), 3.66 (m, 1H), 5.57 (d, 1H), 7.12 (d, 1H), 7.22 (m, 1H), 7.39 (m, 1H) 7.49 (m, 1H), 9.31 (s, 1H, NH).

The racemate is resolved into enantiomers by preparative HPLC (Daicel Chiralpak AD, eluent: iso-hexane - ethanol, v/v 30:70).

### Example 2-1: (5aS,9aS)-4-(2,4-difluorobenzoyl)-5a,6,7,8,9,9a-hexahydropyrido[1,2-a]benzimidazol-1(5H)-one: Rₜ = 5.64 min, e.e. > 99.5%

### Example 2-2: (5aR,9aR)-4-(2,4-difluorobenzoyl)-5a,6,7,8,9,9a-hexahydropyrido-[1,2-a]benzimidazol-1(5H)-one: R_{f} = 8.01 min, e.e. > 99.5%

### Example 3

### (Rac-cis-5a,9a)-4-benzoyl-5a,6,7,8,9,9a-hexahydropyrido[1,2-a]benzimidazol-1(5H)-one

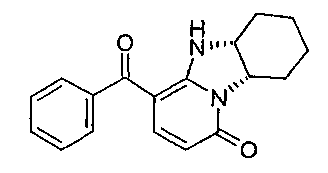

130 mg (1.9 mmol) propiolic acid and 370 mg (2.3 mmol) 1,1-carbonyldiimidazol are dissolved in 30 ml tetrahydrofuran and stirred during 1h at room temperature. 310 mg (1.3 mmol) of the compound of example 5a are added and the mixture is heated to reflux during 24h. The mixture is cooled to room temperature, concentrated under vacuum and dissolved in ethyl acetate. The organic phase is washed with saturated sodium hydrogencarbonate solution and water, dried over magnesium sulfate, filtered and concentrated under vacuum. The crude is washed with diethyl ether, filtered and dried to yield 35 mg (9% of th.) (*rac*-*cis*-5a,9a)-4-benzoyl-5a,6,7,8,9,9a-hexahydropyrido[1,2-a]benzimidazol-1(5H)-one.

¹H-NMR (300MHz, DMSO-d₆): δ = 1.2 - 1.8 (m, 6H), 2.08 - 2.26 (m, 2H), 4.13 (m, 1H), 4.53 (m, 1H), 5.56 (d, 1H), 7.35 (d, 1H) 7.45 - 7.60 (m, 5H), 8.98 (s, 1H, NH).

### Example 4

### (Rac-trans-5a,9a)-4-benzoyl-5a,6,7,8,9,9a-hexahydropyrido[1,2-a]benzimidazol-1(5H)-one

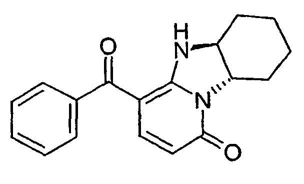

350 mg (5.0 mmol) propiolic acid and 960 mg (5.9 mmol) 1,1-carbonyldiimidazol are dissolved in 30 ml tetrahydrofuran and stirred during 1h at room temperature. 800 mg (3.3 mmol) of the compound of example 6a are added and the mixture is heated to reflux during 24h. The mixture is cooled to room temperature, concentrated under vacuum and dissolved in ethyl acetate. The organic phase is washed with saturated sodium hydrogencarbonate solution and water, dried over magnesium sulfate, filtered and concentrated under vacuum. The residue is washed with diethyl ether, filtered and dried to yield 800 mg (82% of th.) (*rac*-*trans*-5a,9a)-4-benzoyl-5a,6,7,8,9,9a-hexahydropyrido[1,2-a]benzimidazol-1(5H)-one.

¹H-NMR (300MHz, DMSO-d₆): δ = 1.24 - 1.46 (m, 2H), 1.53 (m, 1H), 1.65 (m, 1H), 1.85 (m, 2H), 2.32 (m, 1H), 3.04 (m, 1H), 3.41 (m, 1H), 3.64 (m, 1H), 5.56 (d, 1H), 7.34 (d, 1H) 7.42 - 7.62 (m, 5H), 9.20 (s, 1H, NH).

The racemate is resolved into enantiomers by preparative HPLC (Daicel Chiralpak AD, eluent: iso-hexane - ethanol, v/v 30:70).
(5aS,9aS)-4-benzoyl-5a,6,7,8,9,9a-hexahydropyrido[1,2-a]benzimidazol-1(5H)-one: Rₜ = 6.52 min, e.e. > 99.5%
(5aR,9aR)-4-benzoyl-5a,6,7,8,9,9a-hexahydropyrido[1,2-a]benzimidazol-1(5H)-one: Rₜ= 8.34 min,e.e.> 99.5%

### Example 5

### (Rac-cis-5a,9a)-4-(3-fluorobenzoyl)-5a,6,7,8,9,9a-hexahydropyrido[1,2-a]benzimidazol-1(5H)-one

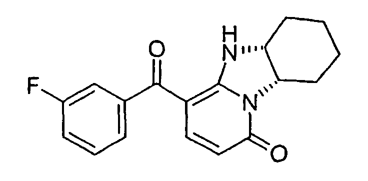

81 mg (1.15 mmol) propiolic acid and 224 mg (1.4 mmol) 1,1-carbonyldiimidazol are dissolved in 30 ml tetrahydrofuran and stirred during 1h at room temperature. 200 mg (0.77 mmol) of the compound of example 7a are added and the mixture is heated to reflux during 24h. The mixture is cooled to room temperature, concentrated under vacuum and dissolved in ethyl acetate. The organic phase is washed with saturated sodium hydrogencarbonate solution and water, dried over magnesium sulfate, filtered and concentrated under vacuum. The crude is purified over preparative HPLC (RP18-column, eluent: acetonitrile-water gradient) to yield 187 mg (78% of th.) (*rac-cis*-5a,9a)-4-(3-fluorobenzoyl)-5a,6,7,8,9,9a-hexahydropyrido[1,2-a]benzimidazol-1(5H)-one.

¹H-NMR (300MHz, DMSO-d₆): δ = 1.17 - 1.62 (m, 5H), 1.71 (m, 1H), 2.08 - 2.31 (m, 2H), 4.14 (m, 1H), 4.54 (m, 1H), 5.57 (d, 1H), 7.23 - 7.44 (d, 1H + m, 3H) 7.48 - 7.62 (m, 1H), 9.01 (s, 1H, NH).

### Example 6

### (Rac-trans-5a,9a)-4-(3-fluorobenzoyl)-5a,6,7,8,9,9a-hexahydropyrido[1,2-a]benzimidazol-1(5H)-one

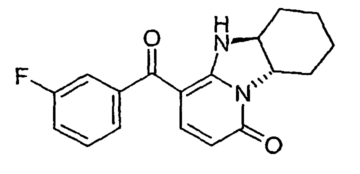

103 mg (1.47 mmol) propiolic acid and 286 mg (1.76 mmol) 1,1-carbonyldiimidazol are dissolved in 30 ml tetrahydrofuran and stirred during 1h at room temperature. 250 mg (0.98 mmol) of the compound of example 8a are added and the mixture is heated to reflux during 24h. The mixture is cooled to room temperature, concentrated under vacuum and dissolved in ethyl acetate. The organic phase is washed with saturated sodium hydrogencarbonate solution and water, dried over magnesium sulfate, filtered and concentrated under vacuum. The residue is washed with diethyl ether, filtered and dried to yield 143 mg (47% of th.) (*rac*-*trans*-5a,9a)-4-(3-fluorobenzoyl)-5a,6,7,8,9,9a-hexahydropyrido[1,2-a]benzimidazol-1(5H)-one.

¹H-NMR (300MHz, CDCl₃): δ = 1.33 - 1.56 (m, 2H), 1.63 - 1.80 (m, 2H), 1.96 (m, 1H), 2.29 (m, 1H), 3.30 (m, 1H), 3.49 (m, 1H), 3.68 (m, 1H), 5.75 (d, 1H), 7.13 - 7.35 (m, 3H, under CHCl₃), 7.5 - 7.65 (m, 1H + d, 1H), 9.31 (s, 1H, NH).

In analogy to the procedure for example 6 the enantiopure products are synthesized using the enantiopure compounds of example 8a, respectively.

(5aS,9aS)-4-(3-fluorobenzoyl)-5a,6,7,8,9,9a-hexahydropyrido[1,2-a]benzimidazol-1(5H)-one: yield: 96%, e.e. > 99.5%, determined by analytical HPLC (Daicel Chiralpak AD, eluent: iso-hexane - ethanol, v/v 30:70), Rₜ = 8.23 min.

(5aR,9aR)-4-(3-fluorobenzoyl)-5a,6,7,8,9,9a-hexahydropyrido[1,2-a]benzimidazol-1(5H)-one: yield: 63%, e.e. > 99.5%, determined by analytical HPLC (Daicel Chiralpak AD, eluent: iso-hexane - ethanol, v/v 30:70), Rₜ = 22.06 min

### Example 7

### (Rac-cis-5aR,9aS)-4-(4-fluorobenzoyl)-5a,6,7,8,9,9a-hexahydropyrido[1,2-a]-benzimidazol-1(5H)-one

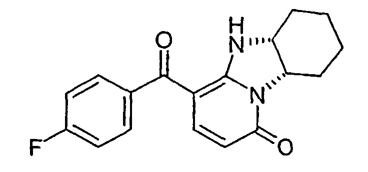

60 mg (0.92 mmol) propiolic acid and 180 mg (1.1 mmol) 1,1-carbonyldiinudazol are dissolved in 30 ml tetrahydrofuran and stirred during 1h at room temperature. 160 mg (0.61 mmol) of the compound of example 9a are added and the mixture is heated to reflux during 24h. The mixture is cooled to room temperature, concentrated under vacuum and dissolved in ethyl acetate. The organic phase is washed with saturated sodium hydrogen carbonate solution and water, dried over magnesium sulfate, filtered and concentrated under vacuum. The crude is purified over preparative HPLC (RP18-column, eluent: acetonitrile-water gradient) to yield 140 mg (71% of th.) *(rac-cis*-5a,9a)-4-(4-fluorobenzoyl)-5a,6,7,8,9,9a-hexahydropyrido[1,2-a]benzimidazol-1(5H)-one.

¹H-NMR (300MHz, DMSO-d₆): δ = 1.11 - 1.61 (m, 5H), 1.61 - 1.80 (m, 1H), 2.05 - 2.30 (m, 2H), 4.13 (m, 1H), 4.53 (m, 1H), 5.56 (d, 1H), 7.22 - 7.42 (d, 1H + m, 2H) 7.52 - 7.64 (m, 2H), 8.97 (s, 1H, NH).

### Example 8

### (5aR,9aR)-4-(4-fluorobenzoyl)-5a,6,7,8,9,9a-hexahydropyrido[1,2-a]benzimidazol-1(5H)-one

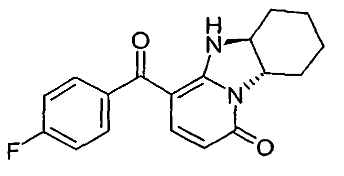

190 mg (2.7 mmol) propiolic acid and 530 mg (3.3 mmol) 1,1-carbonyldiimidazol are dissolved in 30 ml tetrahydrofuran and stirred during 1h at room temperature. 470 mg (1.81 mmol) of the compound of example 10a are added and the mixture is heated to reflux during 24h. The mixture is cooled to room temperature, concentrated under vacuum and dissolved in ethyl acetate. The organic phase is washed with saturated sodium hydrogen carbonate solution and water, dried over magnesium sulfate, filtered and concentrated under vacuum. The crude is purified over preparative HPLC (RP18-column, eluent: acetonitrile-water gradient) to yield 237 mg (42% of th.) (*rac-trans*-5a,9a)-4-(4-fluorobenzoyl)-5a,6,7,8,9,9a-hexahydropyrido[1,2-a]benzimidazol-1(5H)-one.

¹H-NMR (300MHz, DMSO-d₆): δ = 1.24 - 1.46 (m, 2H), 1.53 (m, 1H), 1.65 (m, 1H), 1.80 (m, 2H), 2.31 (m, 1H), 3.04 (m, 1H), 3.40 (m, 1H), 3.64 (m, 1H), 5.57 (d, 1H), 7.26 - 7.4 (d, 1H + m, 2H), 7.5 - 7.63 (m, 2H), 9.18 (s, 1H, NH).

### Example 9

### (Rac-cis-5a,9a)-4-(3-chlorobenzoyl)-5a,6,7,8,9,9a-hexahydropyrido[1,2-a]benzimidazol-1(5H)-one

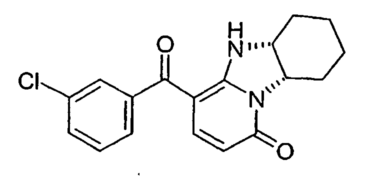

90 mg (1.3 mmol) propiolic acid and 250 mg (1.6 mmol) 1,1-carbonyldiimidazol are dissolved in 30 ml tetrahydrofuran and stirred during 1h at room temperature. 240 mg (0.87 mmol) of the compound of example 11a are added and the mixture is heated to reflux during 24h. The mixture is cooled to room temperature, concentrated under vacuum and dissolved in ethyl acetate. The organic phase is washed with saturated sodium hydrogen carbonate solution and water, dried over magnesium sulfate, filtered and concentrated under vacuum. The crude is purified over preparative HPLC (RP18-column, eluent: acetonitrile-water gradient) to yield 74 mg (26% of th.) (*rac-cis*-5a,9a)-4-(3-chlorobenzoyl)-5a,6,7,8,9,9a-hexahydropyrido[1,2-a]benzimidazol-1(5H)-one.

¹H-NMR (300MHz, DMSO-d₆): δ = 1.13 - 1.8 (m, 6H), 2.07 - 2.31 (m, 2H), 4.14 (m, 1H), 4.54 (m, 1H), 5.58 (d, 1H), 7.32 (d, 1H), 7.45 (m, 1H), 7.49 - 7.56 (m, 2H), 7.6 (m, 1H), 9.02 (s, 1H, NH).

### Example 10

### (Rac-trans-5a,9a)-4-(3-chlorobenzoyl)-5a,6,7,8,9,9a-hexahydropyrido[1,2-a]benzimidazol-1(5H)-one

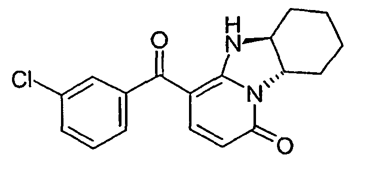

260 mg (3.7 mmol) propiolic acid and 720 mg (4.4 mmol) 1,1-carbonyldiimidazol are dissolved in 30 ml tetrahydrofuran and stirred during 1h at room temperature. 680 mg (2.5 mmol) of the compound of example 12a are added and the mixture is heated to reflux during 24h. The mixture is cooled to room temperature, concentrated under vacuum and dissolved in ethyl acetate. The organic phase is washed with saturated sodium hydrogen carbonate solution and water, dried over magnesium sulfate, filtered and concentrated under vacuum. The crude is purified over preparative HPLC (RP18-column, eluent: acetonitrile-water gradient) to yield 92 mg (11% of th.) *(rac-trans*-5a,9a)-4-(3-chlorobenzoyl)-5a,6,7,8,9,9a-hexahydropyrido[1,2-a]benzimidazol-1(5H)-one.

¹H-NMR (300MHz, DMSO-d₆): δ = 1.17 - 1.73 (m, 4H), 1.80 (m, 2H), 2.32 (m, 1H), 3.04 (m, 1H), 3.41 (m, 1H), 3.64 (m, 1H), 5.57 (d, 1H), 7.30 (d, 1H), 7.43 (m, 1H), 7.47 - 7.56 (m, 2H), 7.61 (m, 1H), 9.20 (s, 1H, NH).

### Example 11

### (Rac-cis-5a,9a)-4-(4-chlorobenzoyl)-5a,6,7,8,9,9a-hexahydropyrido[1,2-a]benzimidazol-1(5H)-one

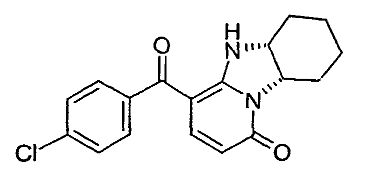

140 mg (2.0 mmol) propiolic acid and 380 mg (2.3 mmol) 1,1-carbonyldiimidazol are dissolved in 30 ml tetrahydrofuran and stirred during 1h at room temperature. 360 mg (1.30 mmol) of the compound of example 13a are added and the mixture is heated to reflux during 24h. The mixture is cooled to room temperature, concentrated under vacuum and dissolved in ethyl acetate. The organic phase is washed with saturated sodium hydrogen carbonate solution and water, dried over magnesium sulfate, filtered and concentrated under vacuum. The crude is purified over preparative HPLC (RP18-column, eluent: acetonitrile-water gradient) to yield 167 mg (39% of th.) (*rac-cis*-5a,9a)-4-(4-chlorobenzoyl)-5a,6,7,8,9,9a-hexahydropyrido[1,2-a]benzimidazol-1(5H)-one.

¹H-NMR (300MHz, DMSO-d₆): δ = 1.16 - 1.33 (m, 2H), 1.33 - 1.48 (m, 1H), 1.48 - 1.60 (m, 2H), 1.62 - 1.76 (m, 1H), 2.09 - 2.28 (m, 2H), 4.12 (m, 1H), 4.53 (m, 1H), 5.56 (d, 1H), 7.34 (d, 1H), 7.55 (m, 4H), 9.02 (s, 1H, NH).

The racemate is resolved into enantiomers by preparative HPLC (Daicel Chiralpak AD, eluent: *iso*-hexane - ethyl acetate, v/v 50:50).
(*Cis*-5a,9a)-4-(4-chlorobenzoyl)-5a,6,7,8,9,9a-hexahydropyrido[1,2-a]benzimidazol-1(5H)-one: Rₜ = 5.187 min, e.e. > 98%
(*Cis*-5a,9a)-4-(4-chlorobenzoyl)-5a,6,7,8,9,9a-hexahydropyrido[1,2-a]benzimidazol-1(5H)-one: Rₜ = 5.96 min, e.e. = 92%

### Example 12

### (Rac-trans-5a,9a)-4-(4-chlorobenzoyl)-5a,6,7,8,9,9a-hexahydropyrido[1,2-a]benzimidazol-1(5H)-one

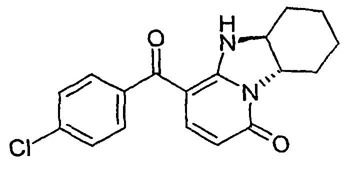

310 mg (4.4 mmol) propiolic acid and 860 mg (5.3 mmol) 1,1-carbonyldiimidazol are dissolved in 30 ml tetrahydrofuran and stirred during 1h at room temperature. 810 mg (2.9 mmol) of the compound of example 14a are added and the mixture is heated to reflux during 24h. The mixture is cooled to room temperature, concentrated under vacuum and dissolved in ethyl acetate. The organic phase is washed with saturated sodium hydrogen carbonate solution and water, dried over magnesium sulfate, filtered and concentrated under vacuum. The residue is washed with diethyl ether, filtered and dried to yield 590 mg (61% of th.) (*rac*-*trans*-5a,9a)-4-(4-chlorobenzoyl)-5a,6,7,8,9,9a-hexahydropyrido[1,2-a]benzimidazol-1(5H)-one.

¹H-NMR (300MHz, CDCl₃): δ = 1.13 - 1.82 (m, 4H), 1.95 (m, 2H), 2.28 (m, 1H), 3.30 (m, 1H), 3.48 (m, 1H), 3.67 (m, 1H), 5.74 (d, 1H), 7.34 - 7.56 (d, 1H + m, 4H), 9.18 (s, 1H, NH).

### Example 13

### (Rac-cis-5a,9a)-4-(2-chlorobenzoyl)-5a,6,7,8,9,9a-hexahydropyrido[1,2-a]benzimidazol-1(5H)-one

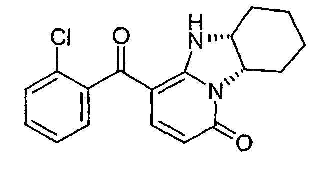

134 mg (1.9 mmol) propiolic acid and 375 mg (2.3 mmol) 1,1-carbonyldiimidazol are dissolved in 30 ml tetrahydrofuran and stirred during 1h at room temperature. 355 mg (1.30 mmol) of the compound of example 15a are added and the mixture is heated to reflux during 24h. The mixture is cooled to room temperature, concentrated under vacuum and dissolved in ethyl acetate. The organic phase is washed with saturated sodium hydrogen carbonate solution and water, dried over magnesium sulfate, filtered and concentrated under vacuum. The crude is purified over preparative HPLC (RP18-column, eluent: acetonitrile-water gradient) to yield 134 mg (32% of th.) (*rac-cis*-5a,9a)-4-(2-chlorobenzoyl)-5a,6,7,8,9,9a-hexahydropyrido[1,2-a]benzimidazol-1(5H)-one.

¹H-NMR (300MHz, DMSO-d₆): δ = 1.13 - 1.63 (m, 5H), 1.71 (m, 1H), 2.06 - 2.31 (m, 2H), 4.16 (m, 1H), 4.53 (m, 1H), 5.53 (d, 1H), 6.91 (d, 1H), 7.37 (m, 1H), 7.41 - 7.59 (m, 3H), 9.07 (s, 1H, NH).

### Example 14

### (Rac-trans-5a,9a)-4-(2-chlorobenzoyl)-5a,6,7,8,9,9a-hexahydropyrido[1,2-a]benzimidazol-1(5H)-one

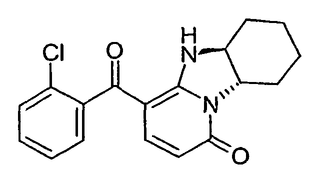

326 mg (4.7 mmol) propiolic acid and 907 mg (5.6 mmol) 1,1-carbonyldiimidazol are dissolved in 30 ml tetrahydrofuran and stirred during 1h at room temperature. 860 mg (3.1 mmol) of the compound of example 16a are added and the mixture is heated to reflux during 24h. The mixture is cooled to room temperature, concentrated under vacuum and dissolved in ethyl acetate. The organic phase is washed with saturated sodium hydrogen carbonate solution and water, dried over magnesium sulfate, filtered and concentrated under vacuum. The residue is washed with diethyl ether, filtered and dried to yield 530 mg (52% of th.) (*rac*-*trans*-5a,9a)-4-(2-chlorobenzoyl)-5a,6,7,8,9,9a-hexahydropyrido[1,2-a]benzimidazol-1(5H)-one.

¹H-NMR (300MHz, DMSO-d₆): δ = 1.12 - 1.95 (m, 4H), 2.33 (m, 1H), 3.02 (m, 1H), 3.43 (m, 1H), 3.66 (m, 1H), 5.53 (d, 1H), 6.90 (d, 1H), 7.22 - 7.66 (m, 4H), 9.32 (s, 1H, NH).

### Example 15

### (Rac-cis-5a,9a)-4-(2-fluorobenzoyl)-5a,6,7,8,9,9a-hexahydropyrido[1,2-a]benzimidazol-1(5H)-one

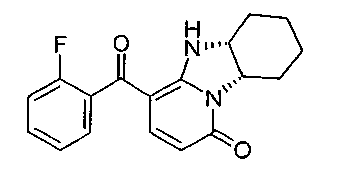

161 mg (2.3 mmol) propiolic acid and 448 mg (2.8 mmol) 1,1-carbonyldiimidazol are dissolved in 30 ml tetrahydrofuran and stirred during 1h at room temperature. 400 mg (1.50 mmol) of the compound of example 18a are added and the mixture is heated to reflux during 24h. The mixture is cooled to room temperature, concentrated under vacuum and dissolved in ethyl acetate. The organic phase is washed with saturated sodium hydrogen carbonate solution and water, dried over magnesium sulfate, filtered and concentrated under vacuum. The crude is purified over preparative HPLC (RP18-column, eluent: acetonitrile-water gradient) to yield 340 mg (71% of th.) (*rac-cis*-5a,9a)-4-(2-fluorobenzoyl)-5a,6,7,8,9,9a-hexahydropyrido[1,2-a]benzimidazol-1(5H)-one.

¹H-NMR (300MHz, CDCl₃): δ = 1.19 - 1.95 (m, 4H), 2.12 (m, 1H), 2.43 (m, 1H), 4.23 (m, 1H), 4.63 (m, 1H), 5.74 (d, 1H), 7.08 - 7.31 (d, 1H + m, 2H), 7.33 - 7.52 (m, 2H), 8.42 (s, 1H, NH).

### Example 16

### (Rac-trans-5a,9a)-4-(2-fluorobenzoyl)-5a,6,7,8,9,9a-hexahydropyrido[1,2-a]benzimidazol-1(5H)-one

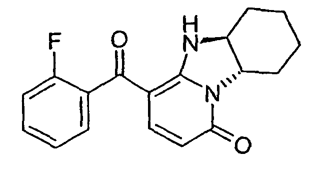

130 mg (1.8 mmol) propiolic acid and 350 mg (2.1 mmol) 1,1-carbonyldiimidazol are dissolved in 30 ml tetrahydrofuran and stirred during 1h at room temperature. 310 mg (1.2 mmol) of the compound of example 19a are added and the mixture is heated to reflux during 24h. The mixture is cooled to room temperature, concentrated under vacuum and dissolved in ethyl acetate. The organic phase is washed with saturated sodium hydrogen carbonate solution and water, dried over magnesium sulfate, filtered and concentrated under vacuum. The residue is washed with diethyl ether, filtered and dried to yield 260 mg (68% of th.) (*rac*-*trans*-5a,9a)-4-(2-fluorobenzoyl)-5a,6,7,8,9,9a-hexahydropyrido[1,2-a]benzimidazol-1(5H)-one.

¹H-NMR (300MHz, CDCl₃): δ = 1.17 - 1.85 (m, 4H), 1.85 - 2.13 (m, 2H), 2.30 (m, 1H), 3.29 (m, 1H), 3.39 - 3.80 (m, 2H), 5.74 (d, 1H), 7.02 - 7.59 (d, 1H + m, 4H), 8.77 (s, 1H, NH).

### Example 17

### (Rac-cis-5a,9a)-4-(3-bromobenzoyl)-5a,6,7,8,9,9a-hexahydropyrido[1,2-a]benzimidazol-1(5H)-one

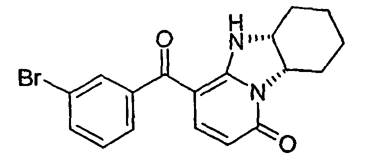

130 mg (1.8 mmol) propiolic acid and 350 mg (2.2 mmol) 1,1-carbonyldiimidazol are dissolved in 30 ml tetrahydrofuran and stirred during 1h at room temperature. 390 mg (1.2 mmol) of the compound of example 20a are added and the mixture is heated to reflux during 24h. The mixture is cooled to room temperature, concentrated under vacuum and dissolved in ethyl acetate. The organic phase is washed with saturated sodium hydrogen carbonate solution and water, dried over magnesium sulfate, filtered and concentrated under vacuum. The residue is washed with diethyl ether, filtered and dried to yield 50 mg (11% of th.) (*rac-cis*-5a,9a)-4-(3-bromobenzoyl)-5a,6,7,8,9,9a-hexahydropyrido[1,2-a]benzimidazol-1(5H)-one.

¹H-NMR (300MHz, DMSO-d₆): δ = 1.2 - 1.8 (m, 6H), 2.07 - 2.31 (m, 2H), 4.14 (m, 1H), 4.53 (m, 1H), 5.57 (d, 1H), 7.31 (d, 1H), 7.39 - 7.56 (m, 2H), 7.64 (s, 1H), 7.73 (m, 1H), 9.01 (s, 1H, NH).

### Example 18

### (Rac-trans-5a,9a)-4-(3-bromobenzoyl)-5a,6,7,8,9,9a-hexahydropyrido[1,2-a]benzimidazol-1(5H)-one

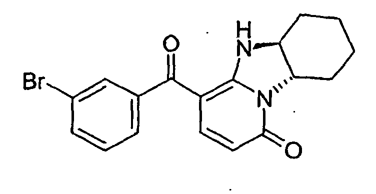

280 mg (4.0 mmol) propiolic acid and 770 mg (4.8 mmol) 1,1-carbonyldiimidazol are dissolved in 30 ml tetrahydrofuran and stirred during 1h at room temperature. 850 mg (2.7 mmol) of the compound of example 21a are added and the mixture is heated to reflux during 24h. The mixture is cooled to room temperature, concentrated under vacuum and dissolved in ethyl acetate. The organic phase is washed with saturated sodium hydrogen carbonate solution and water, dried over magnesium sulfate, filtered and concentrated under vacuum. The residue is washed with diethyl ether, filtered and dried to yield 400 mg (39% of th.) (*rac-trans*-5a,9a)-4-(3-bromobenzoyl)-5a,6,7,8,9,9a-hexahydropyrido[1,2-a]benzimidazol-1(5H)-one.

¹H-NMR (300MHz, DMSO-d₆): δ = 1.21-1.73 (m, 4H), 1.80 (m, 2H), 2.32 (m, 1H), 3.04 (m, 1H), 3.41 (m, 1H), 3.64 (m, 1H), 5.57 (d, 1H), 7.29 (d, 1H), 7.41 - 7.52 (m, 2H), 7.63 (m, 1H), 7.69 - 7.79 (m, 1H), 9.20 (s, 1H, NH).

### Example 19

### (Rac-cis-5a,9a)-4-(2-methylbenzoyl)-5a,6,7,8,9,9a-hexahydropyrido[1,2-a]benzimidazol-1(5H)-one

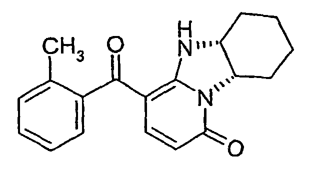

160 mg (2.2 mmol) propiolic acid and 430 mg (2.7 mmol) 1,1-carbonyldiimidazol are dissolved in 30 ml tetrahydrofuran and stirred during 1h at room temperature. 380 mg (1.50 mmol) of the compound of example 22a are added and the mixture is heated to reflux during 24h. The mixture is cooled to room temperature, concentrated under vacuum and dissolved in ethyl acetate. The organic phase is washed with saturated sodium hydrogen carbonate solution and water, dried over magnesium sulfate, filtered and concentrated under vacuum. The residue is washed with diethyl ether, filtered and dried to yield 216 mg (47% of th.) (*rac*-*cis*-5a,9a)-4-(2-methylbenzoyl)-5a,6,7,8,9,9a-hexahydropyrido[1,2-a]benzimidazol-1(5H)-one.

¹H-NMR (300MHz, DMSO-d₆): δ = 1.16 - 1.61 (m; 4H), 1.71 (m, 1H), 2.06 - 2.33 (m, 1H + s, 3H), 4.15 (m, 1H), 4.52 (m, 1H), 5.50 (d, 1H), 6.96 (d, 1H) 7.17 (m, 1H), 7.21 - 7.40 (m, 3H), 9.03 (s, 1H, NH).

### Example 20

### (Rac-trans-5a,9a)-4-(2-methylbenzoyl)-5a,6,7,8,9,9a-hexahydropyrido[1,2-a]-benzimidazol-1(5H)-one

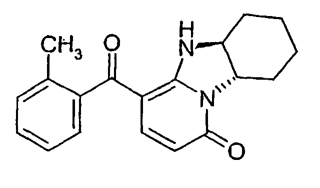

120 mg (1.7 mmol) propiolic acid and 320 mg (2.0 mmol) 1,1-carbonyldiimidazol are dissolved in 30 ml tetrahydrofuran and stirred during 1h at room temperature. 290 mg (1.1 mmol) of the compound of example 23a are added and the mixture is heated to reflux during 24h. The mixture is cooled to room temperature, concentrated under vacuum and dissolved in ethyl acetate. The organic phase is washed with saturated sodium hydrogen carbonate solution and water, dried over magnesium sulfate, filtered and concentrated under vacuum. The residue is washed with diethyl ether, filtered and dried to yield 132 mg (38% of th.) (*rac-trans*-5a,9a)-4-(2-methylbenzoyl)-5a,6,7,8,9,9a-hexahydropyrido[1,2-a]benzimidazol-1 (5H)-one.

¹H-NMR (300MHz, DMSO-d₆): δ = 1.21 - 1.48 (m, 2H), 1.54 (m, 1H), 1.65 (m, 1H), 1.8 (m, 2H), 2.19 (s, 2H), 2.33 (m, 1H), 3.03 (m, 1H), 3.43 (m, 1H), 3.64 (m, 1H), 5.51 (d, 1H), 6.95 (d, 1H), 7.15 (m, 1H), 7.21 - 7.43 (m, 3H), 9.27 (s, 1H, NH).

### Example 21

### 8-(4-Chlorobenzoyl)-2,2-dimethyl-2,3-dihydroimidazo[1,2-a]pyridin-5(1H)-one

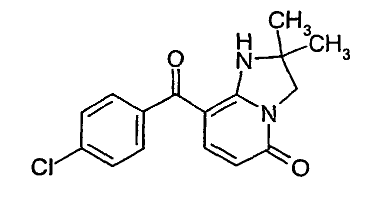

87 mg (1.2 mmol) propiolic acid and 233 mg (1.44 mmol) 1,1-carbonyldiimidazol are dissolved in 4 ml tetrahydrofuran and stirred during 1.5h at room temperature. 200 mg (0.80 mmol) of the compound of example 24a are added and the mixture is heated to reflux during 3h. The mixture is cooled to room temperature and ethyl acetate is added. The organic phase is washed with saturated sodium hydrogen carbonate solution, dried over sodium sulfate, filtered and concentrated in vacuum. The crude product is purified by preparative HPLC (RP18-column, eluent: acetonitrile-water gradient) to yield 181 mg (75% of th.) 8-(4-chlorobenzoyl)-2,2-dimethyl-2,3-dihydroimidazo[1,2-a]pyidin-5(1H)-one.

¹H-NMR (300MHz, DMSO-d₆): δ = 1.43 (m, 6H), 3.83 (s, 2H), 5.54 (d, 1H), 7.34 (d, 1H), 7.54 (m, 4H), 9.35 (s, 1H, NH).

### Example 22

### 9-Benzoyl-3,3-dimethyl-1,2,3,4-tetrahydro-6H-pyrido[1,2-a]pyrimidin-6-one

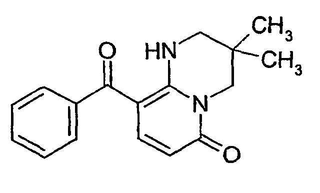

67 mg (0.96 mmol) propiolic acid and 187 mg (1.15 mmol) 1,1-carbonyldiimidazol are dissolved in 20 ml tetrahydrofuran and stirred during 1 h at room temperature. 148 mg (0.64 mmol) of the compound of example 25a are added and the mixture is heated to reflux during 24 h. The mixture is cooled to room temperature, concentrated under vacuum and dissolved in ethyl acetate. The organic phase is washed with saturated sodium hydrogencarbonate solution and water, dried over magnesium sulfate, filtered and concentrated under vacuum. The crude is purified over preparative HPLC (RP18-column, eluent: acetonitrile-water gradient) to yield 81 mg (43% of th.) 9-benzoyl-3,3-dimethyl-1,2,3,4-tetrahydro-6H-pyrido[1,2-a]pyrimidin-6-one.

¹H-NMR (200MHz, CDCl₃): δ = 1.14 (s, 6H), 3.21 (m, 2H), 3.75 (m, 2H), 5.71 (d, 1H), 7.40-7.51 (m, 5H + d, 1H), 11.29 (s, br, 1H, NH).
LC-MS (B): Rₜ = 3.63 min, MS (ESIpos): m/z = 283 (M+H)⁺.

### Example 23

### 9-(4-Chlorobenzoyl)-3,3-dimethyl-1,2,3,4-tetrahydro-6H-pyrido[1,2-a]pyrimidin-6-one

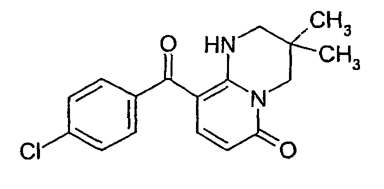

54 mg (0.77 mmol) propiolic acid and 150 mg (0.93 mmol) 1,1-carbonyldiimidazol are dissolved in 20 ml tetrahydrofuran and stirred during 1 h at room temperature. 136 mg (0.51 mmol) of the compound of example 26a are added and the mixture is heated to reflux during 24 h. The mixture is cooled to room temperature, concentrated under vacuum and recrystallized out of ethyl acetate to yield 83 mg (51% of th.) 9-(4-chlorobenzoyl)-3,3-dimethyl-1,2,3,4-tetrahydro-6H-pyrido[1,2-a]pyrimidin-6-one.

¹H-NMR (200MHz, CDCl₃): δ = 1.14 (s, 6H), 3.21 (m, 2H), 3.75 (m, 2H), 5.72 (d, 1H), 7.41 (d, 1H), 7.42 (m, 4H), 11.23 (s, br, 1H, NH).
LC-MS (B): Rₜ = 4.05 min, MS (ESIpos): m/z = 316 (M+H)⁺.

### Example 24

### 9-(4-Fluorobenzoyl)-3,3-dimethyl-1,2,3,4-tetrahydro-6H-pyrido[1,2-a]pyrimidin-6-one

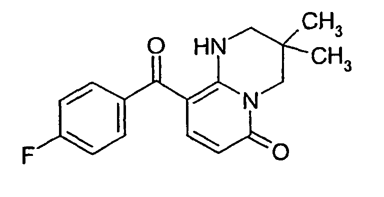

190 mg (2.73 mmol) propiolic acid and 530 mg (3.28 mmol) 1,1-carbonyldiimidazol are dissolved in 25 ml tetrahydrofuran and stirred during 1 h at room temperature. 450 mg (1.82 mmol) of the compound of example 27a are added and the mixture is heated to reflux during 24 h. The mixture is cooled to room temperature, concentrated under vacuum and dissolved in ethyl acetate. The organic phase is washed with saturated sodium hydrogencarbonate solution and water, dried over magnesium sulfate, filtered and concentrated under vacuum. The crude is washed with diethyl ether, filtered and dried to yield 400 mg (73% of th.) 9-(4-fluorobenzoyl)-3,3-dimethyl-1,2,3,4-tetrahydro-6H-pyrido[1,2-a]pyrimidin-6-one.

¹H-NMR (200MHz, CDCl₃): δ = 1.14 (s, 6H), 3.20 (m, 2H), 3.75 (m, 2H), 5.72 (d, 1H), 7.13 (m, 2H), 7.41 (d, 1H), 7.49 (m, 2H), 11.23 (s, br, 1H, NH).
LC-MS (B): Rₜ = 4.31 min, MS (ESIpos): m/z = 301 (M+H)⁺.

### Example 25

### 6-Benzoyl-4-methyl-2,3,3a,4,5,10a-hexahydrocyclopenta[e]pyrido[1,2-a]pyrimidin-9(1H)-one

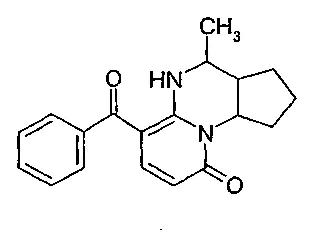

61 mg (0.88 mmol) propiolic acid and 170 mg (1.05 mmol) 1,1-carbonyldiimidazol are dissolved in 20 ml tetrahydrofuran and stirred during 1 h at room temperature. 150 mg (0.59 mmol) of the mixture of isomers of example 29a are added and the mixture is heated to reflux during 24 h. The mixture is cooled to room temperature, concentrated under vacuum and dissolved in ethyl acetate. The organic phase is washed with saturated sodium hydrogencarbonate solution and water, dried over magnesium sulfate, filtered and concentrated under vacuum. The crude is purified over preparative HPLC (RP18-column, eluent: acetonitrile-water gradient) to yield 10 mg (5% of th.) (*rac*)-6-benzoyl-4-methyl-2,3,3a,4,5,10a-hexahydrocyclopenta[e]-pyrido[1,2-a]pyrimidin-9(1H)-one as a single regio isomer.

¹H-NMR (200MHz, CDCl₃): δ = 1.29 (d, 3H), 1.43 (m, 1H), 1,64 (m, 1H), 1.82 (m, 1H), 1.91 (m, 2H), 2.22 (m, 1H), 3.25 (m, 1H), 3.69 (m, 1H), 3.93 (m, 1H), 5.59 (d, 1H), 7.39 (d, 1H), 7.45 (m, 5H), 11.89 (s, br, 1H, NH).
LC-MS (B): Rₜ = 4.03 min, MS (ESIpos): m/z = 309 (M+H)⁺.

A second fraction (yield: 35 mg (20% of th.); LC-MS (B): Rₜ = 4.14 min, MS (ESIpos): m/z = 309 (M+H)⁺) consists of a mixture of stereo and regio isomers. Two enantiopure regioisomers are isolated by preparative HPLC (Daicel Chiralpak AD, eluent: iso-hexane - ethanol, v/v 30:70).

### Example 25-1

(ent)-6-benzoyl-4-methyl-2,3,3a,4,5,10a-hexahydrocyclopenta[e]pyrido[1,2-a]pyrimidin-9(1H)-one:
yield: 10 mg (5% of th.)
HPLC (Chiral), Rₜ = 10.66 min, e.e. > 99.5%
¹H-NMR (200MHz, CDCl₃): δ =1.41 (d, 3H), 1.45 - 2.1 (m, 6H), 3.25 (m, 1H), 3.44 - 3.67 (m, 2H), 5.60 (d, 1H), 7.40 (d, 1H), 7.45 (m, 5H), 11.57 (s, br, 1H, NH).

### Example 25-2

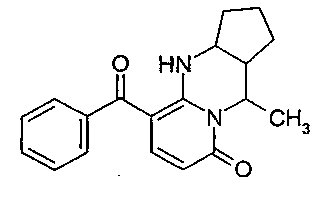

(ent)-5-benzoyl-10-methyl-2,3,3a,4,10,10a-hexahydrocylopenta[d]pylido[1,2-a]-pyrimidin-8(1H)-one
yield: 4 mg (2% of th.)
HPLC (Chiral), Rₜ = 5.13 min, e.e. > 99.5%
¹H-NMR (200MHz, CDCl₃): δ = 1.32 (d, 3H), 1.45 - 2.15 (m, 6H), 2.26 (m, 1H), 3.39 (m, 1H), 5.18 (m, 1H), 5.71 (d, 1H), 7.38 - 7.58 (d, 1H + m, 5H), 11.71 (s, br, 1H, NH).

### Example 26

### (Rac)-3-(benzyloxy)-9-(4-fluorobenzoyl)-1,2,3,4-tetrahydro-6H-pyrido[1,2-a]pyrimidin-6-one

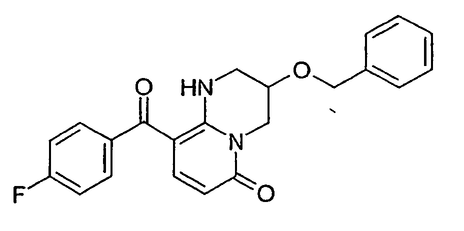

100 mg (0.35 mmol) of the compound of example 31a and 17 mg (0.42 mmol) sodium hydride (60% on paraffin oil) are dissolved in 2 ml dimethylformamide and stirred during 2 h at 60 °C. The mixture is cooled to room temperature and a solution of 65 mg (0.38 mmol) of benzyl bromide and 5 mg (0.03 mmol) potassium iodide in 0.7 mL dimethylformamide is added dropwise. The mixture is heated to 60°C during 20 h, cooled to room temperature, concentrated under vacuum, and dissolved in ethyl acetate. The organic layer is washed with a 1N solution of sodium hydroxide and water, dried over magnesium sulfate, and concentrated under vacuum. The crude is purified over preparative HPLC (RP18-column, eluent: acetonitrile-water gradient) to yield 6 mg (4.4% of th.) (*rac*)-3-(benzyloxy)-9-(4-fluorobenzoyl)-1,2,3,4-tetrahydro-6H-pyrido[1,2-a]pyrimidin-6-one.

¹H-NMR (200MHz, CDCl₃): δ = 3.61 (m, 2H), 3.69 (d, 1H), 4.14 (m, 1H), 4.56 - 4.74 (m, 3H), 5.74 (d, 1H), 7.13 (m, 2H), 7.29 - 7.40 (m, 4H), 7.41 - 7.56 (d, 1H + m, 3H), 11.2 (s, br, 1H, NH).
LC-MS (B): Rₜ = 4.44 min, MS (ESIpos): m/z = 379 (M+H)⁺.

### Example 27

### (Rac)-2-ethyl-9-(4-fluorobenzoyl)-1,2,3,4-tetrahydro-6H-pyrido[1,2-a]pyrimidin-6-one

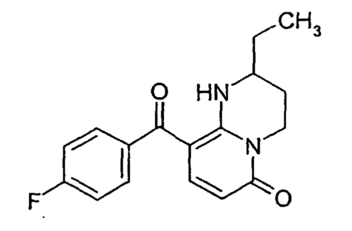

110 mg (1.51 mmol) propiolic acid and 290 mg (1.81 mmol) 1,1-carbonyldiimidazol are dissolved in 15 ml tetrahydrofuran and stirred during 1 h at room temperature. 250 mg (1.01 mmol) of the compound of example 32a are added and the mixture is heated to reflux during 24 h. The mixture is cooled to room temperature, concentrated under vacuum and dissolved in ethyl acetate. The organic phase is washed with saturated sodium hydrogencarbonate solution and water, dried over magnesium sulfate, filtered and concentrated under vacuum. The crude is purified over preparative HPLC (RP18-column, eluent: acetonitrile-water gradient) to yield 110 mg (36% of th.) (*rac*)-2-ethyl-9-(4-fluorobenzoyl)-1,2,3,4-tetrahydro-6H-pyrido[1,2-a]pyrimidin-6-one.

¹H-NMR (200MHz, DMSO-d₆): δ = 1.03 (t, 3H), 1.45 - 1.83 (m, 2H + m, 1H), 2.18 (m, 1H), 3.43 - 3.75 (m, 2H), 4.25 (m, 1H), 5.58 (d, 1H), 7.21 - 7.41 (m, 2H + d, 1H), 7.53 (m, 2H), 11.21 (s, br, 1H, NH).

### Example 28

**(*****Rac-trans*****-5a,9a)-4-(3-hydroxybenzoyl)-5a,6,7,8,9,9a-hexahydropyrido[1,2-a]benzimidazol-1(5H)-one**

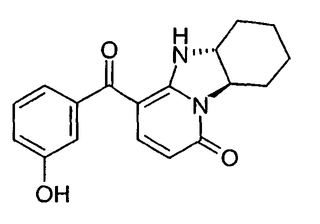

220 mg (0.72 mmol) (*rac*-*trans*-5a,9a)-4-(3-methoxybenzoyl)-5a,6,7,8,9,9a-hexahydropyrido[1,2-a]benzimidazol-1(5H)-one are dissolved in 6 ml dichloromethane and stirred under inert gas. 0.386 ml (4.1 mmol) boron tribromide are added at -78°C. After addition the cooling bath is removed and the mixture is allowed to return to room temperature. The mixture is stirred at room temperature for 12 h, quenched ith methanol, concentrated under vacuum and dissolved in ethyl acetate. The organic phase is washed with saturated sodium hydrogen carbonate solution and water, dried over magnesium sulfate, filtered and concentrated under vacuum. The residue is washed with diethyl ether, filtered and dried to yield 152 mg (68% of th.) *(rac-trans*-5a,9a)-4-(3-hydroxybenzoyl)-5a,6,7,8,9,9a-hexahydropyrido[1,2-a]benzimidazol-1(5H)-one.

¹H-NMR (200MHz, DMSO-d₆): δ = 1.15-1.95 (m, 6H), 2.31 (m, 1H), 3.04 (m, 1H), 3.43 (m, 1H, under H₂O), 3.61 (m, 1H), 5.56 (d, 1H), 6.78-7.01 (m, 3H), 7.28 (m, 1H) 7.38 (d, 1H), 9.17 (s, 1H, NH), 9.73 (s, 1H, OH).

### Example 29

### (Rac-cis-5a,9a)-4-(3-hydroxybenzoyl)-5a,6,7,8,9,9a-hexahydropyrido[1,2-a]benzimidazol-1(5H)-one

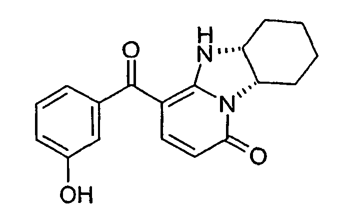

The cis-isomer is synthesized in an analogeous fashion starting from the compound of example 35 to yield (*rac*-*cis*-5a,9a)-4-(3-hydroxybenzoyl)-5a,6,7,8,9,9a-hexahydropyrido[1,2-a]benzimidazol-1(5H)-one in 75%.

¹H-NMR (200MHz, DMSO-d₆): δ = 1.1-1.85 (m, 6H), 2.05-2.35 (m, 2H), 4.12 (m, 1H), 4.52 (m, 1H), 5.56 (d, 1H), 6.78-7.01 (m, 3H), 7.29 (m, 1H) 7.39 (d, 1H), 8.97 (s, 1H, NH), 9.73 (s, 1H, OH).

### Example 30

### (Rac-cis-5a,9a)-4-(2-hydroxybenzoyl)-5a,6,7,8,9,9a-hexahydropyrido[1,2-a]benzimidazol-1(5H)-one

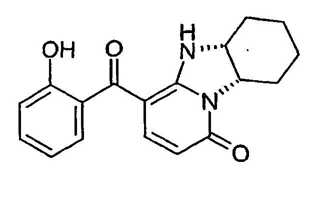

The compound is synthesized in an analogeous fashion as the compound of example 28 starting from the compound of example 34 to yield (*rac*-*cis*-5a,9a)-4-(2-hydroxybenzoyl)-5a,6,7,8,9,9a-hexahydropyrido[1,2-a]benzimidazol-1(5H)-one in 75%.

¹H-NMR (200MHz, DMSO-d₆): δ = 1.05-1.55 (m, 6H), 2.05-2.35 (m, 2H), 4.11 (m, 1H), 4.50 (m, 1H), 5.53 (d, 1H), 6.78-7.00 (m, 2H), 7.05-7.21 (m, 1H + d, 1H), 7.29 (m, 1H), 8.94 (s, 1H, NH), 9.81 (s, 1H, OH).

### Example 31

### 9-(4-Fluoro-3-hydroxybenzoyl)-3,3-dimethyl-1,2,3,4-tetrahydro-6H-pyrido[1,2-a]pyrimidin-6-one

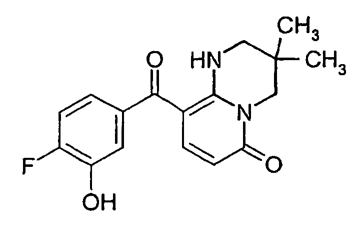

The compound is synthesized in an analogeous fashion as the compound of example 28 starting from the compound of example 59 to yield 9-(4-fluoro-3-hydroxybenzoyl)-3,3-dimethyl-1,2,3,4-tetrahydro-6H-pyrido[1,2-a]pyrimidin-6-one in 76%.

¹H-NMR (400MHz, DMSO-d₆): δ = 1.02 (m, 6H), 3.22 (m, 2H), 3.64 (m, 2H), 5.57 (d, 1H), 6.86 (m, 1H), 7.03 (m, 1H), 7.21 (m, 1H), 7.39 (d, 1H), 10.214 (s, 1H, OH), 10.96 (s, 1H, NH).

### Example 32

### (Rac-cis-5a,9a)-4-[4-fluoro-3-(phenylethynyl)benzoyl]-5a,6,7,8,9,9a-hexahydropyrido[1,2-a]benzimidazol-1(5H)-one

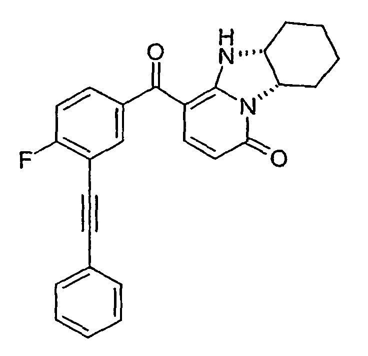

120 mg (0.3 mmol) (*rac-cis*-5a,9a)-4-(3-bromo-4-fluorobenzoyl)-5a,6,7,8,9,9a-hexahydropyrido[1,2-a]benzimidazol-1(5H)-one, 20 mg (0.03 mmol) bis-(triphenylphosphin)-palladium(II)-chloride, 10 mg (0.03 mmol) copper(I) iodide are dissolved in 7 ml diisopropylamine and 3 ml tetrahydrofuran under inert gas atmosphere. 120 mg (1.2 mmol) phenylacetylene are added dropwise at room temperature. The mixture is refluxed for 12 h, cooled down to room temperature and filtered over kieselgur. The filtrate is concentrated under vacuum. The crude is purified over preparative HPLC (RP18-column, eluent: acetonitrile-water gradient) to yield 58 mg (48% of th.) (*rac*-*cis*-5a,9a)-4-[4-fluoro-3-(phenylethynyl)benzoyl]-5a,6,7,8,9,9a-hexahydropyrido[1,2-a]benzimidazol-1(5H)-one.

¹H-NMR (200MHz, DMSO-d₆): δ = 1.1-1.85 (m, 6H), 2.05-2.35 (m, 2H), 4.13 (m, 1H), 4.55 (m, 1H), 5.59 (d, 1H), 7.2-7.9 (m, 8H + d, 1H), 9.03 (s, 1H, NH).

### Example 33

### (Rac-cis-5a,9a)-4-(3-ethynyl-4-fluorobenzoyl)-5a,6,7,8,9,9a-hexahydropyrido[1,2-a]benzimidazol-1(5H)-one

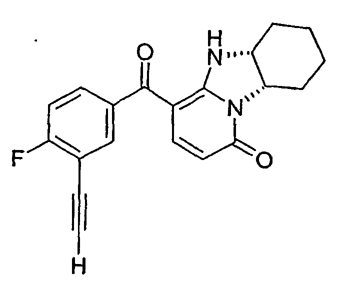

### Example 33-1

### (Rac-cis-5a,9a)-4-{4-fluoro-3-[(trimethylsilyl)ethynyl]benzoyl}-5a,6,7,8,9,9a-hexahydropyrido[1,2-a]benzimidazol-1(5H)-one

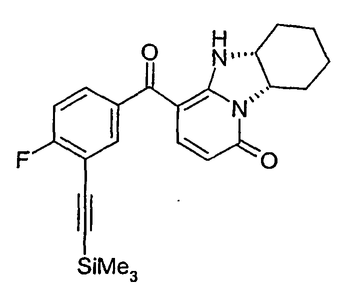

350 mg (0.88 mmol) of the compound of example 44, 60 mg (0.09 mmol) bis-(triphenylphosphin)-palladium(II)-chloride, 20 mg (0.09 mmol) copper(I) iodide are dissolved in 10 ml diisopropylamine and 6 ml dioxane under inert gas atmosphere. 350 mg (3.5 mmol) trimethylsilylacetylene are added dropwise at room temperature. The mixture is stirred at 50°C for 12 h, cooled down to room temperature and filtered over kieselguhr. The filtrate is concentrated under vacuum. The crude is purified over preparative HPLC (RP18-column, eluent: acetonitrile-water gradient) to yield 236 mg (63% of th.) (*rac-cis*-5a,9a)-4-{4-fluoro-3-[(trimethylsilyl)ethynyl]benzoyl}-5a,6,7,8,9,9a-hexahydropyrido[1,2-a]benzimidazol-1(5H)-one.
MS (ESIpos): m/z = 409 (M+H)⁺
HPLC (D): Rₜ = 5.16 min.

### (Rac-cis-5a,9a)-4-(3-ethynyl-4-fluorobenzoyl)-5a,6,7,8,9,9a-hexahydropyrido[1,2-a]benzimidazol-1(5H)-one

236 mg (0.58 mmol) of the compound of example 33-1 are dissolved in 5 ml of a 1M solution of tetrabutylammonium fluoride in tetrahydrofuran and stirred for 12 h at room temperature. The mixture is concentrated under vacuum. The crude is purified over preparative HPLC (RP18-column, eluent: acetonitrile-water gradient) to yield 92 mg (47% of th.) (*rac*-*cis*-5a,9a)-4-(3-ethynyl-4-fluorobenzoyl)-5a,6,7,8,9,9a-hexahydropyrido[1,2-a]benzimidazol-1(5H)-one.

¹H-NMR (300MHz, DMSO-d₆): δ = 1.15-1.8 (m, 6H), 2.05-2.35 (m, 2H), 4.12 (m, 1H), 4.54 (m, 1H), 4.58 (s, 1H), 5.57 (d, 1H), 7.33 (d, 1H), 7.42 (m, 1H), 7.59 (m, 1H), 7.65 (m, 1H), 8.98 (s, 1H, NH).

The following examples have been prepared according to the abovementioned procedure of example 1:

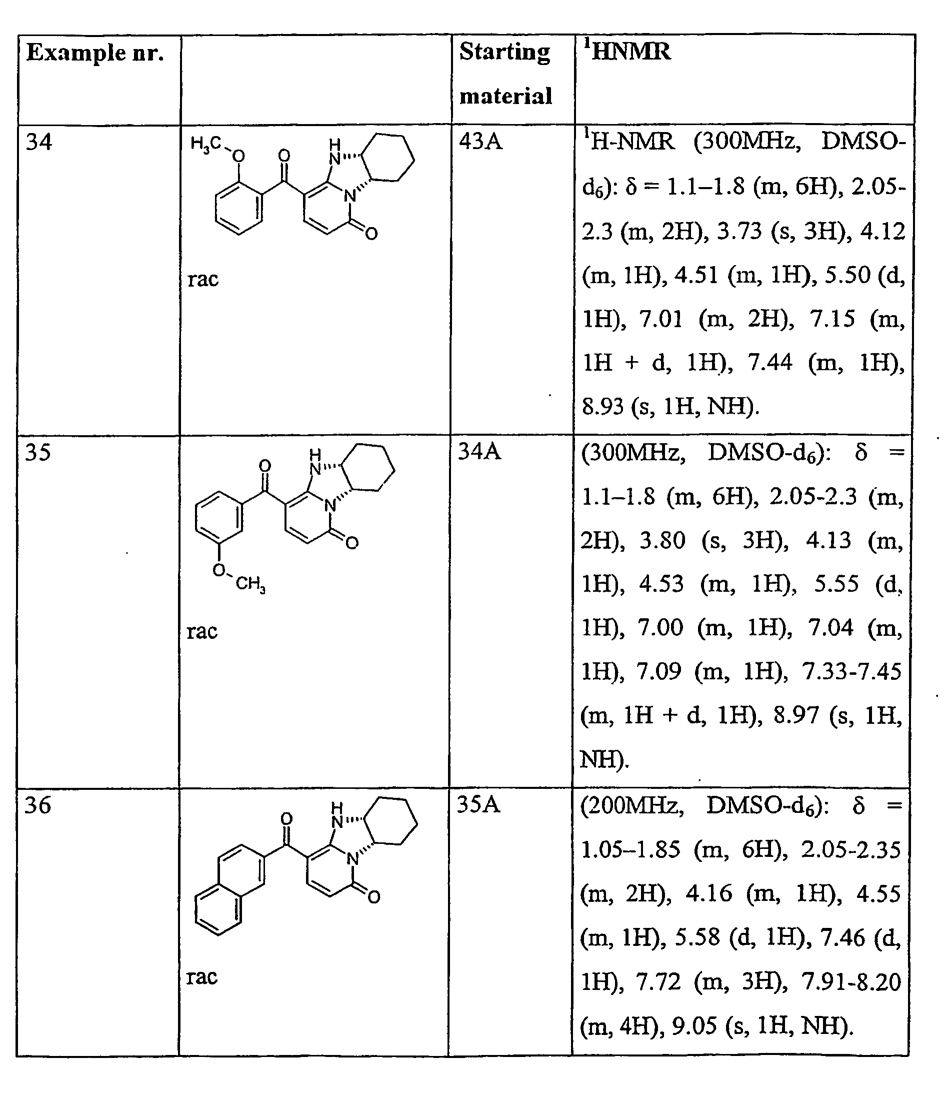

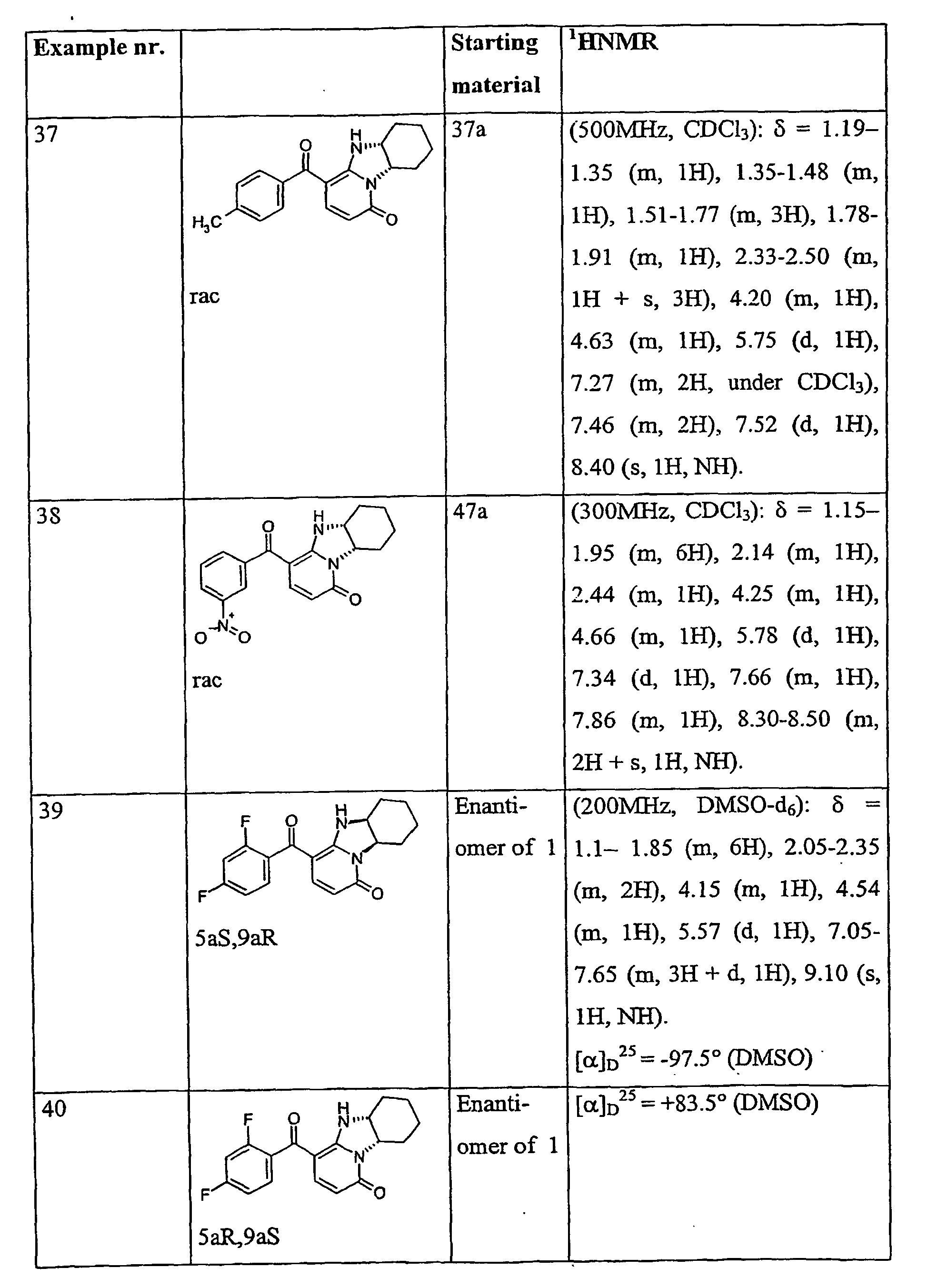

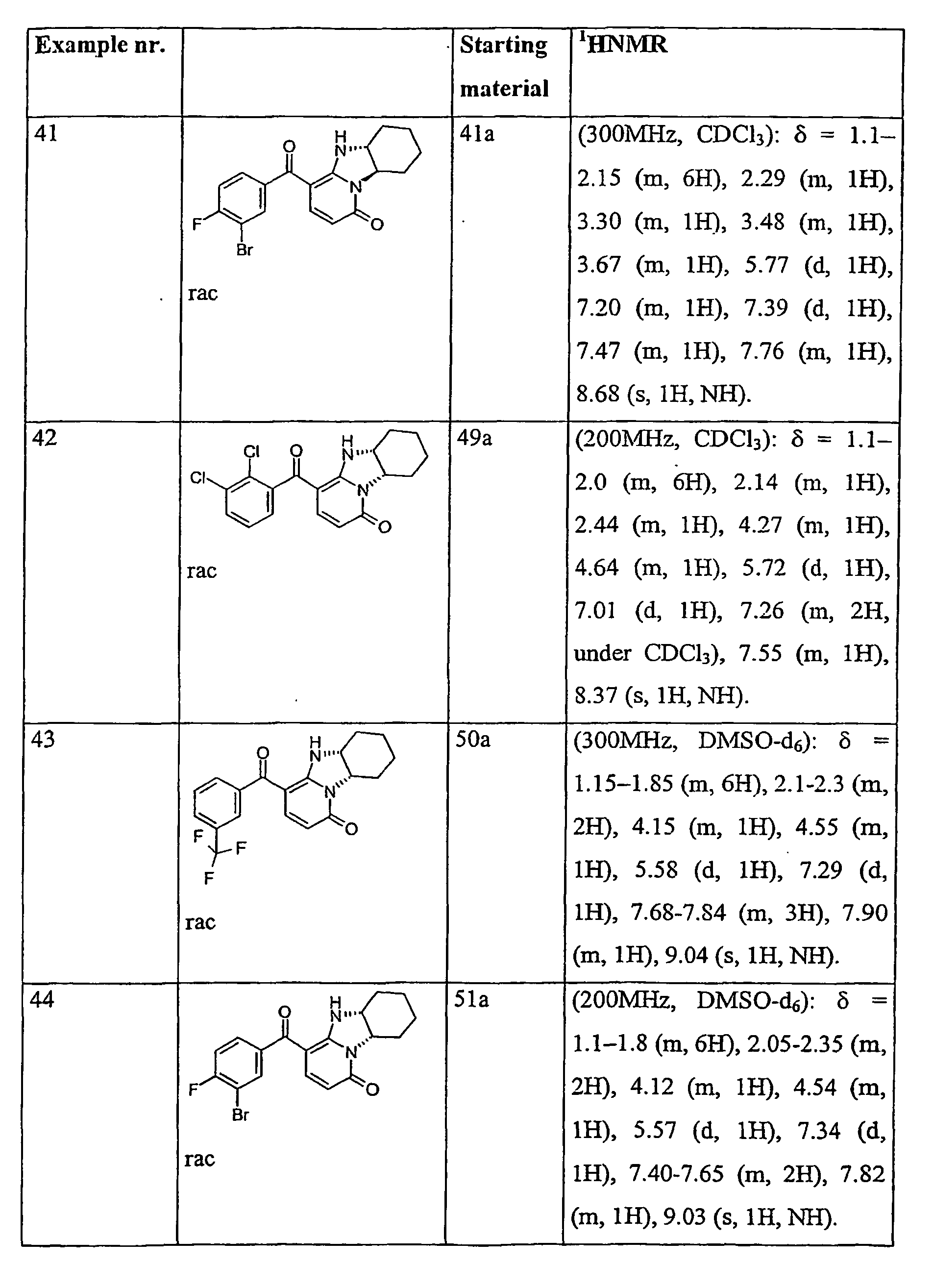

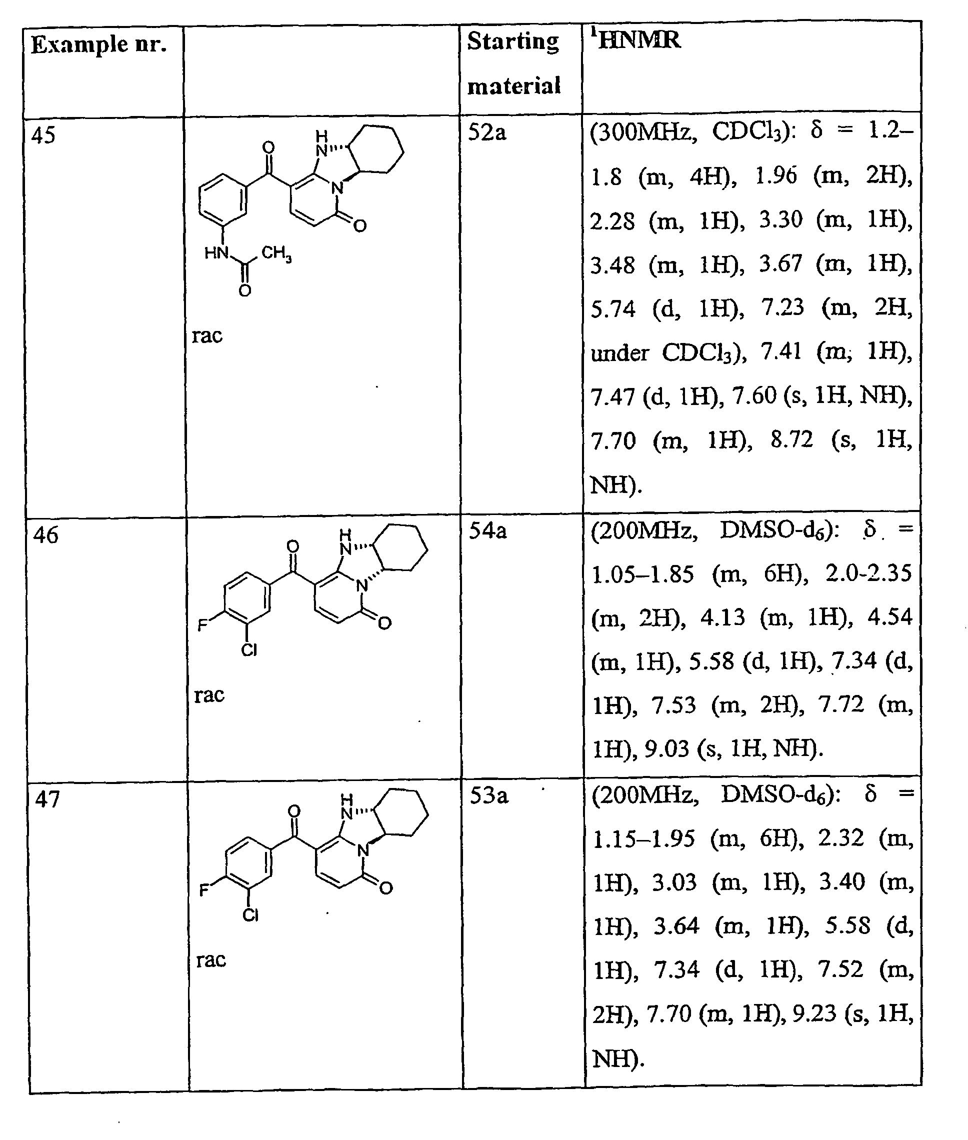

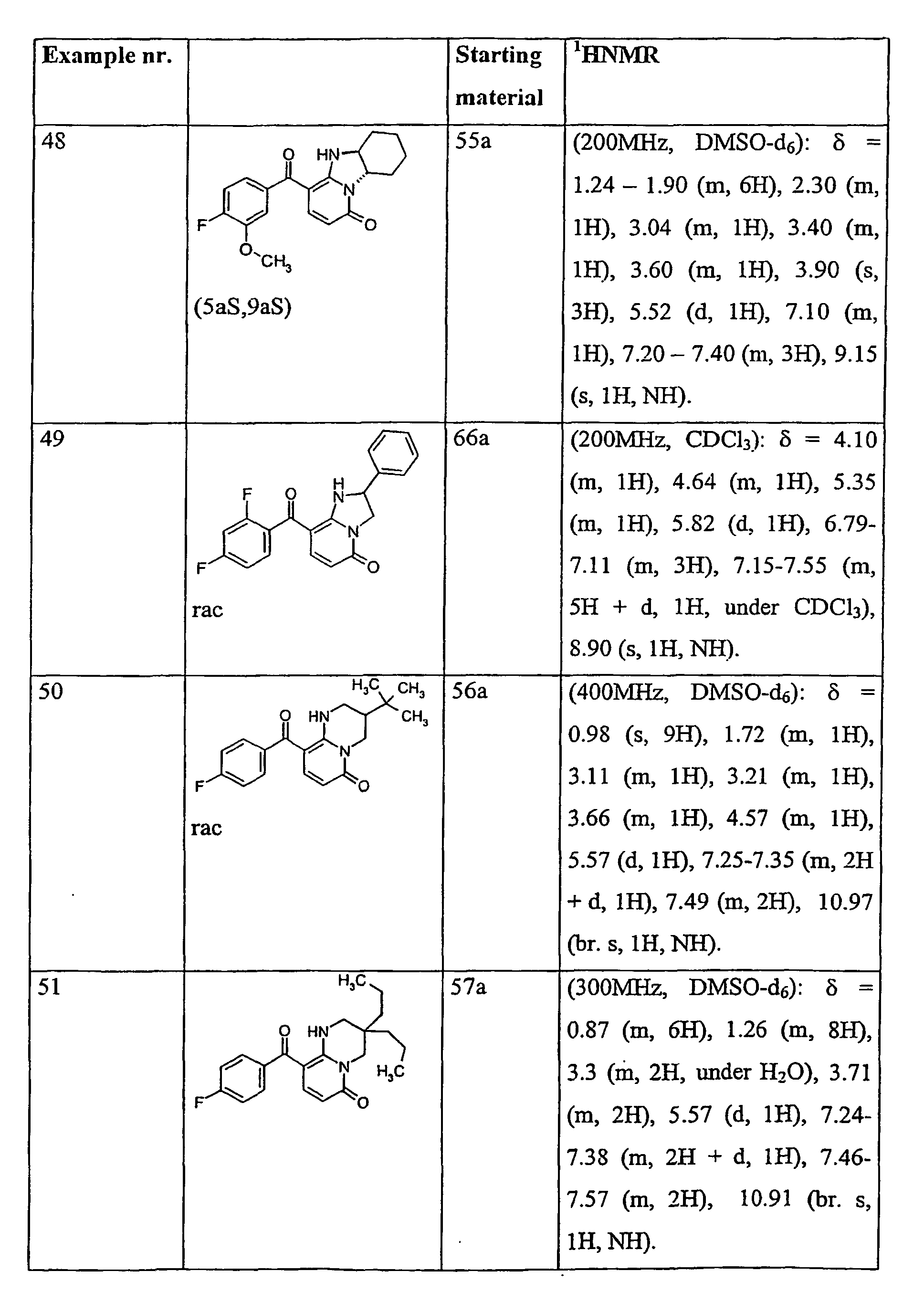

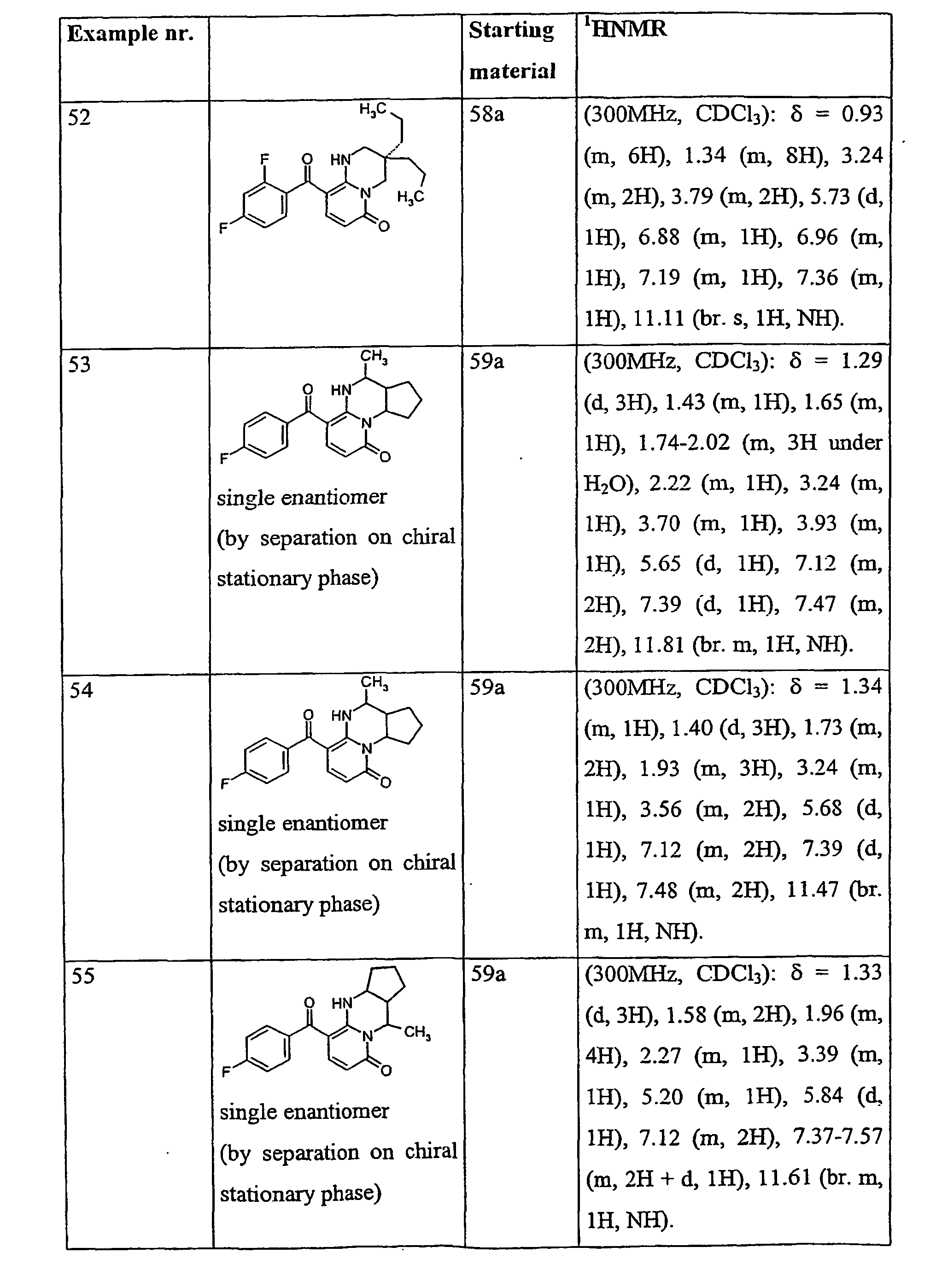

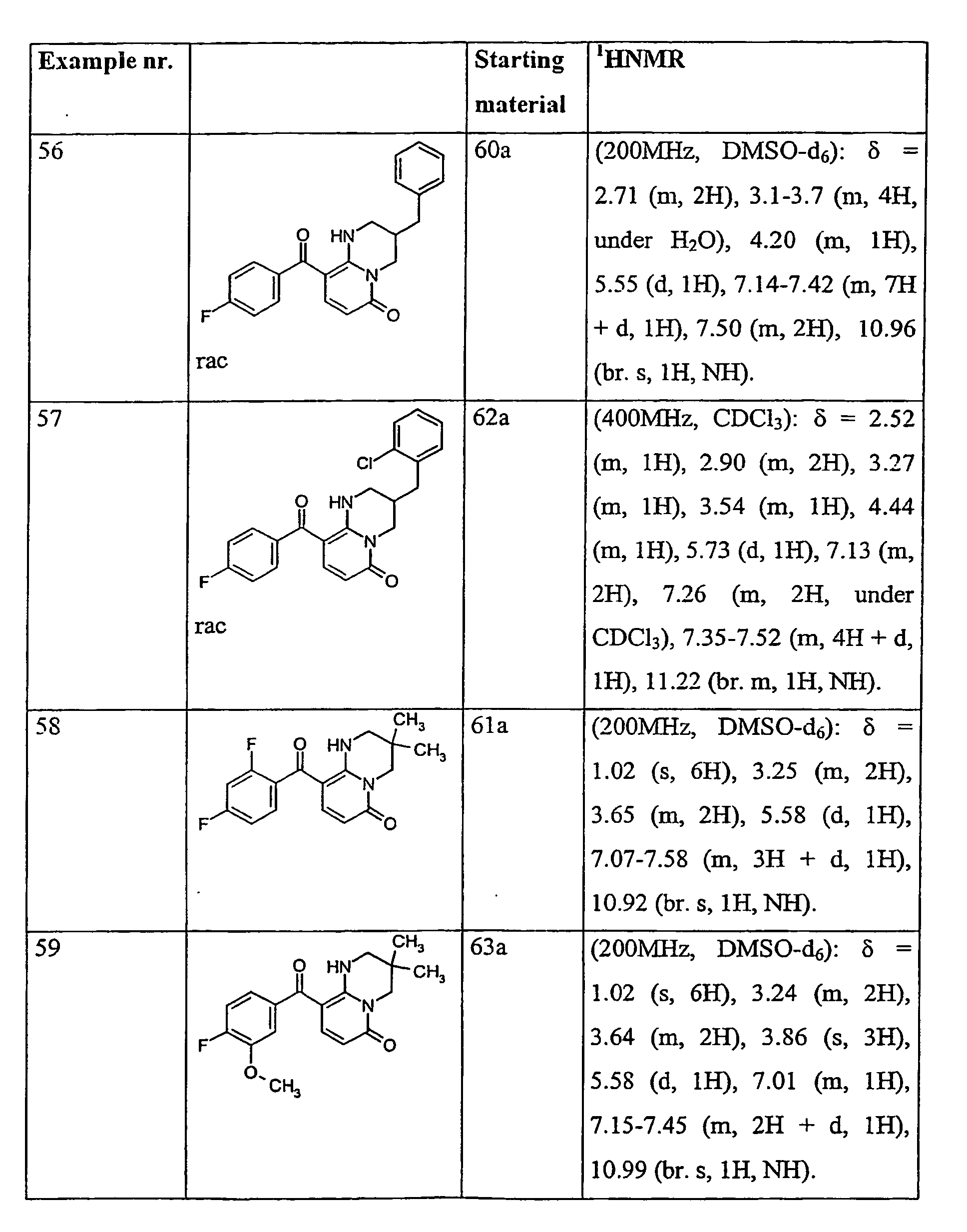

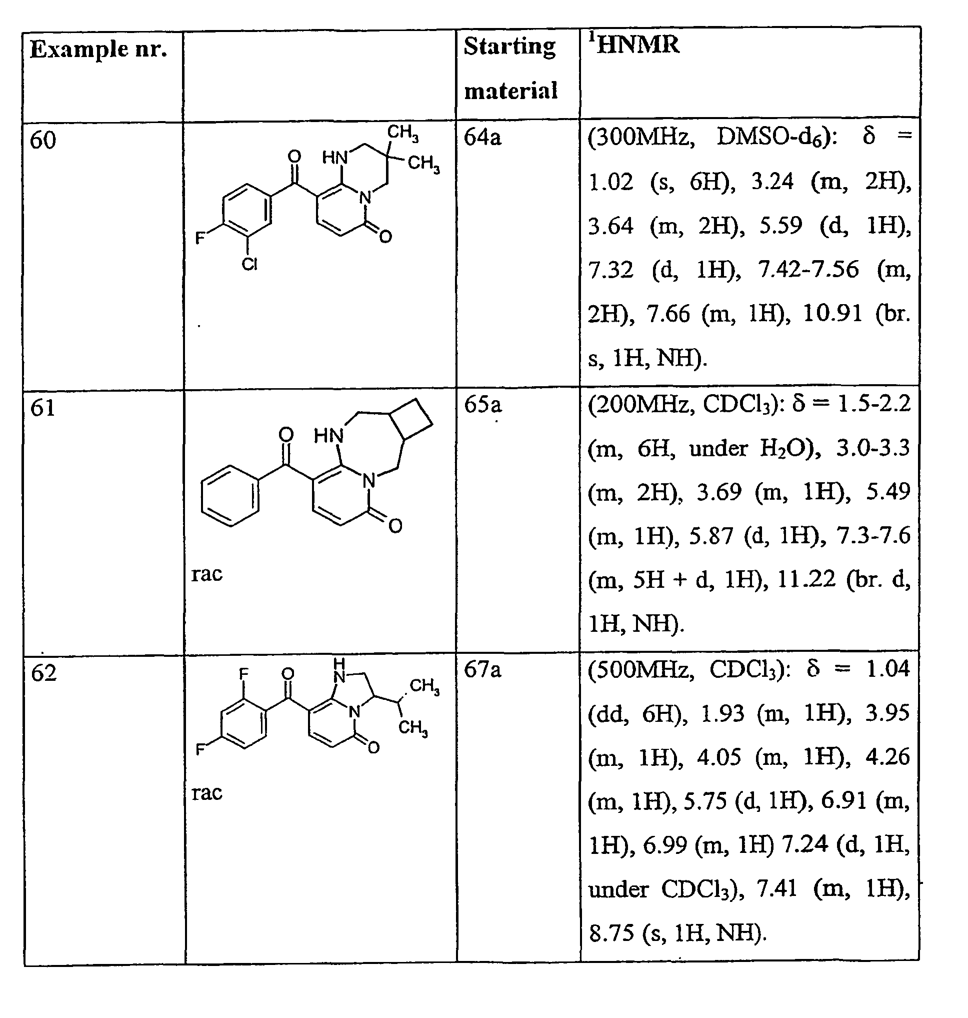

### C. Operative examples relating to pharmaceutical compositions

The compounds according to the invention can be converted into pharmaceutical preparations as follows:

### Tablet

### Composition

100 mg of the compound of Example 1, 50 mg of lactose (monohydrate), 50 mg of maize starch (native), 10 mg of polyvinylpyrrolidone (PVP 25) (from BASF, Ludwigshafen, Germany) and 2 mg of magnesium stearate.

Tablet weight 212 mg, diameter 8 mm, curvature radius 12 mm.

### Preparation

The mixture of active component, lactose and starch is granulated with a 5% solution (m/m) of the PVP in water. After drying, the granules are mixed with magnesium stearate for 5 min. This mixture is moulded using a customary tablet press (tablet format, see above). The moulding force applied is typically 15 kN.

### Orally administrable suspension

### Composition

1000 mg of the compound of Example 1, 1000 mg of ethanol (96%), 400 mg of Rhodigel (xanthan gum from FMC, Pennsylvania, USA) and 99 g of water.

A single dose of 100 mg of the compound according to the invention is provided by 10 ml of oral suspension.

### Preparation

The Rhodigel is suspended in ethanol and the active component is added to the suspension. The water is added with stirring. Stirring is continued for about 6h until the swelling of the Rhodigel is complete.

## Claims

1. Compounds of formula (I) 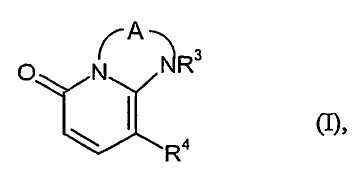 wherein
A represents Ethan-1,2-diyl, Propan-1,3-diyl or Butan-1,4-diyl,
which is substituted with 1 to 4 substituents independently selected from the group consisting of C₁-C₆-alkyl, C₁-C₆-alkoxy, C₆-C₁₀-aryl, C₅-C₈-heteroaryl, C₃-C₈-cycloalkyl or COR^{A-1},
wherein C₁-C₆-alkyl, C₁-C₆-alkoxy, C₆-C₁₀-aryl, C₅-C₈-heteroaryl or C₃-C₈-cycloalkyl can be substituted with 0 to 3 substituents R¹⁻¹,
wherein R¹⁻¹ is independently selected from the group consisting of halogen, amino, mono- or di-C₁-C₆-alkylamino, C₁-C₆-alkoxy, hydroxy, C₆-C₁₀-aryl or halogen-C₆-C₁₀-aryl,
and wherein R^{A-1} is OH, C₁-C₆-alkoxy, C₆-C₁₀-aryloxy, amino, mono- or di-C₁-C₆-alkylamino,
and/or
2 substituents on A together with the carbon atoms to which they are attached form a C₃-C₈-cycloalkyl- or C₃-C₈-heterocyclyl-ring, wherein the C₃-C₈-cycloalkyl- or C₃-C₈-heterocyclyl-ring can be substituted with 0 to 3 substituents independently selected from the group consisting of C₁-C₆-alkyl, C₁-C₆-alkoxy, halogen, cyano, amino, mono- or di-C₁-C₆-alkylamino or COR²⁻², wherein R²⁻² is OH, C₁-C₆-alkoxy, C₆-C₁₀-aryloxy, amino, mono- or di-C₁-C₆-alkylamino,
R³ represents hydrogen or C₁-C₆-alkyl,
R⁴ represents -COR⁴⁻¹, wherein
R⁴⁻¹ represents C₆-C₁₀-aryl or C₅-C₈-heteroaryl,
wherein R⁴⁻¹ can be substituted with 0 to 3 substituents independently selected from the group consisting of halogen, amino, C₁-C₆-alkyl, C₂-C₆-alkenyl, C₂-C₆-alkinyl, C₁-C₆-alkoxy, hydroxy, mono or di-C₁-C₆-alkylamino, C₁-C₆-alkycarbonylamino, trifluoromethyl, cyano and nitro,
wherein C₁-C₆-alkyl, C₂-C₆-alkenyl, C₂-C₆-alkinyl and C₁-C₆-alkoxy can be substituted with 0 to 3 substituents independently selected from the group consisting of hydroxy, amino, dimethylamino, C₁-C₄-alkoxy, 1,3-dioxolan and phenyl,
or
R⁴⁻¹ can be substituted with C₆-C₁₀-aryl or C₅-C₈-heteroaryl, which can be optionally be substituted with 0 to 3 substituents independently selected from the group consisting of halogen, amine, C₁-C₆-alkoxy, hydroxy or C₆-C₁₀-aryl.

2. Compounds of formula (I) according to claim 1,
wherein
A represents Ethan-1,2-diyl, Propan-1,3-diyl or Butan-1,4-diyl,
which is substituted with 1 to 4 substituents independently selected from the group consisting of C₁-C₆-alkyl, C₁-C₆-alkoxy, C₆-C₁₀-aryl or C₃-C₈-cycloalkyl,
wherein C₁-C₆-alkyl, C₁-C₆-alkoxy, C₆-C₁₀-aryl or C₃-C₈-cycloalkyl can be substituted with 0 to 3 substituents R¹⁻¹,
wherein R¹⁻¹ is independently selected from the group consisting of halogen, C₁-C₆-alkoxy, hydroxy, C₆-C₁₀-aryl C₆-C₁₀-aryl or halogen-C₆-C₁₀-aryl,
and/or
2 substituents on A together with the carbon atoms to which they are attached form a C₃-C₈-cycloalkyl- or C₃-C₈-heterocyclyl-ring,
wherein the C₃-C₈-cycloalkyl- or C₃-C₈-heterocyclyl-ring can be substituted with 0 to 3 substituents independently selected from the group consisting of C₁-C₆-alkyl, C₁-C₆-alkoxy, halogen and cyano,
R³ represents hydrogen,
R⁴ represents -COR⁴⁻¹, wherein
R⁴⁻¹ represents phenyl or naphtyl,
wherein R⁴⁻¹ can be substituted with 0 to 3 substituents independently selected from the group consisting of halogen, C₁-C₆-alkyl, C₂-C₆-alkenyl, C₂-C₆-akinyl, C₁-C₆-alkoxy, hydroxy, C₁-C₆-alkycarbonylamino, trifluoromethyl and nitro,
wherein C₂-C₆-alkinyl can be substituted with 0 to 2 phenyl.

3. Compounds of formula (I) according to claim 1 or 2,
wherein
A represents Ethan-1,2-diyl or Propan-1,3-diyl,
which is substituted with 1 to 4 substituents independently selected from the group consisting of C₁-C₆-alkyl, phenyl, benzyl and chlorobenzyl,
and/or
2 substituents on A together with the carbon atoms to which they are attached form a C₃-C₈-cycloalkyl-ring,
wherein the C₃-C₈-cycloalkyl-ring can be substituted with 0 to 3 substituents independently selected from the group consisting of C₁-C₆-alkyl, C₁-C₆-alkoxy and halogen,
R³ represents hydrogen,
R⁴ represents -COR⁴⁻¹, wherein
R⁴⁻¹ represents phenyl,
wherein R⁴⁻¹ can be substituted with 0 to 2 substituents independently selected from the group consisting of fluorine, chlorine, bromine, methyl, hydroxy, methoxy and C₁-C₆-alkycarbonylamino.

4. Compounds of formula (I) according to claim 1, 2 or 3,
wherein A represents Ethan-1,2-diyl, and 2 substituents on A together with the carbon atoms to which they are attached form a cyclohexylring, which can be substituted as described in claim 1, 2 or 3.

5. Compounds according to general formula (I), according to claim 1, 2 or 3,
wherein
R³ is hydrogen.

6. Compounds according to general formula (I), according to claim 1, 2 or 3,
wherein R⁴ is -C(O)C₆H₅, wherein R⁴ can be substituted with 0 to 3 substituents independently selected from the group consisting of fluorine, chlorine, bromine, hydroxy or methyl.

7. A process for synthesizing the compounds of general formula (I), according to claim 1, 2 or 3, **characterized in that** compounds of general formula (II) 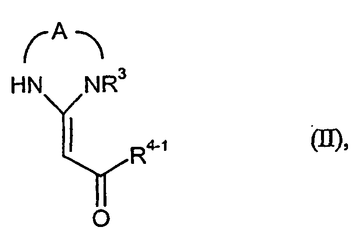 wherein
A, R³ and R⁴⁻¹ have the meaning described above,
are reacted
[A] with propiolic acid in the presence of 1,1-carbonyldiimidazol, or
[B] with C₁-C₆-alkyl propiolate or
[C] with 3-aminoacrylic acid C₁-C₆-alkyl ester or
[D] with 3-alkoxyacrylic acid C₁-C₆-alkyl ester.
[E] with propiolic acid chloride.
[F] with α-chloro acrylic acid chloride.

8. The composition containing at least one compound of general formula (I) according to claim 1, 2 or 3 and a pharmacologically acceptable diluent.

9. A composition according to claim 8 for the treatment of acute and chronic inflammatory processes.

10. The process for the preparation of compositions according to claim 8 and 9 **characterized in that** the compounds of general formula (I) according to claim 1, 2 or 3 together with customary auxiliaries in brought into a suitable application form.

11. Use compounds of general formula (I) according to according to claim 1, 2 or 3 for the preparation of medicaments.

12. Use according to claim 11 for the preparation of medicaments for the treatment of acute and chronic inflammatory processes.

13. Use according to claim 12, wherein the process is asthma or COPD.

14. Use of an antiinflammatorily effective amount of at least one compound according to any of Claims 1 to 3 in the preparation of medicaments for controlling acute and chronic inflammatory processes in humans and animals.

15. Compounds of general formula (II) 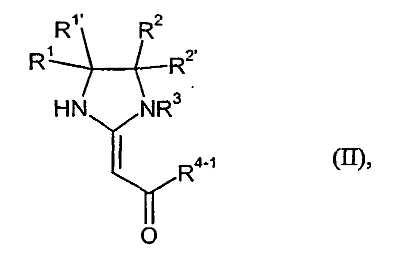 wherein
R¹ and R² together with the carbon atoms, to which they are attached form a cyclohexyl ring, R^{1'} and R^{2'} are hydrogen and R⁴⁻¹ represents C₆-C₁₀-aryl or heteroaryl,
wherein R⁴⁻¹ can be substituted with 0 to 3 substituents independently selected from the group consisting of halogen, amino, C₁-C₆-alkyl, C₁-C₆-alkoxy, mono or di-C₁-C₆-alkylamino, trifluoromethyl, cyano and nitro,
wherein C₁-C₆-alkyl and C₁-C₆-alkoxy can be substituted with 0 to 3 substituents independently selected from the group consisting of hydroxy, amino, dimethylamino, C₁-C₄-alkoxy and 1,3-dioxolan, with the exception of such compounds, wherein R⁴⁻¹ represents phenyl, p-methylphenyl or p-methoxyphenyl.

## Patentansprüche

1. Verbindungen der Formel (I): 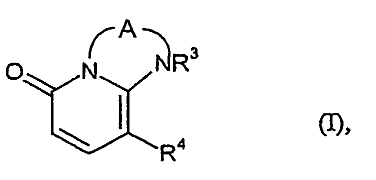 worin gilt:
A stellt Ethan-1,2-diyl, Propan-1,3-diyl oder Butan-1,4-diyl dar, die mit 1 bis 4 Substituenten substituiert sind, unabhängig ausgewählt aus der Gruppe, bestehend aus C₁₋₆-Alkyl, C₁₋₆-Alkoxy, C₆₋₁₀-Aryl, C₅₋₈-Heteroaryl, C₃₋₈-Cycloalkyl oder aus COR^{A-1},
worin C₁₋₆-Alkyl, C₁₋₆-Alkoxy, C₆₋₁₀-Aryl, C₅₋₈-Heteroaryl oder C₃₋₈-Cycloalkyl mit 0 bis 3 Substituenten R¹⁻¹ substituiert sein können,
worin R¹⁻¹ unabhängig aus der Gruppe ausgewählt ist, bestehend aus Halogen, Amino, Mono- oder Di-C₁₋₆-Alkylamino, C₁₋₆-Alkoxy, Hydroxy, C₆₋₁₀-Aryl oder aus Halogen-C₆₋₁₀-aryl,
und worin R^{A-1} OH, C₁₋₆-Alkoxy, C₆₋₁₀-Aryloxy, Amino, Mono- oder Di-C₁₋₆-Alkylamino ist,
und/oder
2 Substituenten an A bilden zusammen mit den Kohlenstoffatomen, an die sie gebunden sind, einen C₃₋₈-Cycloalkyl- oder C₃₋₈-Heterocyclyl-Ring,
worin der C₃₋₈-Cycloalkyl- oder C₃₋₈-Heterocyclyl-Ring mit 0 bis 3 Substituenten substituiert sein kann, die unabhängig aus der Gruppe ausgewählt sind, bestehend aus C₁₋₆-Alkyl,
C₁₋₆-Alkoxy, Halogen, Cyano, Amino, Mono- oder Di-C₁₋₆-alkylamino oder aus COR²⁻², worin R²⁻² OH, C₁₋₆-Alkoxy, C₆₋₁₀-Aryloxy, Amino, Mono- oder Di-C₁₋₆-alkylamino ist,
R³ stellt Wasserstoff oder C₁₋₆-Alkyl dar,
R⁴ stellt -COR⁴⁻¹ dar, worin
R⁴⁻¹ C₆₋₁₀-Aryl oder C₅₋₈-Heteroaryl darstellt mit 0 bis 3 Substituenten substituiert sind, die unabhängig aus der Gruppe ausgewählt sind, bestehend aus Halogen, Amino, C₁₋₆-Alkyl, C₂₋₆-Alkenyl, C₂₋₆-Alkinyl, C₁₋₆-Alkoxy, Hydroxy, Mono- oder Di-C₁₋₆-alkylamino, C₁₋₆-Alkylcarbonylamino, Trifluormethyl, Cyano und aus Nitro, worin C₁₋₆-Alkyl, C₂₋₆-Alkenyl, C₂₋₆-Alkinyl und C₁₋₆-Alkoxy mit 0 bis 3 Substituenten substituiert sind, die unabhängig aus der Gruppe ausgewählt sind, bestehend aus Hydroxy, Amino, Dimethylamino, C₁₋₄-Alkoxy, 1,3-Dioxolan und aus Phenyl,
oder
R⁴⁻¹ kann mit C₆₋₁₀-Aryl oder mit C₅₋₈-Heteroaryl substituiert sein, die gegebenenfalls mit 0 bis 3 Substituenten substituiert sind, die unabhängig aus der Gruppe ausgewählt sind, bestehend aus Halogen, Amino, C₁₋₆-Alkoxy, Hydroxy oder aus C₆₋₁₀-Aryl.

2. Verbindungen der Formel (I) gemäß Anspruch 1,
worin gilt:
A stellt Ethan-1,2-diyl, Propan-1,3-diyl oder Butan-1,4-diyl dar, die mit 1 bis 4 Substituenten substituiert sind, die unabhängig aus der Gruppe ausgewählt sind, bestehend aus C₁₋₆-Alkyl, C₁₋₆-Alkoxy, C₆₋₁₀-Aryl oder aus C₃₋₈-Cycloalkyl,
worin C₁₋₆-Alkyl, C₁₋₆-Alkoxy, C₆₋₁₀-Aryl oder C₃₋₈-Cycloalkyl mit 0 bis 3 Substituenten R¹⁻¹ substituiert sind,
worin R¹⁻¹ unabhängig aus der Gruppe ausgewählt ist, bestehend aus Halogen, C₁₋₆-Alkoxy, Hydroxy, C₆₋₁₀-Aryl oder aus Halogen-C₆₋₁₀-aryl,
und/oder
2 Substituenten an A bilden zusammen mit den Kohlenstoffatomen, an die sie gebunden sind, einen C₃₋₈-Cycloalkyl- oder C₃₋₈-Heterocyclyl-Ring,
worin der C₃₋₈-Cycloalkyl- oder C₃₋₈-Heterocyclyl-Ring mit 0 bis 3 Substituenten substituiert sind, die unabhängig aus der Gruppe ausgewählt sind, bestehend aus C₁₋₆-Alkyl, C₁₋₆-Alkoxy, Halogen und aus Cyano,
R³ stellt Wasserstoff dar,
R⁴ stellt -COR⁴⁻¹ dar, worin
R⁴⁻¹ Phenyl oder Naphthyl darstellt,
die mit 0 bis 3 Substituenten substituiert sind,
die unabhängig aus der Gruppe ausgewählt sind, bestehend aus Halogen, C₁₋₆-Alkyl, C₂₋₆-Alkenyl, C₂₋₆-Alkinyl, C₁₋₆-Alkoxy, Hydroxy, C₁₋₆-Alkylcarbonylamino, Trifluormethyl und aus Nitro, worin
der C₂₋₆-Alkinylrest mit 0 bis 2 Phenylresten substituiert ist.

3. Verbindungen der Formel (I) gemäß Anspruch 1 oder 2,
worin gilt:
A stellt Ethan-1,2-diyl oder Propan-1,3-diyl dar,
die mit 1 bis 4 Substituenten substituiert sind, die unabhängig aus der Gruppe ausgewählt sind, bestehend aus C₁₋₆-Alkyl, Phenyl, Benzyl und aus Chlorbenzyl,
und/oder
2 Substituenten an A bilden zusammen mit den Kohlenstoffatomen, an die sie gebunden sind, einen C₃₋₈-Cycloalkyl-Ring, der mit 0 bis 3 Substituenten substituiert ist, die unabhängig aus der Gruppe ausgewählt sind, bestehend aus C₁₋₆-Alkyl, C₁₋₆-Alkoxy und aus Halogen,
R³ stellt Wasserstoff dar,
R⁴ stellt -COR⁴⁻¹ dar, worin
R⁴⁻¹ einen Phenylrest darstellt, der mit 0 bis 2 Substituenten substituiert ist, die unabhängig aus der Gruppe ausgewählt sind, bestehend aus Fluor, Chlor, Brom, Methyl, Hydroxy, Methoxy und aus C₁₋₆-Alkylcarbonylamino.

4. Verbindungen der Formel (I) gemäß einem der Ansprüche 1, 2 oder 3,
worin A Ethan-1,2-diyl darstellt und 2 Substituenten an A zusammen mit den Kohlenstoffatomen, an die sie gebunden sind, einen Cyclohexylring bilden, der wie in Anspruch 1, 2 oder 3 substituiert sein kann.

5. Verbindungen der allgemeinen Formel (I) gemäß einem der Ansprüche 1, 2 oder 3,
worin
R³ Wasserstoff ist.

6. Verbindungen der allgemeinen Formel (I) gemäß einem der Ansprüche 1, 2 oder 3,
worin R⁴ -C(O)C₆H₅ ist, das mit 0 bis 3 Substituenten substituiert ist, die unabhängig aus der Gruppe ausgewählt sind, bestehend aus Fluor, Chlor, Brom, Hydroxy oder aus Methyl.

7. Verfahren zur Synthese der Verbindungen der allgemeinen Formel (I) gemäß einem der Ansprüche 1 , 2 oder 3, **dadurch gekennzeichnet, dass** Verbindungen der allgemeinen Formel (II): 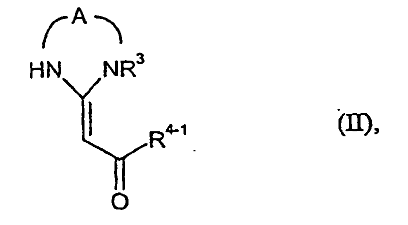 worin
A, R³ und R⁴⁻¹ die oben beschriebene Bedeutung haben, zur Reaktion gebracht werden:
[A] mit Propiolsäure in der Gegenwart von 1,1-Carbonyldiimidazol oder
[B] mit C₁₋₆-Alkylpropiolat oder
[C] mit 3-Aminoacrylsäure-C₁₋₆-alkylester oder
[D] mit 3-Alkoxyacrylsäure-C₁₋₆-alkylester,
[E] mit Propiolsäurechlorid,
[F] mit α-Chloracrylsäurechlorid.

8. Zusammensetzung, enthaltend mindestens eine Verbindung der allgemeinen Formel (I) gemäß einem der Ansprüche 1, 2 oder 3 und ein pharmakologisch zulässiges Verdünnungsmittel.

9. Zusammensetzung gemäß Anspruch 8 zur Behandlung akuter und chronischer Entzündungsprozesse.

10. Verfahren zur Herstellung von Zusammensetzungen gemäß Anspruch 8 oder 9, **dadurch gekennzeichnet, dass** die Verbindungen der allgemeinen Formel (I) gemäß einem der Ansprüche 1, 2 oder 3 zusammen mit üblichen Hilfsstoffen in eine geeignete Anwendungsform gebracht werden.

11. Verwendung von Verbindungen der allgemeinen Formel (I) gemäß einem der Ansprüche 1, 2 oder 3 zur Herstellung von Medikamenten.

12. Verwendung gemäß Anspruch 11 zur Herstellung von Medikamenten zur Behandlung akuter und chronischer Entzündungsprozesse.

13. Verwendung gemäß Anspruch 12, worin der Krankheitsverlauf Asthma oder COPD ist.

14. Verwendung einer entzündungshemmend wirksamen Menge von mindestens einer Verbindung gemäß einem der Ansprüche 1 bis 3 zur Herstellung von Medikamenten zur steuernden Behandlung und Bekämpfung akuter und chronischer Entzündungsprozesse bei Mensch und Tier.

15. Verbindungen der allgemeinen Formel (II): 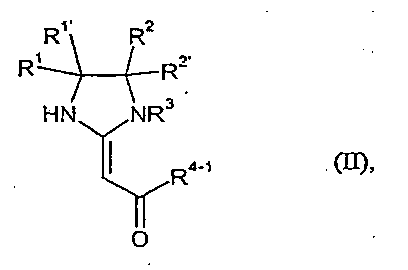 worin gilt:
R¹ und R² bilden zusammen mit den Kohlenstoffatomen, an die sie gebunden sind, einen Cyclohexylring, R^{1'} und R^{2'} sind Wasserstoff, und R⁴⁻¹ stellt C₆₋₁₀-Aryl oder Heteroaryl dar,
worin R⁴⁻¹ mit 0 bis 3 Substituenten substituiert ist, die unabhängig aus der Gruppe ausgewählt sind, bestehend aus Halogen, Amino, C₁₋₆-Alkyl, C₁₋₆-Alkoxy, Mono- oder Di-C₁₋₆-alkylamino, Trifluormethyl, Cyano und aus Nitro, worin C₁₋₆-Alkyl und C₁₋₆-Alkoxy mit 0 bis
3 Substituenten substituiert sind, die unabhängig aus der Gruppe ausgewählt sind, bestehend aus Hydroxy, Amino, Dimethylamino, C₁₋₄-Alkoxy und aus 1,3-Dioxolan, ausgenommen solche Verbindungen, worin R⁴⁻¹ Phenyl, p-Methylphenyl oder p-Methoxyphenyl darstellt.

## Revendications

1. Composés de formule (I) 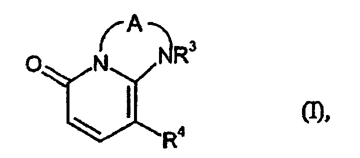 dans laquelle
A représente un groupe éthane-1,2-diyle, propane-1,3-diyle ou butane-1,4-diyle,
qui est substitué par 1 à 4 substituants choisis indépendamment dans la classe formée par les groupes alkyle en C₁-C₆, alkoxy en C₁-C₆, aryle en C₆-C₁₀, hétéroaryle en C₅-C₈, cycloalkyle en C₃-C₈ ou COR^{A-1},
où les groupes alkyle en C₁-C₆, alkoxy en C₁-C₆, aryle en C₆-C₁₀, hétéroaryle en C₅-C₈ ou cycloalkyle en C₃-C₈ peuvent être substitués par 0 à 3 substituants R¹⁻¹,
où R¹⁻¹ est choisi indépendamment dans la classe formée par les groupes halogéno, amino, mono- ou di(alkyle en C₁-C₆)amino, alkoxy en C₁-C₆, hydroxyle, aryle en C₆-C₁₀ ou halogéno (aryle en C₆-C₁₀),
et où R^{A-1} est OH, un groupe alkoxy en C₁-C₆, aryloxy en C₆-C₁₀, amino, mono- ou di(alkyle en C₁-C₆)amino,
et/ou
2 substituants sur A avec les atomes de carbone auxquels ils sont attachés forment un cycle cycloalkyle en C₃-C₈ ou hétérocyclyle en C₃-C₈,
où le cycle cycloalkyle en C₃-C₈ ou hétérocyclyle en C₃-C₈ peut être substitué par 0 à 3 substituants choisis indépendamment dans la classe formée par les groupes alkyle en C₁-C₆, alkoxy en C₁-C₆, halogéno, cyano, amino, mono- ou di(alkyle en C₁-C₆)amino ou COR²⁻², où R²⁻² est OH, un groupe alkoxy en C₁-C₆, aryloxy en C₆-C₁₀, amino, mono- ou di(alkyle en C₁-C₆)amino,
R³ représente l'hydrogène ou un groupe alkyle en C₁-C₆,
R⁴ représente -COR⁴⁻¹, où
R⁴⁻¹ représente un groupe aryle en C₆-C₁₀ ou hétéroaryle en C₅-C₈,
où R⁴⁻¹ peut être substitué par 0 à 3 substituants choisis indépendamment dans la classe formée par les groupes halogéno, amino, alkyle en C₁-C₆, alcényle en C₂-C₆, alcynyle en C₂-C₆, alkoxy en C₁-C₆, hydroxyle, mono- ou di(alkyle en C₁-C₆)amino, (alkyle en C₁-C₆)carbonylamino, trifluorométhyle, cyano et nitro,
où les groupes alkyle en C₁-C₆, alcényle en C₂-C₆, alcynyle en C₂-C₆ et alkoxy en C₁-C₆ peuvent être substitués par 0 à 3 substituants choisis indépendamment dans la classe formée par les groupes hydroxyle, amino, diméthylamino, alkoxy en C₁-C₄, 1,3-dioxolanne et phényle,
ou bien
R⁴⁻¹ peut être substitué par un groupe aryle en C₆-C₁₀ ou hétéroaryle en C₅-C₈, qui peut facultativement être substitué par 0 à 3 substituants choisis indépendamment dans la classe formée par les groupes halogéno, amino, alkoxy en C₁-C₆, hydroxyle ou aryle en C₆-C₁₀.

2. Composés de formule (I) selon la revendication 1, dans lesquels
A représente un groupe éthane-1,2-diyle, propane-1,3-diyle ou butane-1,4-diyle
qui est substitué par 1 à 4 substituants choisis indépendamment dans la classe formée par les groupes alkyle en C₁-C₆, alkoxy en C₁-C₆, aryle en C₆-C₁₀ ou cycloalkyle en C₃-C₈,
où les groupes alkyle en C₁-C₆, alkoxy en C₁-C₆, aryle en C₆-C₁₀ ou cycloalkyle en C₃-C₈ peuvent être substitués par 0 à 3 substituants R¹⁻¹,
où R¹⁻¹ est choisi indépendamment dans la classe formée par les groupes halogéno, alkoxy en C₁-C₆, hydroxyle, aryle en C₆-C₁₀(aryle en C₆-C₁₀) ou halogéno (aryle en C₆-C₁₀),
et/ou
2 substituants sur A avec les atomes de carbone auxquels ils sont attachés forment un cycle cycloalkyle en C₃-C₈ ou hétérocyclyle en C₃-C₈,
où le cycle cycloalkyle en C₃-C₈ ou hétérocyclyle en C₃-C₈ peut être substitué par 0 à 3 substituants choisis indépendamment dans la classe formée par les groupes alkyle en C₁-C₆, alkoxy en C₁-C₆, halogéno et cyano,
R³ représente l'hydrogène,
R⁴ représente -COR⁴⁻¹, où
R⁴⁻¹ représente un groupe phényle ou naphtyle,
où R⁴⁻¹ peut être substitué par 0 à 3 substituants choisis indépendamment dans la classe formée par les groupes halogéno, alkyle en C₁-C₆, alcényle en C₂-C₆, alcynyle en C₂-C₆, alkoxy en C₁-C₆, hydroxyle, (alkyle en C₁-C₆)carbonylamino, trifluorométhyle et nitro,
où les groupes alcynyle en C₂-C₆ peuvent être substitués par 0 à 2 groupes phényle.

3. Composés de formule (I) selon la revendication 1 ou 2, dans lesquels
A représente un groupe éthane-1,2-diyle ou propane-1,3-diyle,
qui est substitué par 1 à 4 substituants choisis indépendamment dans la classe formée par les groupes alkyle en C₁-C₆, phényle, benzyle et chlorobenzyle,
et/ou
2 substituants sur A avec les atomes de carbone auxquels ils sont attachés forment un cycle cycloalkyle en C₃-C₈,
où le cycle cycloalkyle en C₃-C₈ peut être substitué par 0 à 3 substituants choisis indépendamment dans la classe formée par les groupes alkyle en C₁-C₆, alkoxy en C₁-C₆ et halogéno,
R³ représente l'hydrogène,
R⁴ représente -COR⁴⁻¹, où
R⁴⁻¹ représente un groupe phényle,
où R⁴⁻¹ peut être substitué par 0 à 2 substituants choisis indépendamment dans la classe formée par le fluor, le chlore, le brome, les groupes méthyle, hydroxyle, méthoxy et (alkyle en C₁-C₆)carbonylamino.

4. Composés de formule (I) selon la revendication 1, 2 ou 3, dans lesquels
A représente un groupe éthane-1,2-diyle, et
2 substituants sur A avec les atomes de carbone auxquels ils sont attachés forment un cycle cyclohexyle qui peut être substitué comme décrit dans la revendication 1, 2 ou 3.

5. Composés selon la formule générale (I), selon la revendication 1, 2 ou 3, dans lesquels
R³ est l'hydrogène.

6. Composés selon la formule générale (I), selon la revendication 1, 2 ou 3, dans lesquels
R⁴ est -C(O)C₆H₅,
où R⁴ peut être substitué par 0 à 3 substituants choisis indépendamment dans la classe formée par le fluor, le chlore, le brome, les groupes hydroxyle et méthyle.

7. Procédé de synthèse des composés de formule générale (I) selon la revendication 1, 2 ou 3, **caractérisé en ce que** des composés de formule générale (II) 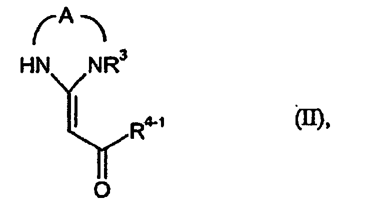 où A, R³ et R⁴⁻¹ ont les significations données ci-dessus, sont amenés à réagir
[A] avec l'acide propiolique en présence de 1,1-carbonyldiimidazole, ou
[B] avec un propiolate d'alkyle en C₁-C₆ ou
[C] avec un ester d'alkyle en C₁-C₆ d'acide 3-aminoacrylique ou
[D] avec un ester d'alkyle en C₁-C₆ d'acide 3-alkoxyacrylique,
[E] avec le chlorure d'acide propiolique,
[F] avec le chlorure d'acide α-chloroacrylique.

8. Composition contenant au moins un composé de formule générale (I) selon la revendication 1, 2 ou 3 et un diluant pharmacologiquement acceptable.

9. Composition selon la revendication 8, pour le traitement de processus inflammatoires aigus et chroniques.

10. Procédé pour la préparation de compositions selon les revendications 8 et 9, **caractérisé en ce que** les composés de formule générale (I) selon la revendication 1, 2 ou 3 ainsi que des auxiliaires usuels sont transformés en une forme d'application appropriée.

11. Utilisation de composés de formule générale (I) selon la revendication 1, 2 ou 3, pour la préparation de médicaments.

12. Utilisation selon la revendication 11, pour la préparation de médicaments destinés au traitement de processus inflammatoires aigus et chroniques.

13. Utilisation selon la revendication 12, dans laquelle le processus est l'asthme ou la bronchopneumopathie chronique obstructive.

14. Utilisation d'une quantité à effet anti-inflammatoire d'au moins un composé selon l'une quelconque des revendications 1 à 3 dans la préparation de médicaments destinés à combattre des processus inflammatoires aigus et chroniques chez les êtres humains et les animaux.

15. Composés de formule générale (II) 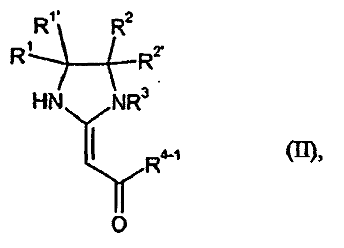 dans laquelle
R¹ et R² avec les atomes de carbone auxquels ils sont attachés forment un cycle cyclohexyle, R^{1'} et R^{2'} sont de l'hydrogène et R⁴⁻¹ représente un groupe aryle ou hétéroaryle en C₆-C₁₀,
où R⁴⁻¹ peut être substitué par 0 à 3 substituants choisis indépendamment dans la classe formée par les groupes halogéno, amino, alkyle en C₁-C₆, alkoxy en C₁-C₆, mono- ou di(alkyle en C₁-C₆)amino, trifluorométhyle, cyano et nitro,
où les groupes alkyle en C₁-C₆ et alkoxy en C₁-C₆ peuvent être substitués par 0 à 3 substituants choisis indépendamment dans la classe formée par les groupes hydroxyle, amino, diméthylamino, alkoxy en C₁-C₄ et 1,3-dioxolanne,
à l'exception de tels composés dans lesquels R⁴⁻¹ représente un groupe phényle, p-méthylphényle ou p-méthoxyphényle.
